# EUROPEAN PATENT APPLICATION

(11) **EP 4 381 961 A1**
(43) Date of publication of application: **12.06.2024**
(21) Application number: 22853074.7
(22) Date of filing: 02.08.2022
(51) Int. Cl.: A23L 27/10, A23L 27/20, A23L 27/22, A23L 27/23, C12N 1/00

(54) **METHOD FOR IMPROVING FLAVOR OF FOOD**

(30) Priority: 02.08.2021 JP 2021126904
(71) Applicant: AJINOMOTO CO., INC., Chuo-ku Tokyo 104-8315 (JP)
(72) Inventor: TOYAZAKI, Miku, Kawasaki-shi, Kanagawa 210-8681 (JP); HIRATANI, Moe, Kawasaki-shi, Kanagawa 210-8681 (JP); HAYASHI, Kazuyuki, Kawasaki-shi, Kanagawa 210-8681 (JP); SUZUKI, Masashi, Kawasaki-shi, Kanagawa 210-8681 (JP); YAHAGI, Daiki, Kawasaki-shi, Kanagawa 210-8681 (JP); USAMI, Riku, Kawasaki-shi, Kanagawa 210-8681 (JP); KURODA, Motonaka, Kawasaki-shi, Kanagawa 210-8681 (JP); MIZUNO, Masaki, Kawasaki-shi, Kanagawa 210-8681 (JP); SEKI, Toshihito, Kawasaki-shi, Kanagawa 210-8681 (JP); TAJIMA, Yoshinori, Kawasaki-shi, Kanagawa 210-8681 (JP)
(74) Representative: Hoffmann Eitle
(86) International application number: PCT/JP2022/029709
(87) International publication number: WO 2023/013655

(57) **Abstract**

Provided is a technology for improving the flavor of food. The flavor of food is improved by adding the following ingredient (A):
(A) a cell wall-containing fraction of a gram-positive bacterium.

## Description

### TECHNICAL FIELD

The present invention relates to a technology for improving a flavor of food.

### BACKGROUND ART

Various technologies for improving the flavor of food have been reported.

Patent Document 1 discloses a flavor base for taste sensation enhancement, obtained by fermentation of bacteria such as *Corynebacterium glutamicum, Corynebacterium ammoniagenes, Corynebacterium casei, Corynebacterium efficiens, Brevibacterium lactofermentum,* and *Bacillus subtilis.*

Patent Document 2 discloses a flavor enhancer comprising yeast cells from which the content of yeast is removed, and illustrates pungency or the like as a flavor.

Patent Document 3 discloses a yeast extract which contains peptide and nucleic acid-based taste ingredients and which can simultaneously impart umami to be continuously felt, and a thickness feeling, thereby imparting or enhancing a rich taste to or in food.

Patent Document 4 discloses an aroma improver for food, comprising a yeast-derived product such as a yeast extract, and illustrates a spice aroma as an aroma.

Patent Document 5 discloses a spicy-feeling enhancer comprising a product obtained by heating reaction of sugar and amino acid in the presence of alcohol.

Patent Document 6 discloses a fermented seasoning composition which contains a fermented product with lactic acid bacteria and yeast and which can be utilized for enhancing sensation of irritation by spices.

### PRIOR ART DOCUMENTS

### [Patent Documents]

[Patent Document 1] JP2012-521205A
[Patent Document 2] JP2018-050562A
[Patent Document 3] JP2007-049988A
[Patent Document 4] JP2010-166886A
[Patent Document 5] JP2016-158507A
[Patent Document 6] WO2017/014253

### SUMMARY OF THE INVENTION

### PROBLEMS TO BE SOLVED BY THE INVENTION

An object of the present invention is to provide a technology for improving the flavor of food.

### MEANS FOR SOLVING THE PROBLEMS

The present inventors have found that addition of a cell wall-containing fraction of a gram-positive bacterium can improve the flavor of food, thereby completing the present invention.

Accordingly, the present invention can be illustrated as follows.
[1] A composition for improving a flavor of food, comprising the following ingredient (A):
   (A) a cell wall-containing fraction of a gram-positive bacterium.
[2] The composition mentioned above, wherein the improvement of the flavor is enhancement of spiciness and/or impartment of a rich taste.
[3] The composition mentioned above, wherein the improvement of the flavor is enhancement of pungency of a spice.
[4] The composition mentioned above, further comprising a spice.
[5] A composition comprising the following ingredient (A) and a spice:
   (A) a cell wall-containing fraction of a gram-positive bacterium.
[6] The composition mentioned above, wherein the ingredient (A) is a cell of the gram-positive bacterium, or a fragment thereof.
[7] The composition mentioned above, wherein the gram-positive bacterium is a bacterium belonging to the *Actinobacteria* division or the *Firmicutes* division.
[8] The composition mentioned above, wherein the gram-positive bacterium is a bacterium belonging to the *Actinobacteria* division.
[9] The composition mentioned above, wherein the gram-positive bacterium is selected from the group consisting of a coryneform bacterium, a bacterium belonging to the *Bifidobacteriaceae* family, a bacterium belonging to the *Dermabacteraceae* family, a bacterium belonging to the *Bacillaceae* family, a bacterium belonging to the *Enterococcaceae* family, or a bacterium belonging to the *Lactobacillaceae* family.
[10] The composition mentioned above, wherein the gram-positive bacterium is selected from the group consisting of a *Corynebacterium* genus bacterium, a *Brevibacterium* genus bacterium, a *Bifidobacterium* genus bacterium, a *Brachybacterium* genus bacterium, a *Bacillus* genus bacterium, an *Enterococcus* genus bacterium, or a *Lactobacillus* genus bacterium.
[11] The composition mentioned above, wherein the gram-positive bacterium is selected from the group consisting of *Corynebacterium casei, Corynebacterium flavescens, Brevibacterium casei, Bifidobacterium longum, Brachybacterium alimentarium, Bacillus subtilis, Enterococcus faecalis, Lactobacillus mali, Lactobacillus hilgardii,* or *Lactobacillus Brevis.*
[12] The composition mentioned above, wherein the amount of the ingredient (A) contained, converted into the dry mass of a cell of the gram-positive bacterium, is 0.1% (w/w) or more.
[13] The composition mentioned above, further comprising the following ingredient (B):
   (B) one or more ingredients selected from the group consisting of L-amino acid, nucleic acid, and organic acid.
[14] The composition mentioned above, further comprising at least the L-amino acid, wherein the L-amino acid is L-glutamic acid.
[15] The composition mentioned above, wherein the amount of L-glutamic acid contained is 0.1 to 20 parts by weight based on 1 part by weight of the ingredient (A) converted into the dry mass of a cell of the gram-positive bacterium.
[16] The composition mentioned above, wherein the amount of the spice contained is 0.2 to 500 parts by weight based on 1 part by weight of the ingredient (A) converted into the dry mass of a cell of the gram-positive bacterium.
[17] The composition mentioned above, wherein the spice is one or more spices selected from the group consisting of spices of the *Lauraceae* family, spices of the *Piperaceae* family, spices of the *Lamiaceae* family, spices of the *Apiaceae* family, spices of the *Solanaceae* family, spices of the *Myristicaceae* family, spices of the *Alliaceae* family, spices of the *Myrtaceae* family, spices of the *Schisandraceae* family, spices of the *Fabaceae* family, spices of the *Polygonaceae* family, spices of the *Brassicaceae* family, spices of the *Zingiberaceae* family, and spices of the *Rutaceae* family.
[18] The composition mentioned above, wherein the spice is a spice having pungency.
[19] The composition mentioned above, wherein the composition is a seasoning.
[20] The composition mentioned above, wherein the ingredient (A) is produced by culturing the gram-positive bacterium in a medium containing a raw material of food.
[21] The composition mentioned above, wherein the raw material contained in the medium is a tomato.
[22] The composition mentioned above, wherein the ingredient (A) is heat-treated.
[23] The composition mentioned above, wherein the gram-positive bacterium is a bacterium which is a bacterium having an L-glutamic acid-producing ability and which has one or more mutations selected from mutations shown in Table 1 described later.
[24] The composition mentioned above, further comprising a culture product of the gram-positive bacterium.
[25] A method of improving a flavor of food, the method comprising:
   a step of adding the following ingredient (A) to a raw material of food:
   (A) a cell wall-containing fraction of a gram-positive bacterium.
[26] A method of producing food with improved flavor, the method comprising:
   a step of adding the following ingredient (A) to a raw material of food:
   (A) a cell wall-containing fraction of a gram-positive bacterium.
[27] The method mentioned above, wherein the improvement of the flavor is enhancement of spiciness and/or impartment of a rich taste.
[28] The method mentioned above, wherein the improvement of the flavor is enhancement of pungency of a spice.
[29] The method mentioned above, wherein the ingredient (A) is a cell of the gram-positive bacterium, or a fragment thereof.
[30] The method mentioned above, wherein the gram-positive bacterium is a bacterium belonging to the *Actinobacteria* division or the *Firmicutes* division.
[31] The method mentioned above, wherein the gram-positive bacterium is a bacterium belonging to the *Actinobacteria* division.
[32] The method mentioned above, wherein the gram-positive bacterium is selected from the group consisting of a coryneform bacterium, a bacterium belonging to the *Bifidobacteriaceae* family, a bacterium belonging to the *Dermabacteraceae* family, a bacterium belonging to the *Bacillaceae* family, a bacterium belonging to the *Enterococcaceae* family, or a bacterium belonging to the *Lactobacillaceae* family.
[33] The method mentioned above, wherein the gram-positive bacterium is selected from the group consisting of a *Corynebacterium* genus bacterium, a *Brevibacterium* genus bacterium, a *Bifidobacterium* genus bacterium, a *Brachybacterium* genus bacterium, a *Bacillus* genus bacterium, an *Enterococcus* genus bacterium, or a *Lactobacillus* genus bacterium.
[34] The method mentioned above, wherein the gram-positive bacterium is selected from the group consisting of *Corynebacterium casei, Corynebacterium flavescens, Brevibacterium casei, Bifidobacterium longum, Brachybacterium alimentarium, Bacillus subtilis, Enterococcus faecalis, Lactobacillus mali, Lactobacillus hilgardii,* or *Lactobacillus Brevis.*
[35] The method mentioned above, wherein the ingredient (A) is added so that the concentration thereof at the time of eating, converted into the dry mass of a cell of the gram-positive bacterium, is 0.005 to 2% (w/w).
[36] The method mentioned above, further comprising a step of adding the following ingredient (B) to a raw material of food:
   (B) one or more ingredients selected from the group consisting of L-amino acid, nucleic acid, and organic acid.
[37] The method mentioned above, wherein at least the L-amino acid is added and the L-amino acid is L-glutamic acid.
[38] The method mentioned above, wherein L-glutamic acid is added so that the concentration thereof at the time of eating is 0.01 to 2% (w/w).
[39] The method mentioned above, wherein the method is performed so that the amount of L-glutamic acid contained in the food is 0.1 to 20 parts by weight based on 1 part by weight of the ingredient (A) converted into the dry mass of a cell of the gram-positive bacterium.
[40] The method mentioned above, wherein the food is spice-containing food.
[41] The method mentioned above, wherein the amount of the spice contained in the food, as a concentration at the time of eating, is 0.01 to 2% (w/w).
[42] The method mentioned above, wherein the method is performed so that the amount of the spice contained in the food is 0.2 to 500 parts by weight based on 1 part by weight of the ingredient (A) converted into the dry mass of a cell of the gram-positive bacterium.
[43] The method mentioned above, wherein the spice is one or more spices selected from the group consisting of spices of the *Lauraceae* family, spices of the *Piperaceae* family, spices of the *Lamiaceae* family, spices of the *Apiaceae* family, spices of the *Solanaceae* family, spices of the *Myristicaceae* family, spices of the *Alliaceae* family, spices of the *Myrtaceae* family, spices of the *Schisandraceae* family, spices of the *Fabaceae* family, spices of the *Polygonaceae* family, spices of the *Brassicaceae* family, spices of the *Zingiberaceae* family, and spices of the *Rutaceae* family.
[44] The method mentioned above, wherein the spice is a spice having pungency.
[45] The method mentioned above, wherein the ingredient (A) is produced by culturing the gram-positive bacterium in a medium containing a raw material of food.
[46] The method mentioned above, wherein the raw material contained in the medium is a tomato.
[47] The method mentioned above, wherein the ingredient (A) is heat-treated.
[48] The method mentioned above, wherein the gram-positive bacterium is a bacterium which is a bacterium having an L-glutamic acid-producing ability and which has one or more mutations selected from mutations shown in Table 1 described later.
[49] The method mentioned above, further comprising a step of adding a culture product of the gram-positive bacterium, to a raw material of food.
[50] A seasoning comprising the following ingredient (A):
   (A) a cell wall-containing fraction of a gram-positive bacterium.
[51] The seasoning mentioned above, wherein the ingredient (A) is a cell of the gram-positive bacterium, or a fragment thereof.
[52] The seasoning mentioned above, further comprising the following ingredient (B):
   (B) one or more ingredients selected from the group consisting of L-amino acid, nucleic acid, and organic acid.
[53] The seasoning mentioned above, further comprising at least the L-amino acid, wherein the L-amino acid is L-glutamic acid.
[54] The seasoning mentioned above, further comprising a spice.
[55] The seasoning mentioned above, wherein the ingredient (A) is produced by culturing the gram-positive bacterium in a medium containing a raw material of food.
[56] The seasoning mentioned above, wherein the raw material contained in the medium is a tomato.
[57] The seasoning mentioned above, wherein the ingredient (A) is heat-treated.
[58] The seasoning, wherein the gram-positive bacterium is a bacterium which is a bacterium having an L-glutamic acid-producing ability and which has one or more mutations selected from mutations shown in Table 1 described later.
[59] The seasoning mentioned above, further comprising a culture product of the gram-positive bacterium.
[60] A bacterium having an L-glutamic acid-producing ability, the bacterium having:
   one or more mutations selected from mutations A-1 to A-135 shown in Table 1 described later.
[61] The bacterium mentioned above, having 50 or more mutations selected from mutations A-1 to A-135 shown in Table 1 described later.
[62] The bacterium mentioned above, having 100 or more mutations selected from mutations A-1 to A-135 shown in Table 1 described later.
[63] The bacterium mentioned above, having mutations A-1 to A-135 shown in Table 1 described later.
[64] The bacterium mentioned above, further having one or more mutations selected from mutations B-1 to B-92 shown in Table 1 described later.
[65] The bacterium mentioned above, having 30 or more mutations selected from mutations B-1 to B-92 shown in Table 1 described later.
[66] The bacterium mentioned above, having 60 or more mutations selected from mutations B-1 to B-92 shown in Table 1 described later.
[67] The bacterium mentioned above, wherein the bacterium is a coryneform bacterium.
[68] The bacterium mentioned above, wherein the bacterium is a *Corynebacterium* genus bacterium.
[69] The bacterium mentioned above, wherein the bacterium is *Corynebacterium casei.*
[70] The bacterium mentioned above, wherein the bacterium is a *Corynebacterium casei* JCM 12072 strain-derived modified strain.
[71] A method of producing a composition for improving a flavor of food, the method comprising:
   a step of culturing a gram-positive bacterium in a medium to provide a culture product,
   wherein the composition conprisesthe following ingredient (A):
      (A) a cell wall-containing fraction of the gram-positive bacterium.
[72] The method mentioned above, wherein the improvement of the flavor is enhancement of spiciness and/or impartment of a rich taste.
[73] The method mentioned above, wherein the improvement of the flavor is enhancement of pungency of a spice.
[74] The method mentioned above, wherein the composition further comprises a spice.
[75] The method mentioned above, wherein the ingredient (A) is a cell of the gram-positive bacterium, or a fragment thereof.
[76] The method mentioned above, wherein the composition further comprises the following ingredient (B):
   (B) one or more ingredients selected from the group consisting of L-amino acid, nucleic acid, and organic acid.
[77] The method mentioned above, wherein the composition further comprises at least the L-amino acid and the L-amino acid is L-glutamic acid.
[78] The method mentioned above, further comprising a step of heat-treating the culture product.
[79] The method mentioned above, wherein the gram-positive bacterium is a bacterium which is a bacterium having an L-glutamic acid-producing ability and which has one or more mutations selected from mutations shown in Table 1 described later.
[80] The method mentioned above, wherein the composition further comprises a culture product of the gram-positive bacterium.
[81] A method of producing a composition for enhancement of umami of food, the method comprising:
   a step of culturing the bacterium in a medium to provide a culture product containing L-glutamic acid,
   wherein the composition contains the L-glutamic acid.
[82] The method mentioned above, wherein the composition further comprises the following ingredient (A):
   (A) a cell wall-containing fraction of a gram-positive bacterium.
[83] A method of producing L-glutamic acid, the method comprising:
   a step of culturing the bacterium in a medium to provide a culture product containing L-glutamic acid; and
   a step of recovering the L-glutamic acid.
[84] A composition for enhancement of umami of food, the composition comprising L-glutamic acid, wherein
   the L-glutamic acid is produced by culturing the bacterium in a medium.
[85] The composition mentioned above, further comprising the following ingredient (A):
   (A) a cell wall-containing fraction of a gram-positive bacterium.
[86] A method of enhancing umami of food, the method comprising:
   a step of adding L-glutamic acid to a raw material of food,
   wherein the L-glutamic acid is produced by culturing the bacterium in a medium.
[87] A method of producing food with enhanced umami, the method comprising:
   a step of adding L-glutamic acid to a raw material of food,
   wherein the L-glutamic acid is produced by culturing the bacterium in a medium.
[88] The method mentioned above, further comprising
   a step of adding the following ingredient (A) to a raw material of food:
   (A) a cell wall-containing fraction of a gram-positive bacterium.

### BRIEF DESCRIPTION OF THE DRAWINGS

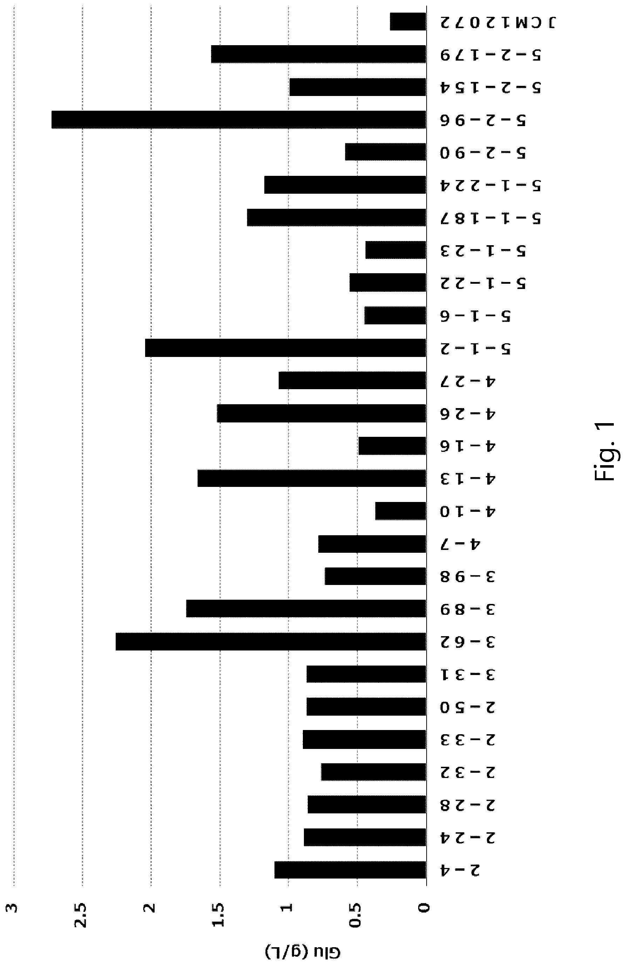

Figure 1 shows a diagram representing the produced amount of L-glutamic acid of a mutant strain library.

### MODE FOR CARRYING OUT THE INVENTION

### <1> Active Ingredient

In the present invention, the following ingredient (A) is used as an active ingredient:
(A) a cell wall-containing fraction of a gram-positive bacterium.

The ingredient (A) is also referred to as "active ingredient" or "cell wall fraction".

The active ingredient can be used to improve the flavor of food, i.e., obtain an effect of improving the flavor of food. The effect is also referred to as "flavor improvement effect". Improvement of the flavor of food is also simply referred to as "improvement of the flavor". Specifically, the active ingredient can be used to improve the flavor of food as compared with a case where the active ingredient is not used. Thus, the active ingredient can be used for improvement of the flavor of food.

In addition, the active ingredient can be used to produce food with improved flavor. Thus, the active ingredient may be used for production of food (specifically, production of food with improved flavor).

Examples of the flavor include tastes and perfumes. That is, tastes and/or perfumes may be improved by using the active ingredient. Here, the term "perfumes" mentioned may mean perfumes (i.e., retronasal aroma) to be felt from the throat toward the nasal cavity and/or perfumes (i.e., orthonasal aroma) to be felt directly from the nose, at the time of eating of food. Herein, the term "perfumes" may particularly mean perfumes (i.e., retronasal aroma) to be felt from the throat toward the nasal cavity at the time of eating of food.

Examples of improvement of the flavor include enhancement of spiciness and impartment of a rich taste. Examples of improvement of the flavor particularly include enhancement of spiciness. The active ingredient may be used to improve one flavor singly, or improve two or more flavors in combination. That is, the active ingredient may be used to achieve, for example, enhancement of spiciness and/or impartment of a rich taste.

The term "spiciness" may mean a sensation felt due to the presence of a spice. "Spiciness" can also be called Spice Flavor, Spice aroma, Pungency, Hotness, or Burningness. The term "spiciness" may specifically mean a sensation felt due to the presence of a spice at the time of eating of spice-containing food. "Spiciness" may be interchangeably used with "flavor of a spice". Examples of spiciness include spice tastes and spice perfumes. Examples of spiciness specifically include spice's pungency. "Enhancement of spiciness" is not limited to enhancement of spiciness of food having spiciness (for example, spice-containing food), and may also include impartment of spiciness to food having no spiciness (for example, spice-free food). For example, use of the active ingredient and a spice in combination can impart spiciness (specifically, spiciness enhanced as compared with the case of no use of any active ingredient) to food having no spiciness.

The term "rich taste" means a sensation which cannot be expressed by five basic tastes expressed by a sweet taste, a salty taste, a sour taste, a bitter taste, and umami, and may specifically mean a taste sensation in which not only these basic tastes, but also marginal tastes and marginal flavors of the basic tastes, such as thickness, growth (mouthfulness), continuity, and harmony are enhanced. Examples of "impartment of a rich taste" include enhancement of the basic tastes, and the corresponding impartment or enhancement marginal tastes of the basic tastes, such as thickness, growth (mouthfulness), continuity, and harmony. Examples of "impartment of a rich taste" include impartment or enhancement of any flavor such as a complex taste, a ripe feeling, robustness, a meat feeling, a milk feeling, a juice feeling, a body feeling (sugar-like body feeling, body feeling of wine, and/or the like), and/or the like.

Flavors (for example, spiciness or rich taste) may be classified to, for example, an initial taste, a middle taste, and an aftertaste. The terms "initial taste", "middle taste", and "aftertaste" of flavors in the case of liquids (liquid foods) mean flavors felt at 0 seconds to one second, at one second to 3 seconds, and at 3 seconds to 5 seconds after eating (after putting food in mouth), respectively. The terms "initial taste", "middle taste", and "aftertaste" of flavors in the case of solids (solid foods) mean flavors felt at 0 seconds to 4 seconds, at 4 seconds to 10 seconds, and at 10 seconds to 15 seconds after eating (after putting food in mouth), respectively. In the present invention, the term "solid" refers to other forms than liquids, and also includes paste, gel, and the like. The active ingredient may be used to improve, specifically, for example, the flavor in the initial taste, the flavor in the middle taste, the flavor in the aftertaste, or any combination thereof. That is, the active ingredient may be used to enhance, specifically, for example, the spiciness in the initial taste, the spiciness in the middle taste, the spiciness in the aftertaste, or any combination thereof. The active ingredient may be used to impart, specifically, for example, the rich taste in the initial taste, the rich taste in the middle taste, the rich taste in the aftertaste, or any combination thereof.

Measurement and comparison of flavors (for example, spiciness or rich taste) can be performed, for example, by sensory evaluation with an expert panel.

The active ingredient may be used for improvement of the flavor or production of food in aspects described in the method of the present invention as described later.

The active ingredient is not particularly limited as long as it contains a cell wall of a gram-positive bacterium. The active ingredient may or may not be composed of a cell wall of a gram-positive bacterium. The cell wall of a gram-positive bacterium may be, for example, present as a cellof a gram-positive bacterium, or a fragment thereof. That is, specific examples of the active ingredient include a cell of a gram-positive bacterium, and a fragment thereof. Examples of the active ingredient particularly include a cell of a gram-positive bacterium. For example, peptide glycan and/or a lipid-soluble ingredient may be contained in a cell wall of a gram-positive bacterium. Examples of the lipid-soluble ingredient in a cell wall of a gram-positive bacterium include teichoic acid, lipoteichoic acid, mycolic acid, mycolic acid sugar esters, lipoarabinomannan, and glycolipids.

The gram-positive bacterium is not particularly limited. Examples of the gram-positive bacterium include bacteria belonging to the *Actinobacteria* division and the *Firmicutes* division. Examples of the gram-positive bacterium particularly include bacteria belonging to the *Actinobacteria* division.

Examples of bacteria belonging to the *Actinobacteria* division include coryneform bacteria, bacteria belonging to the *Bifidobacteriaceae* family, and bacteria belonging to the *Dermabacteraceae* family. Examples of bacteria belonging to the *Actinobacteria* division particularly include coryneform bacteria.

Examples of coryneform bacteria include *Corynebacterium* genus bacteria, *Brevibacterium* genus bacteria, and *Microbacterium* genus bacteria.

Specific examples of coryneform bacteria include the following species.
*Corynebacterium acetoacidophilum*
*Corynebacterium acetoglutamicum*
*Corynebacterium alkanolyticum*
*Corynebacterium callunae*
*Corynebacterium casei*
*Corynebacterium crenatum*
*Corynebacterium flavescens*
*Corynebacterium glutamicum*
*Corynebacterium lilium*
*Corynebacterium melassecola*
*Corynebacterium thermoaminogenes (Corynebacterium efficiens*)
*Corynebacterium herculis*
*Brevibacterium casei*
*Brevibacterium divaricatum (Corynebacterium glutamicum)*
*Brevibacterium flavum (Corynebacterium glutamicum)*
*Brevibacterium immariophilum*
*Brevibacterium lactofermentum (Corynebacterium glutamicum)*
*Brevibacterium roseum*
*Brevibacterium saccharolyticum*
*Brevibacterium thiogenitalis*
*Corynebacterium ammoniagenes (Corynebacterium stationis)*
*Brevibacterium album*
*Brevibacterium cerinum*
*Microbacterium ammoniaphilum*

More specific examples of coryneform bacteria include the following strains.
*Corynebacterium acetoacidophilum* ATCC 13870
*Corynebacterium acetoglutamicum* ATCC 15806
*Corynebacterium alkanolyticum* ATCC 21511
*Corynebacterium callunae* ATCC 15991
*Corynebacterium casei* JCM 12072
*Corynebacterium crenatum* AS 1.542
*Corynebacterium flavescens* ATCC 10340(NBRC 14136)
*Corynebacterium glutamicum* ATCC 13020, ATCC 13032, ATCC 13060, ATCC 13869, FERM BP-734
*Corynebacterium lilium* ATCC 15990
*Corynebacterium melassecola* ATCC 17965
*Corynebacterium efficiens (Corynebacterium thermoaminogenes)* AJ12340 (FERM BP-1539)
*Corynebacterium herculis* ATCC 13868
*Brevibacterium casei* ATCC 35513(DSM 20657)
*Brevibacterium divaricatum (Corynebacterium glutamicum)* ATCC 14020
*Brevibacterium flavum (Corynebacterium glutamicum)* ATCC 13826, ATCC 14067, AJ12418 (FERM BP-2205)
*Brevibacterium immariophilum* ATCC 14068
*Brevibacterium lactofermentum (Corynebacterium glutamicum)* ATCC 13869
*Brevibacterium roseum* ATCC 13825
*Brevibacterium saccharolyticum* ATCC 14066
*Brevibacterium thiogenitalis* ATCC 19240
*Corynebacterium ammoniagenes (Corynebacterium stationis)* ATCC 6871, ATCC 6872
*Brevibacterium album* ATCC 15111
*Brevibacterium cerinum* ATCC 15112
*Microbacterium ammoniaphilum* ATCC 15354

Examples of coryneform bacteria particularly include *Corynebacterium* genus bacteria and *Brevibacterium* genus bacteria. Examples of *Corynebacterium* genus bacteria particularly include *Corynebacterium casei* such as *Corynebacterium casei* JCM 12072 and *Corynebacterium flavescens* such as *Corynebacterium flavescens* ATCC 10340. Examples of *Brevibacterium* genus bacteria particularly include *Brevibacterium casei* such as *Brevibacterium casei* ATCC 35513.

*Corynebacterium* genus bacteria also currently include bacteria integrated into the *Corynebacterium* genus (Int. J. Syst. Bacteriol., 41, 255 (1991)), which have been conventionally classified to the *Brevibacterium* genus. *Corynebacterium stationis* also includes bacteria re-classified to *Corynebacterium stationis* according to 16S rRNA base sequence analysis or the like (Int. J. Syst. Evol. Microbiol., 60, 874-879 (2010)), which has been conventionally classified to *Corynebacterium ammoniagenes.*

Examples of bacteria belonging to the *Bifidobacteriaceae* family include *Bifidobacterium* genus bacteria. Examples of *Bifidobacterium* genus bacteria include *Bifidobacterium longum, Bifidobacterium breve, Bifidobacterium bifidum, Bifidobacterium adolescentis, Bifidobacterium angulalum, Bifidobacterium dentium, Bifidobacterium pseudocatenulatum, Bifidobacterium animalis, Bifidobacterium pseudolongum,* and *Bifidobacterium thermophilum.* Examples of *Bifidobacterium* genus bacteria particularly include *Bifidobacterium longum.* Specific examples of *Bifidobacterium longum* include ATCC 15697, ATCC 15707, ATCC 25962, ATCC 15702, ATCC 27533, BG7, DSM 24736, SBT 2928, NCC 490 (CNCM I-2170), and NCC 2705 (CNCM I-2618). Specific examples of *Bifidobacterium breve* include ATCC 15700, B632 (DSM 24706), Bb99 (DSM 13692), ATCC 15698, DSM 24732, UCC2003, YIT4010, YIT4064, BBG-001, BR-03, C50, and R0070. Specific examples of *Bifidobacterium bifidum* specifically include ATCC 29521, OLB6378, BF-1. Specific examples of *Bifidobacterium adolescentis* include ATCC 15703. Specific examples of *Bifidobacterium dentium* include DSM 20436. Specific examples of *Bifidobacterium pseudocatenulatum* include ATCC 27919. Specific examples of *Bifidobacterium animalis* include DSM 10140, Bb-12, DN-173 010, GCL2505, and CNCM I-3446. Specific examples of *Bifidobacterium pseudolongum* include JCM 5820 and ATCC 25526. Specific examples of *Bifidobacterium thermophilum* include ATCC 25525.

Examples of bacteria belonging to the *Dermabacteraceae* family include *Brachybacterium* genus bacteria. Examples of *Brachybacterium* genus bacteria include *Brachybacterium alimentarium* and *Brachybacterium tyrofermentans.* Examples of *Brachybacterium* genus bacteria particularly include *Brachybacterium alimentarium.* Specific examples of *Brachybacterium alimentarium* include ATCC 700067 (NBRC 16118). Specific examples of *Brachybacterium tyrofermentan* include DSM 10673.

Examples of bacteria belonging to the *Firmicutes* division include bacteria belonging to the *Bacillaceae* family, bacteria belonging to the *Enterococcaceae* family, and bacteria belonging to the *Lactobacillaceae* family.

Examples of bacteria belonging to the *Bacillaceae* family include *Bacillus* genus bacteria. Examples of *Bacillus* genus bacteria include *Bacillus subtilis, Bacillus amyloliquefaciens, Bacillus pumilus, Bacillus licheniformis, Bacillus megaterium, Bacillus brevis, Bacillus polymixa, Bacillus stearothermophilus,* and *Bacillus velezensis.* Examples of *Bacillus* genus bacteria particularly include *Bacillus subtilis.* Specific examples of *Bacillus subtilis* include 168 Marburg strains (ATCC 6051, JCM 1465) and PY79 strains (Plasmid, 1984, 12, 1-9). Specific examples of *Bacillus amyloliquefaciens* include T strains (ATCC 23842), N strains (ATCC 23845), AJ11708 strains (NITE BP-02609), and FZB42 strains (DSM 23117).

Examples of bacteria belonging to the *Enterococcaceae* family include *Enterococcus* genus bacteria. Examples of *Enterococcus* genus bacteria include *Enterococcus faecalis* and *Enterococcus faecium.* Examples of *Enterococcus* genus bacteria particularly include *Enterococcus faecalis.* Specific examples of *Enterococcus faecalis* include ATCC 19433. Specific examples of *Enterococcus faecium* include ATCC 19434.

Examples of bacteria belonging to the *Lactobacillaceae* family include *Lactobacillus* genus bacteria. Examples of *Lactobacillus* genus bacteria include *Lactobacillus mali, Lactobacillus hilgardii, Lactobacillus Brevis, Lactobacillus delbrueckii, Lactobacillus casei, Lactobacillus paracasei, Lactobacillus gasseri,* and *Lactobacillus acidophilus.* Examples of *Lactobacillus* genus bacteria particularly include *Lactobacillus* mali, *Lactobacillus hilgardii,* and *Lactobacillus Brevis.* Specific examples of *Lactobacillus mali* include NBRC 102159 (ATCC 27053). Specific examples of *Lactobacillus hilgardii* include NBRC 15886 (ATCC 8290). Specific examples of *Lactobacillus Brevis* include JCM 1102 (ATCC 27305).

Furnishing of these strains can be made from, for example, the American Type Culture. Collection (address: 12301 Parklawn Drive, Rockville, Maryland 20852 P.O. Box 1549, Manassas, VA 20108, United States of America). That is, the registry number is imparted to each strain, and the registry number can be used for furnishing (see http://www.atcc.org/). The registry number corresponding to each strain is recorded on the catalog from the American Type Culture. Collection. These strains can be available from, for example, the depositary institution where each of the strains is deposited.

The gram-positive bacterium may be modified as appropriate. That is, examples of the gram-positive bacterium also include modified strains derived from the above-illustrated strains. Specific examples of such modified strains include *Corynebacterium casei* JCM 12072-derived modified strains. An object of modification is not particularly limited. Examples of modification include modification for impartment or enhancement of an objective substance-producing ability. Such a modified strain may be, for example, one bred by artificial modification. Examples of artificial modification include modification by a genetic engineering procedure and modification by mutation treatment. Examples of the mutation treatment include irradiation with X-ray, irradiation with ultraviolet light, and each treatment with mutagens such as N-methyl-N'-nitro-N-nitrosoguanidine (MNNG), ethylmethane sulfonate (EMS), and methylmethane sulfonate (MMS). Such a modified strain may naturally occur, for example, in use of the gram-positive bacterium. Examples of such a modified strain include mutant strains naturally occurring in culture of the gram-positive bacterium. Such a modified strain may be constructed by one modification or any combination of two or more modifications.

The gram-positive bacterium may have an objective substance-producing ability.

The term "objective substance-producing ability" means an ability to produce an objective substance. That is, the phrase "bacterium having an objective substance-producing ability" means a bacterium having an ability to produce an objective substance. The phrase "bacterium having an objective substance-producing ability" may specifically mean a bacterium which, when cultured in a medium, has an ability to produce an objective substance and accumulate it in the medium and/or a bacterial cell to such an extent as to be recovered. When the bacterium having an objective substance-producing ability is a modified strain, the bacterium having an objective substance-producing ability may be a bacterium which can allow a larger amount of an objective substance to be accumulated in a medium and/or a bacterial cell than a non-modified strain. Examples of the non-modified strain include wild strains and parent strains. The bacterium having an objective substance-producing ability may be particularly a bacterium which can allow an objective substance to be accumulated in a medium. The bacterium having an objective substance-producing ability may be a bacterium which can allow an amount of preferably 0.5 g/L or more, more preferably 1.0 g/L or more of an objective substance to be accumulated in a medium. The bacterium having an objective substance-producing ability may have an ability to produce only one objective substance, or may have an ability to produce two or more objective substances.

The objective substance is not particularly limited as long as it can be produced by culture of the gram-positive bacterium. Examples of the objective substance include ingredients to be added to food and then used. Specific examples of the objective substance include L-amino acids, nucleic acids, and organic acids.

Examples of L-amino acids include basic amino acids such as L-lysine, L-ornithine, L-arginine, L-histidine, and L-citrulline, aliphatic amino acids such as L-isoleucine, L-alanine, L-valine, L-leucine, and glycine, amino acids as hydroxymonoaminocarboxylic acids such as L-threonine and L-serine, cyclic amino acids such as L-proline, aromatic amino acids such as L-phenylalanine, L-tyrosine, and L-tryptophan, sulfur-containing amino acids L-cysteine, L-cystine and L-methionine, acidic amino acids such as L-glutamic acid and L-aspartic acid, and amino acids each having an amide group in a side chain, such as L-glutamine and L-asparagine. Examples of L-amino acids particularly include L-glutamic acid.

Examples of nucleic acids include purine-based substances. Examples of purine-based substances include purine nucleosides and purine nucleotides. Examples of purine nucleosides include inosine, guanosine, xanthosine, and adenosine. Examples of purine nucleotides include purine nucleoside 5'-phosphates. Examples of purine nucleoside 5'-phosphates include inosinic acid (inosine-5'-phosphate; IMP), guanylic acid (guanosine-5'-phosphate; GMP), xanthylic acid (xanthosine-5'-phosphate; XMP), and adenylic acid (adenosine-5'-phosphate; AMP). Examples of purine-based substances particularly include inosine and guanosine. Examples of purine-based substances further particularly include inosine.

Examples of organic acids include carboxylic acids. Examples of carboxylic acids include monocarboxylic acids and dicarboxylic acids. Examples of monocarboxylic acids include monocarboxylic acids each having 3 to 8 carbon atoms (C₃-C₈ monocarboxylic acids). Specific examples of monocarboxylic acids include pyruvic acid. Examples of dicarboxylic acids include dicarboxylic acids each having 3 to 8 carbon atoms (C₃-C₈ dicarboxylic acids). Specific examples of dicarboxylic acids include α-ketoglutaric acid (α-KG; another name 2-oxyglutaric acid), malic acid, fumaric acid, succinic acid, itaconic acid, malonic acid, adipic acid, glutaric acid, pimelic acid, and suberic acid.

When an objective substance can form a salt, the objective substance may be produced and/or used as a free form, or as a salt, or as a combination thereof. That is, unless otherwise noted, the term "objective substance" may mean an objective substance in a free form, or a salt thereof, or a combination thereof. When an objective substance can form a hydrate, the objective substance may be produced and/or used as a non-hydrate, or as a hydrate, or as a combination thereof. That is, unless otherwise noted, the term "objective substance" (for example, "objective substance in a free form" or "salt of an objective substance") may include a non-hydrate and a hydrate. The objective substance may be in any form, such as an ionic form, when being used. The amount of an objective substance (for example, the amount contained (concentration) or the amount used) in the case of the objective substance forming a salt or hydrate will be calculated based on the value obtained by converting the mass of the salt or hydrate into the mass of an equimolar of a free form.

The salt can be selected as appropriate depending on various conditions such as applications of the objective substance. For example, when the objective substance is used in an application of being orally taken, the salt can be selected so as to be orally taken. Examples of salts of acidic groups, such as a carboxyl group, include ammonium salts; salts with alkaline metals, such as sodium and potassium; salts with alkaline earth metals, such as calcium and magnesium; aluminum salts; zinc salts; salts with organic amines, such as triethylamine, ethanolamine, morpholine, pyrrolidine, piperidine, piperazine, and dicyclohexylamine; and salts with basic amino acids such as arginine and lysine. Examples of salts of basic groups, such as an amino group, include salts with inorganic acids, such as hydrochloric acid, sulfuric acid, phosphoric acid, nitric acid, and hydrobromic acid; salts with organic carboxylic acids, such as acetic acid, citric acid, benzoic acid, maleic acid, fumaric acid, tartaric acid, succinic acid, tannic acid, butyric acid, hibenzic acid, pamoic acid, enanthic acid, decanoic acid, teoclic acid, salicylic acid, lactic acid, oxalic acid, mandelic acid, malic acid, methylmalonic acid, and adipic acid; and salts with organic sulfonic acids, such as methanesulfonic acid, benzenesulfonic acid, and p-toluenesulfonic acid. Examples of salts of L-glutamic acid particularly include sodium L-glutamate (for example, monosodium L-glutamate; MSG) and ammonium L-glutamate (for example, monoammonium L-glutamate). As the salt, one salt may be used, or two or more salts may be used in combination.

The gram-positive bacterium may be one naturally having an objective substance-producing ability, or may be one modified so as to have an objective substance-producing ability. A gram-positive bacterium having an objective substance-producing ability can be obtained by, for example, imparting an objective substance-producing ability to the above-mentioned gram-positive bacterium, or enhancing an objective substance-producing ability of the above-mentioned gram-positive bacterium.

The method of imparting or enhancing an objective substance-producing ability is not particularly limited. The method of imparting or enhancing an objective substance-producing ability can be, for example, a known method. Impartment or enhancement of an objective substance-producing ability can be carried out by, for example, a mutation method or a genetic engineering procedure. The method of imparting or enhancing an ability to produce L-amino acid (for example, L-glutamic acid) can be disclosed in, for example, WO2006/070944, WO2015/060391, and WO2018/030507. The method of imparting or enhancing an ability to produce nucleic acid can be disclosed in, for example, WO2015/060391. For example, impartment or enhancement of an L-glutamic acid-producing ability can be carried out by a procedure described in Examples.

Examples of a bacterium having an L-glutamic acid-producing ability (also referred to as "L-glutamic acid-producing bacterium") include bacteria each having a "specified mutation".

Examples of "specified mutation" include mutations shown in Table 1. Variations shown in Table 1 include 135 mutations of mutations A-1 to A-135 and 92 mutations of mutations B-1 to B-92. Variations A-1 to A-135 are also referred to as "mutations of Group A". Variations B-1 to B-92 are also referred to as "mutations of Group B".

**Table 1**

| No. | **Position on genome** | **Base before mutation** | **Base after mutation** |
|---|---|---|---|
| A-1 | 78,486 | C | T |
| A-2 | 83,592 | G | A |
| A-3 | 87,955 | C | T |
| A-4 | 90,041 | C | T |
| A-5 | 186,221 | C | T |
| A-6 | 193,010 | C | T |
| A-7 | 196,531 | C | T |
| A-8 | 225,429 | C | T |
| A-9 | 297,920 | G | A |
| A-10 | 320,354 | C | T |
| A-11 | 335,878 | C | T |
| A-12 | 341,763 | C | T |
| A-13 | 346,969 | C | T |
| A-14 | 349,856 | C | T |
| A-15 | 356,232 | C | T |
| A-16 | 357,008 | C | T |
| A-17 | 366,674 | G | A |
| A-18 | 369,871 | G | A |
| A-19 | 377,420 | G | A |
| A-20 | 378,652 | G | A |
| A-21 | 432,252 | C | A |
| A-22 | 439,021 | G | A |
| A-23 | 440,764 | G | A |
| A-24 | 454,682 | G | A |
| A-25 | 458,729 | G | A |
| A-26 | 470,562 | G | A |
| A-27 | 471,288 | G | A |
| A-28 | 472,023 | G | A |
| A-29 | 504,885 | G | A |
| A-30 | 505,785 | G | A |
| A-31 | 514,371 | G | A |
| A-32 | 518,684 | G | A |
| A-33 | 521,126 | G | A |
| A-34 | 524,551 | G | A |
| A-35 | 660,841 | C | T |
| A-36 | 732,121 | C | T |
| A-37 | 787,055 | C | T |
| A-38 | 806,047 | C | T |
| A-39 | 872,482 | G | A |
| A-40 | 878,069 | C | T |
| A-41 | 903,037 | C | T |
| A-42 | 922,802 | C | T |
| A-43 | 948,145 | C | T |
| A-44 | 955,819 | C | T |
| A-45 | 968,915 | C | T |
| A-45 | 973,013 | C | T |
| A-47 | 974,797 | C | T |
| A-48 | 994,815 | C | T |
| A-49 | 1,000,498 | C | T |
| A-50 | 1,019,704 | C | T |
| A-51 | 1,049,052 | C | T |
| A-52 | 1,069,322 | C | T |
| A-53 | 1,070,554 | C | T |
| A-54 | 1,131,016 | C | T |
| A-55 | 1,138,639 | C | T |
| A-56 | 1,162,588 | C | T |
| A-57 | 1,193,273 | C | T |
| A-58 | 1,203,146 | C | T |
| A-59 | 1,222,633 | C | T |
| A-60 | 1,226,969 | G | A |
| A-61 | 1,264,895 | G | A |
| A-62 | 1,268,790 | G | A |
| A-63 | 1,279,676 | G | A |
| A-64 | 1,363,909 | T | C |
| A-65 | 1,387,476 | G | A |
| A-66 | 1,401,171 | G | A |
| A-67 | 1,416,228 | C | T |
| A-68 | 1,420,034 | C | T |
| A-69 | 1,447,494 | C | T |
| A-70 | 1,448,318 | C | T |
| A-71 | 1,448,776 | C | T |
| A-72 | 1,451,922 | C | T |
| A-73 | 1,466,961 | C | T |
| A-74 | 1,503,736 | C | T |
| A-75 | 1,504,207 | C | T |
| A-76 | 1,505,998 | C | T |
| A-77 | 1,507,027 | C | T |
| A-78 | 1,544,310 | C | T |
| A-79 | 1,554,973 | C | T |
| A-80 | 1,558,509 | C | T |
| A-81 | 1,562,459 | C | T |
| A-82 | 1,572,716 | C | T |
| A-83 | 1,594,314 | C | T |
| A-84 | 1,602,545 | C | T |
| A-85 | 1,659,808 | C | T |
| A-86 | 1,682,132 | C | T |
| A-87 | 1,689,863 | C | T |
| A-88 | 1,744,963 | C | T |
| A-89 | 1,784,642 | C | T |
| A-90 | 1,814,866 | C | T |
| A-91 | 1,829,145 | C | T |
| A-92 | 1,852,511 | G | A |
| A-93 | 1,861,170 | G | A |
| A-94 | 1,902,133 | G | A |
| A-95 | 1,916,048 | C | T |
| A-96 | 1,917,434 | C | T |
| A-97 | 1,938,271 | C | T |
| A-98 | 1,949,357 | G | T |
| A-99 | 1,954,368 | C | T |
| A-100 | 1,967,997 | C | T |
| A-101 | 1,975,599 | C | T |
| A-102 | 2,141,466 | C | T |
| A-103 | 2,308,064 | C | T |
| A-104 | 2,310,428 | C | T |
| A-105 | 2,354,420 | C | T |
| A-106 | 2,449,270 | T | C |
| A-107 | 2,449,278 | C | A |
| A-108 | 2,449,291 | G | C |
| A-109 | 2,449,318 | G | A |
| A-110 | 2,496,945 | C | T |
| A-111 | 2,505,022 | C | T |
| A-122 | 2,505,285 | C | T |
| A-113 | 2,525,513 | G | A |
| A-114 | 2,565,856 | C | T |
| A-115 | 2,601,306 | G | A |
| A-116 | 2,615,688 | G | A |
| A-117 | 2,650,740 | G | A |
| A-118 | 2,653,259 | G | A |
| A-119 | 2,663,827 | G | A |
| A-120 | 2,667,322 | G | A |
| A-121 | 2,674,077 | G | A |
| A-122 | 2,679,915 | G | A |
| A-123 | 2,686,979 | G | A |
| A-124 | 2,693,950 | C | T |
| A-125 | 2,696,737 | C | T |
| A-126 | 2,706,442 | C | T |
| A-127 | 2,709,469 | C | T |
| A-128 | 2,711,214 | C | T |
| A-129 | 2,714,651 | C | T |
| A-130 | 2,721,339 | G | A |
| A-131 | 2,731,030 | G | A |
| A-132 | 2,746,202 | G | A |
| A-133 | 2,805,389 | C | T |
| A-134 | 2,816,733 | G | A |
| A-135 | 2,827,114 | G | A |
| B-1 | 29,724 | G | A |
| B-2 | 92,869 | G | A |
| B-3 | 116,733 | G | A |
| B-4 | 131,184 | G | A |
| B-5 | 156,247 | G | A |
| B-6 | 177,083 | G | A |
| B-7 | 184,379 | G | A |
| B-8 | 212,586 | G | A |
| B-9 | 282,162 | G | A |
| B-10 | 309,483 | G | A |
| B-11 | 376,164 | C | T |
| B-12 | 440,885 | C | T |
| B-13 | 479,120 | G | A |
| B-14 | 722,430 | G | A |
| B-15 | 745,504 | G | A |
| B-16 | 809,993 | G | A |
| B-17 | 859,643 | G | A |
| B-18 | 923,209 | G | A |
| B-19 | 924,973 | G | A |
| 8-20 | 998,893 | C | T |
| B-21 | 1,062,144 | C | T |
| B-22 | 1,095,062 | C | T |
| 8-23 | 1,102,484 | C | T |
| B-24 | 1,103,812 | C | T |
| B-25 | 1,105,749 | C | T |
| B-26 | 1,107,561 | C | T |
| B-27 | 1,205,722 | C | T |
| B-28 | 1,233,449 | C | T |
| B-29 | 1,242,484 | C | T |
| B-30 | 1,248,388 | C | 7 |
| B-31 | 1,249,270 | C | T |
| B-32 | 1,291,377 | C | T |
| B-33 | 1,308,597 | C | T |
| B-34 | 1,329,535 | C | T |
| B-35 | 1,367,486 | C | T |
| B-36 | 1,382,065 | C | T |
| B-37 | 1,403,043 | C | T |
| B-38 | 1,433,914 | C | T |
| B-39 | 1,442,447 | C | T |
| B-40 | 1,501,903 | G | A |
| B-41 | 1,504,744 | C | T |
| B-42 | 1,651,403 | G | A |
| B-43 | 1,695,473 | G | A |
| B-44 | 1,779,939 | G | A |
| B-45 | 1,797,452 | G | A |
| B-46 | 1,801,284 | G | A |
| B-47 | 1,816,679 | G | A |
| B-48 | 1,832,252 | G | A |
| B-49 | 1,843,841 | G | A |
| B-50 | 1,868,285 | G | A |
| B-51 | 1,879,922 | G | A |
| B-52 | 1,892,007 | G | A |
| B-53 | 1,916,016 | G | A |
| B-54 | 1,937,604 | G | A |
| B-55 | 1,947,044 | G | A |
| B-56 | 1,948,411 | G | A |
| B-57 | 1,948,649 | G | A |
| B-58 | 1,967,697 | G | A |
| B-59 | 1,974,137 | G | A |
| B-60 | 2,028,284 | C | T |
| B-61 | 2,050,998 | G | A |
| B-62 | 2,052,708 | G | A |
| 8-63 | 2,054,372 | G | A |
| B-64 | 2,065,568 | G | A |
| B-65 | 2,067,167 | G | A |
| 8-66 | 2,082,577 | G | A |
| B-67 | 2,121,006 | A | G |
| B-68 | 2,149,369 | C | T |
| B-69 | 2,159,680 | G | A |
| B-70 | 2,380,965 | G | A |
| B-71 | 2,477,728 | G | A |
| B-72 | 2,542,800 | G | A |
| B-73 | 2,570,107 | G | A |
| B-74 | 2,647,383 | G | A |
| B-75 | 2,726,248 | C | T |
| B-76 | 2,825,055 | C | T |
| B-77 | 2,837,078 | C | T |
| B-78 | 2,865,322 | C | T |
| 8-79 | 2,872,907 | C | T |
| B-80 | 2,880,351 | C | T |
| B-81 | 2,889,394 | C | T |
| B-82 | 2,906,471 | C | T |
| B-83 | 2,927,044 | C | T |
| B-84 | 2,929,963 | C | T |
| B-85 | 2,940,673 | C | T |
| B-86 | 2,946,285 | C | T |
| B-87 | 2,962,909 | C | T |
| B-88 | 2,975,742 | C | T |
| B-89 | 2,987,052 | C | T |
| B-90 | 3,079,560 | C | T |
| B-91 | 3,083,927 | C | T |
| B-92 | 3,090,163 | C | T |

The "specified mutation" may be one or more mutations selected from mutations shown in Table 1. That is, the L-glutamic acid-producing bacterium may have one or more mutations selected from mutations shown in Table 1.

The L-glutamic acid-producing bacterium, for example, may have one or more mutations selected from mutations of Group A. The L-glutamic acid-producing bacterium, for example, may have one or more mutations selected from mutations of Group B. The L-glutamic acid-producing bacterium, for example, may have one or more mutations selected from mutations of Group A and one or more mutations selected from mutations of Group B. The L-glutamic acid-producing bacterium, for example, may have one or more mutations selected from mutations of Group A and furthermore may have one or more mutations selected from mutations of Group B. The L-glutamic acid-producing bacterium, for example, may have one or more mutations selected from mutations of Group B and furthermore may have one or more mutations selected from mutations of Group A. In other words, "specified mutation" may be, for example, one or more mutations selected from mutations of Group A, one or more mutations selected from mutations of Group B, or any combination of one or more mutations selected from mutations of Group A and one or more mutations selected from mutations of Group B.

The number of mutations selected from mutations of Group A in the L-glutamic acid-producing bacterium may be, for example, one or more, 5 or more, 10 or more, 20 or more, 30 or more, 40 or more, 50 or more, 60 or more, 70 or more, 80 or more, 90 or more, 100 or more, 110 or more, 120 or more, or 130 or more, or may be 135 or less, 130 or less, 120 or less, 110 or less, 100 or less, 90 or less, 80 or less, 70 or less, 60 or less, 50 or less, 40 or less, 30 or less, 20 or less, 10 or less, or 5 or less, or may be any non-contradictory combination thereof. The number of mutations selected from mutations of Group A in the L-glutamic acid-producing bacterium may be, specifically, for example, 1 to 5, 5 to 10, 10 to 20, 20 to 30, 30 to 40, 40 to 50, 50 to 60, 60 to 70, 70 to 80, 80 to 90, 90 to 100, 100 to 110, 110 to 120, 120 to 130, or 130 to 135. The number of mutations selected from mutations of Group A in the L-glutamic acid-producing bacterium may be, in particular, one or more, 50 or more, 100 or more, 120 or more, 130 or more, or 135.

The number of mutations selected from mutations of Group B in the L-glutamic acid-producing bacterium may be, for example, one or more, 5 or more, 10 or more, 20 or more, 30 or more, 40 or more, 50 or more, 60 or more, 70 or more, 80 or more, or 90 or more, or may be 92 or less, 90 or less, 80 or less, 70 or less, 60 or less, 50 or less, 40 or less, 30 or less, 20 or less, 10 or less, or 5 or less, or may be any non-contradictory combination thereof. The number of mutations selected from mutations of Group B in the L-glutamic acid-producing bacterium may be, specifically, for example, 1 to 5, 5 to 10, 10 to 20, 20 to 30, 30 to 40, 40 to 50, 50 to 60, 60 to 70, 70 to 80, 80 to 90, or 90 to 92. The number of mutations selected from mutations of Group B in the L-glutamic acid-producing bacterium may be, in particular, one or more, 30 or more, 60 or more, 70 or more, 80 or more, or 92.

The L-glutamic acid-producing bacterium may have, for example, 50 or more mutations selected from mutations of Group A and 30 or more mutations selected from mutations of Group B. The L-glutamic acid-producing bacterium may have, for example, 100 or more mutations selected from mutations of Group A and 60 or more mutations selected from mutations of Group B. The L-glutamic acid-producing bacterium may have, for example, 120 or more mutations selected from mutations of Group A and 80 or more mutations selected from mutations of Group B. The L-glutamic acid-producing bacterium may have, for example, 135 mutations of Group A and 92 mutations of Group B.

The L-glutamic acid-producing bacterium having "specified mutation" may be particularly a coryneform bacterium. The L-glutamic acid-producing bacterium having "specified mutation" may be further particularly a *Corynebacterium* genus bacterium. The L-glutamic acid-producing bacterium having "specified mutation" may be further particularly *Corynebacterium casei.* The L-glutamic acid-producing bacterium having "specified mutation" may be further particularly a *Corynebacterium casei* JCM 12072-derived modified strain.

Specific examples of the L-glutamic acid-producing bacterium having "specified mutation" include *Corynebacterium casei* RUN5-2-96 strain (NITE ABP-03688). The RUN5-2-96 strain is also referred to as "AJ111891 strain". The RUN5-2-96 strain is a *Corynebacterium casei* JCM 12072-derived modified strain, and has all 135 mutations of Group A. The RUN5-2-96 strain has been originally deposited as an international deposit with the Patent Microorganisms Depositary, National Institute of Technology and Evaluation (independent organization) (NITE NPMD, postal code: 292-0818, address: Room 122, 2-5-8, Kazusakamatari, Kisarazu-shi, Chiba, Japan) on July 7, 2022, and Accession No. NITE ABP-03688 is imparted. The RUN5-2-96 strain is available from, for example, NITE NPMD.

In Table 1, "Position on genome" represents the position of each mutation on a base sequence registered as ACCESSION No. NZ_CP004350.1 on the NCBI (National Center for Biotechnology Information; https://www.ncbi.nlm.nih.gov/). Hereinafter, this base sequence is also referred to as "base sequence of NZ_CP004350.1". The base sequence of NZ_CP004350.1 is the base sequence of the genome of *Corynebacterium casei* JCM 12072 (LMG S-19264), and is published with annotations. The base sequence of NZ_CP004350.1 and the annotation of each position on the genome can be acquired from, for example, NCBI.

Each mutation shown in Table 1 is to be interpreted as the mutation at the position corresponding to the position of each mutation shown in Table 1, on the genome of each bacterium. For example, mutation A-1 is to be interpreted as a mutation at the position corresponding to the 78,486-th position of the base sequence of NZ_CP004350.1, on the genome of each bacterium. The position of each mutation shown in Table 1 is denoted for convenience in order to identify each mutation, and it is not necessary to show any absolute position on the genome of each bacterium. That is, the position of each mutation shown in Table 1 indicates a relative position based on the base sequence of NZ_CP004350.1, and the absolute position may be shifted before or after due to deletion, insertion, or the like of nucleic acid residue(s). For example, when one nucleic acid residue is deleted or inserted at a position at the 5' terminal side relative to the X-th position (X is a positive integer) in the base sequence of NZ_CP004350.1, the original X-th position corresponds to the X-1-th position or the X+1-th position, but the mutation at the original X-th position is regarded as "mutation at the position corresponding to the X-th position of the base sequence of NZ_CP004350.1". The base before mutation shown in Table 1 is denoted for convenience in order to identify each mutation and is not needed to be preserved on the genome of a bacterium before modification. That is, when the genome of a bacterium before modification has no base sequence of NZ_CP004350.1, no base before mutation shown in Table 1 may be preserved. That is, the phrase "introduction of mutation into bacterium" with respect to each mutation shown in Table 1 means that the base (this is any base other than the base after mutation) at the position of each mutation shown in Table 1 on the genome of a bacterium before modification is modified to a base after mutation shown in Table 1. For example, the phrase "introduction of mutation A-1 into bacterium" means that the base (this is C, G, or A) at the position corresponding to the 78,486-th position of the base sequence of NZ_CP004350.1, on the genome of a bacterium before modification, is modified to T.

Which position on the genome of each bacterium is "position corresponding to the position of each mutation shown in Table 1" can be determined by carrying out alignment between the base sequence of the genome of each bacterium and the base sequence of NZ_CP004350.1. Such alignment can be carried out by using, for example, known gene analysis software. Examples of such gene analysis software include DNASIS manufactured by Hitachi Solutions, Ltd. and GENETYX manufactured by GENETYX CORPORATION (Elizabeth C. Tyler et al., Computers and Biomedical Research, 24(1), 72-96, 1991; Barton GJ et al., Journal of molecular biology, 198(2), 327-37. 1987).

The phrase "bacterium having mutation" with respect to each mutation shown in Table 1 means that the base at the position of such a mutation on the genome of such a bacterium is the base after mutation shown in Table 1, and does not necessarily mean that such a bacterium is obtained by introduction of such a mutation. In other words, "bacterium having mutation" with respect to each mutation shown in Table 1 may be naturally a bacterium where the base at the position of such a mutation is the base after mutation shown in Table 1, or may be naturally a bacterium obtained by modifying a bacterium where the base at the position of such a mutation is not the base after mutation shown in Table 1. For example, the phrase "bacterium having mutation A-1" means that the position (the position corresponding to the 78,486-th position of the base sequence of NZ_CP004350.1) of mutation A-1 on the genome of such a bacterium is T, and does not necessarily mean that such a bacterium is obtained by introduction of mutation A-1. In other words, for example, "bacterium having mutation A-1" may be naturally a bacterium where the base at the position of mutation A-1 (the position corresponding to the 78,486-th position of the base sequence of NZ_CP004350.1) on the genome is T, or may be naturally a bacterium obtained by modifying a bacterium where the base at the position of mutation A-1 on the genome is not T. The L-glutamic acid-producing bacterium having "specified mutation" may be particularly one obtained by introduction of one portion or the whole of "specified mutation".

The L-glutamic acid-producing bacterium having "specified mutation" can be obtained by, for example, introduction of "specified mutation" into a bacterium having no "specified mutation". The L-glutamic acid-producing bacterium having "specified mutation" can be obtained by, for example, introducing the balance of "specified mutation" to a bacterium having one portion of "specified mutation".

Variation introduction can be carried out by, for example, a known procedure. For example, an objective mutation can be introduced to an objective position on a genome by a site-specific mutation method. Examples of the site-specific mutation method include methods with PCR (Higuchi, R., 61, in PCR technology, Erlich, H. A. Eds., Stockton press (1989); Carter, P., Meth. in Enzymol., 154, 382 (1987)) and methods with phage (Kramer, W. and Frits, H. J., Meth. in Enzymol., 154, 350 (1987); Kunkel, T. A. et al., Meth. in Enzymol., 154, 367 (1987)).

Each mutation shown in Table 1 may be a mutation which enhances an L-glutamic acid-producing ability of a bacterium. Each mutation shown in Table 1 may be particularly a mutation which enhances an L-glutamic acid-producing ability of a bacterium as compared with a case where the base at the position of each mutation is the base before mutation shown in Table 1.

Each mutation shown in Table 1 may be, for example, a mutation of a gene (which here means a gene-coding region), a mutation in an expression-regulating region of a gene such as a promoter, or a mutation in an intergenic region. Which region (for example, a gene, an expression-regulating region of a gene, or an intergenic region) each mutation shown in Table 1 is located in can be confirmed by, for example, seeing annotations at the position of such each mutation and the base sequence of NZ_CP004350.1.

When each mutation shown in Table 1 is a mutation of a gene, such each mutation may provide, for example, the change (for example, increase or decrease) in expression of such a gene. When each mutation shown in Table 1 is a mutation of a gene, such each mutation may provide, for example, the change (for example, increase or decrease) in activity of protein to be encoded by such a gene.

When each mutation shown in Table 1 is a mutation of an expression-regulating region of a gene, such each mutation may provide, for example, the change (for example, increase or decrease) in expression of such a gene.

The change (for example, increase or decrease) in expression of a gene may provide, for example, the change (for example, increase or decrease) in activity of protein to be encoded by such a gene.

The L-glutamic acid-producing bacterium having "specified mutation" may or may not have other modification than "specified mutation" as long as it has an L-glutamic acid-producing ability. Examples of modification of a mutation other than "specified mutation" include known modification for impartment or enhancement of an L-glutamic acid-producing ability. Examples of modification of a mutation other than "specified mutation" include modification of a mutation not selected as "specified mutation" of mutations shown in Table 1. The L-glutamic acid-producing bacterium having "specified mutation" may have, for example, an L-glutamic acid-producing ability depending on "specified mutation", or an L-glutamic acid-producing ability depending on a combination of "specified mutation" and modification of other mutation.

The L-glutamic acid-producing bacterium having "specified mutation" may have, for example, a remarkably high L-glutamic acid-producing ability than that of *Corynebacterium casei* JCM 12072. The phrase "remarkably high L-glutamic acid-producing ability than that of *Corynebacterium casei* JCM 12072" may mean, for example, an ability to produce and accumulate L-glutamic acid in an amount twice or more, 3 times or more, 5 times or more, or 7 times or more that of JCM 12072, in a medium, in culture under appropriate culture conditions. The L-glutamic acid-producing bacterium having "specified mutation" may have, for example, an L-glutamic acid-producing ability equivalent to or more than that of a *Corynebacterium casei* RUN5-2-96 strain (NITE ABP-03688). The phrase "L-glutamic acid-producing ability equivalent to or more than that of a *Corynebacterium casei* RUN5-2-96 strain (NITE ABP-03688)" may mean, for example, an ability to produce L-glutamic acid in an amount of 80% or more, 90% or more, 95% or more, or 100% or more relative to the RUN5-2-96 strain, in a medium, in culture under appropriate culture conditions. Examples of appropriate culture conditions include culture conditions (i.e., conditions of shaking and culture at 30°C for 48 hours in a 500 µL evaluation medium (Table 4) stuck in a 96 deep well plate) for measuring the amount of L-glutamic acid produced, as mentioned in Example (1-2) later.

The base sequence of the genome of the L-glutamic acid-producing bacterium having "specified mutation" may be 95% or more, 96% or more, 97% or more, 98% or more, 99% or more, 99.5% or more, 99.7% or more, 99.9% or more, 99.95% or more, 99.97% or more, or 99.99% or more identical to that of, for example, a *Corynebacterium casei* JCM 12072 or *Corynebacterium casei* RUN5-2-96 strain (NITE ABP-03688). The term "identity" between base sequences means the identity between base sequences, calculated with Scoring Parameters of default settings (Match/Mismatch scores = 1, -2; Gap Costs = Linear), by blastn.

The active ingredient here used may be a commercially available product or may be produced and obtained as appropriate. The method of producing the active ingredient is not particularly limited.

The active ingredient can be produced by, for example, culturing a gram-positive bacterium in a medium. That is, the method of producing the active ingredient may include, for example, a step of culturing a gram-positive bacterium in a medium. Such a step is also referred to as "culture step". The culture step may be specifically a step of culturing a gram-positive bacterium in a medium to provide a culture product.

The medium used is not particularly limited as long as the gram-positive bacterium can be cultivated. The medium used can be, for example, a common medium for use in the culture of a bacterium such as coryneform bacterium. The medium may, if necessary, contain any medium ingredient such as a carbon source, a nitrogen source, a phosphoric acid source, a sulfur source, and other various organic ingredients and inorganic ingredients. The type and concentration of such a medium ingredient may be set as appropriate depending on various conditions such as the type of the gram-positive bacterium used. For example, medium compositions mentioned in documents published about objective substance production with bacteria (WO2015/060391, WO2018/030507, WO2015/060391, and the like) can be seen as specific medium compositions.

The carbon source is not particularly limited as long as the gram-positive bacterium can be assimilated. Specific examples of the carbon source include sugars such as glucose, fructose, sucrose, lactose, galactose, xylose, arabinose, blackstrap, hydrolysates of starch, and hydrolysates of biomass, organic acids such as acetic acid, citric acid, succinic acid, and gluconic acid, alcohols such as ethanol, glycerol, and crude glycerol, and fatty acids. As the carbon source, one carbon source may be used, or two or more carbon sources may be used in combination.

Specific examples of the nitrogen source include ammonium salts such as ammonium sulfate, ammonium chloride, and ammonium phosphate, organic nitrogen sources such as peptone, yeast extracts, meat extracts, and plant protein hydrolysates (HVP; for example, degradation products of soybean protein, soybean sauces, and pea soy sauces), ammonia, and urea. Ammonia gas or ammonia water for use in the pH adjustment may also be used as the nitrogen source. As the nitrogen source, one nitrogen source may be used, or two or more nitrogen sources may be used in combination.

Specific examples of the phosphoric acid source include phosphates such as potassium dihydrogen phosphate and dipotassium hydrogen phosphate, and phosphoric acid polymers such as pyrophosphoric acid. As the phosphoric acid source, one phosphoric acid source may be used, or two or more phosphoric acid sources may be used in combination.

Specific examples of the sulfur source include inorganic sulfur compounds such as sulfate, thiosulfate and sulfite, and sulfur-containing amino acids such as cysteine, cystine and glutathione. As the sulfur source, one sulfur source may be used, or two or more sulfur sources may be used in combination.

Specific examples of other various organic ingredients and inorganic ingredients include inorganic salts such as sodium chloride and potassium chloride; trace metals such as iron, manganese, magnesium, and calcium; vitamins such as vitamin B1, vitamin B2, vitamin B6, nicotinic acid, nicotinic-acid amide and vitamin B 12; amino acids; nucleic acids; and organic ingredients containing such ingredients, such as peptone, casamino acids, yeast extracts, and plant protein hydrolysates (HVP; for example, degradation products of soybean protein, soybean sauces, and pea soy sauces). Examples of other various organic ingredients and inorganic ingredients include defoamers, osmotic pressure adjusting substances for media, and osmotic pressure compensating substances. Examples of defoamers include silicone-based defoamers (oil-type, solution-type, oil compound-type, emulsion-type, self-emulsification-type, and the like), alcohol-based defoamers, oil-based defoamers, polyether-based defoamers, and vegetable oils (cotton seed oil, linseed oil, soybean oil, olive oil, castor oil, coconut oil, and the like). As such a defoamer, a deformer in any form such as a liquid, paste, solid, powder, emulsion or wax form can be used. Examples of osmotic pressure adjusting substances for media include salts such as sodium chloride and potassium chloride, and microorganism-unassimilable polysaccharides (sorbitol, dextrin, and the like). Examples of osmotic pressure compensating substances include potassium ions, betaine (glycine betaine), glutamic acid, and trehalose. As these other various organic ingredients, one ingredient may be used, or two or more ingredients may be used in combination.

Examples of the medium ingredient include raw materials of food. That is, the medium may contain a raw material of food. Culture of the gram-positive bacterium with a medium containing a raw material of food is also referred to as "fermentation of gram-positive bacterium with raw material of food". That is, the active ingredient may also be, for example, a fermented product of a raw material of food with the gram-positive bacterium. The raw material of food may be used singly or may be combined as appropriate with any other medium ingredient and then used, in the medium ingredient.

Examples of the raw material of food (i.e., the raw material of food, used for culture of the gram-positive bacterium), used as the medium ingredient, include raw materials of food, described later. Such a raw material of food, used as the medium ingredient, can be selected independently from the raw material of food used in the method of the present invention. Such a raw material of food, used as the medium ingredient, may or may not be the same as the raw material of food used in the method of the present invention. As such a raw material of food, used as the medium ingredient, one raw material may be used, or two or more raw materials may be used in combination.

Specific examples of such a raw material of food, used as the medium ingredient, include *Solanaceae* family plants. Examples of *Solanaceae* family plants include *Solanum* genus plants and *Capsicum* genus plants. Examples of *Solanum* genus plants include tomatoes and eggplants. Examples of *Capsicum* genus plants include bell peppers, paprika, sweet green peppers, and hot peppers. Examples of bell peppers include green bell peppers and red bell peppers. Examples of *Solanum* genus plants particularly include tomatoes. For *Solanaceae* family plants, one plant may be used, or two or more plants may be used in combination.

Perishable items (specifically, perishable items of edible parts of fruits or the like) of *Solanaceae* family plants may be, for example, directly cultured, or processed as appropriate and then cultured. Examples of such processing include cutting, crushing, pureeing, squeezing, fractionation, dilution, concentration, drying, and heating. Such processing may be carried out singly or in combination as appropriate. Such processing may remove, for example, peels and/or seeds. Examples of processed products of *Solanaceae* family plants include juices, purees, and pastes of *Solanaceae* family plants. That is, examples of processed products of, for example, tomatoes include tomato juices, tomato purees, and tomato pastes. The term "juice" of *Solanaceae* family plants, such as tomato juices, may mean, for example, one obtained by crushing and squeezing or pureeing of edible parts of fruits or the like, having a salt-free soluble solid content of less than 8% (w/w) (for example, 4.5% (w/w) or more and less than 24% (w/w)). The term "puree" of *Solanaceae* family plants, such as tomato purees, may mean, for example, a concentrated product of juice of *Solanaceae* family plants, having a salt-free soluble solid content of 8% (w/w) or more and less than 24% (w/w). The term "paste" of *Solanaceae* family plants, such as tomato pastes, may mean, for example, a concentrated product of juice of *Solanaceae* family plants, having a salt-free soluble solid content of 24% (w/w) or more. Additives such as sodium chloride may or may not be added to processed products of *Solanaceae* family plants. Such processed products of *Solanaceae* family plants may be concentrated and reduced so as to have the above-illustrated salt-free soluble solid content.

Culture conditions are not particularly limited as long as the gram-positive bacterium can be cultivated. Culture can be performed, for example, under usual conditions for use in the culture of a bacterium such as coryneform bacterium. Culture conditions may be set as appropriate depending on various conditions such as the type of the gram-positive bacterium used. Culture conditions mentioned in documents published about objective substance production with bacteria (WO2015/060391, WO2018/030507, WO2015/060391, and the like) can be seen as specific culture conditions.

Culture may be carried out, for example, with a liquid medium under aerobic conditions or microaerophilic conditions. The term "aerobic conditions" may mean conditions where the concentration of dissolved oxygen in the medium is 0.33 ppm or more, or 1.5 ppm or more. The term "microaerophilic conditions" may mean conditions where the concentration of dissolved oxygen in the medium is 0.35 ppm or less. The concentration of dissolved oxygen in the medium under microaerophilic conditions may be, 0.30 ppm or less, 0.25 ppm or less, 0.20 ppm or less, 0.15 ppm or less, 0.10 ppm or less, or 0.05 ppm or less. Culture can be performed by, for example, aeration culture or shaking culture. The pH of the medium may be, for example, 3 to 10, or 4.0 to 9.5. The pH of the medium can be, if necessary, adjusted during culture. The pH of the medium can be adjusted with various alkaline or acidic substances, such as ammonia gas, ammonia water, sodium carbonate, sodium bicarbonate, potassium carbonate, potassium bicarbonate, magnesium carbonate, sodium hydroxide, potassium hydroxide, calcium hydroxide, and magnesium hydroxide. The culture temperature may be, for example, 20 to 45°C, or 25°C to 37°C. The culture period may be, for example, 10 hours to 120 hours. Culture may be continued until, for example, the carbon source in the medium is consumed or the activity of the gram-positive bacterium disappears.

The gram-positive bacterium is cultured under such conditions, thereby providing a culture product of the gram-positive bacterium (specifically, a culture product comprising a cell of the gram-positive bacterium).

The cell may be directly used as the active ingredient, in the form of being contained in the culture product (specifically medium), or may be recovered from the culture product (specifically medium) and then used as the active ingredient. That is, the method of producing the active ingredient may include, for example, a step of recovering the cell from the culture product (specifically medium). The cell can be recovered by, for example, a known procedure. Examples of such a procedure include spontaneous precipitation, centrifugation, and filtration. The cell may also be subjected as appropriate to treatment and then used as the active ingredient. That is, the method of producing the active ingredient may include, for example, a step of subjecting the cell to treatment. Examples of the treatment include concentration, drying, and heating. All of concentration, drying, and heating can be performed by, for example, known procedures. Examples of such a known drying procedure include spray drying and freeze drying. The heating temperature may be, for example, 60°C or more, 70°C or more, 80°C or more, 90°C or more, 100°C or more, 110°C or more, or 120°C or more. That is, the active ingredient (specifically, a cell of the gram-positive bacterium) is, for example, a culture product of the gram-positive bacterium, a cell recovered from the culture product, and any treated product thereof. That is, the cell may be used as the active ingredient, for example, in the form of a culture product of the gram-positive bacterium, a cell recovered from the culture product, any treated product thereof, or any combination thereof. In other words, the active ingredient (specifically, a cell of the gram-positive bacterium) is, for example, a cell contained in a culture product of the gram-positive bacterium, a cell recovered from the culture product, or a cell contained in any treated product thereof. That is, the cell may be used as the active ingredient, for example, in the form of a cell contained in a culture product of the gram-positive bacterium, a cell recovered from the culture product, a cell contained in any treated product thereof, or any combination thereof. The treated product is, for example, one obtained by subjecting the cell (for example, the cell contained in the culture product or the cell recovered from the culture product) to treatment. The cell may be a living cell, may be a dead cell, or may be any combination thereof. The cell of the gram-positive bacterium contains a cell wall of the gram-positive bacterium.

A fragment of the cellcell can be prepared from the cell. For example, the cell can be crushed, thereby preparing a cell-crushed product containing a fragment of the cell. That is, the method of producing the active ingredient may include, for example, a step of crushing the cell. Crushing of the cell can be performed by, for example, a known procedure. Examples of such a procedure include ultrasonic crushing. A fragment of the cellcell may be used as the active ingredient as it contained in the cell-crushed product, or may be recovered from the cell-crushed product and then used as the active ingredient. That is, the method of producing the active ingredient may include, for example, a step of recovering a fragment of the cellcell from the cell-crushed product. Recovery of a fragment of the cellcell can be performed by, for example, a known procedure. Examples of such a procedure include spontaneous precipitation, centrifugation, and filtration. A fragment of the cellcell may also be subjected as appropriate to treatment and then used as the active ingredient. That is, the method of producing the active ingredient may include, for example, a step of subjecting a fragment of the cellcell to treatment. Examples of the treatment include concentration, drying, and heating. All of concentration, drying, and heating can be performed by, for example, known procedures. Examples of such a known drying procedure include spray drying and freeze drying. The heating temperature may be, for example, 60°C or more, 70°C or more, 80°C or more, 90°C or more, 100°C or more, 110°C or more, or 120°C or more. That is, examples of the active ingredient (specifically, a fragment of the cellcell of the gram-positive bacterium) include a crushed product of the cell of the gram-positive bacterium, a fragment of the cell, recovered from the crushed product, and any treated product thereof. That is, a fragment of the cellcell may be used as the active ingredient, for example, in the form of a crushed product of the cell of the gram-positive bacterium, a fragment of the cell, recovered from the crushed product, any treated product thereof, or any combination thereof. In other words, examples of the active ingredient (specifically, a fragment of the cellcell of the gram-positive bacterium) include a fragment of the cell, contained in a crushed product of the cell of the gram-positive bacterium, a fragment of the cell, recovered from the crushed product, and a fragment of the cell, contained in any treated product thereof. That is, a fragment of the cellcell may be used as the active ingredient, for example, in the form of a fragment of the cell, contained in a crushed product of the cell of the gram-positive bacterium, a fragment of the cell, recovered from the crushed product, a fragment of the cell, contained in any treated product thereof, or any combination thereof. Examples of the treated product include one obtained by subjecting a fragment of the cellcell (for example, a fragment of the cellcell contained in the crushed product or a fragment of the cellcell recovered from the crushed product) to treatment. A fragment of the cellcell of the gram-positive bacterium (for example, a crushed product of the cell of the gram-positive bacterium, a fragment of the cell, recovered from the crushed product, or any treated product thereof) contains a cell wall of the gram-positive bacterium.

As mentioned above, the cell of the gram-positive bacterium, and a fragment thereof may be subjected to treatment such as heating. That is, the active ingredient may be subjected to treatment such as heating. That is, the method of producing the active ingredient may include, for example, a step of subjecting the active ingredient, for example, the cell of the gram-positive bacterium, or a fragment thereof, to treatment such as heating. The active ingredient may be particularly heat-treated. "Subjecting the active ingredient to treatment" also includes the case of subjecting an active ingredient of any aspect, to treatment, unless otherwise noted, and, in other words, also includes the case of subjecting the active ingredient by itself or a fraction containing it, to treatment. For example, "heat-treating the cell of the gram-positive bacterium" includes the case of heat-treating any cell-containing fraction of the gram-positive bacterium, for example, a culture product of the gram-positive bacterium, a cell recovered from the culture product, or any treated product thereof, unless otherwise noted. The method of producing the active ingredient may include, for example, a step of subjecting a culture product of the gram-positive bacterium to treatment such as heating. "Subjecting the culture product to treatment" also includes the case of subjecting a culture product of any aspect, to treatment, unless otherwise noted, and, in other words, also includes the case of subjecting the culture product by itself or any fraction prepared therefrom to treatment. The culture product may be subjected to treatment such as heating, thereby providing an active ingredient subjected to treatment such as heating.

When the gram-positive bacterium has an objective substance-producing ability, an objective substance may be produced by culture of the gram-positive bacterium. That is, a culture product of the gram-positive bacterium may contain an objective substance.

The objective substance may be recovered as appropriate from the culture product. The objective substance may also be recovered as an appropriate fraction containing the objective substance. Examples of such a fraction include a culture supernatant. The objective substance may also be further separated and purified from the above fraction. For example, the cell of the gram-positive bacterium may be separated from the culture product to provide a culture supernatant and the objective substance may be recovered from the culture supernatant. Herein, "recovery of obj ective substance" may include removal of impurities from a fraction containing the objective substance. Recovery of the objective substance can be performed by, for example, a known procedure for use in the separation and purification of a compound. Examples of such a procedure include ion-exchange resin methods, membrane treatment methods, precipitation methods, extraction methods, distillation methods, crystallization methods, and treatment with activated carbon. The procedure of recovery of the objective substance can be selected as appropriate depending on various conditions, for example, the type of the objective substance. Such procedures may be used singly or in combination as appropriate. The objective substance recovered may include, in addition to the objective substance, for example, any other ingredient such as the cell of the gram-positive bacterium, medium ingredient, moisture, and/or a metabolic by-product of the gram-positive bacterium. The purity of the objective substance recovered may be, for example, 30% (w/w) or more, 50% (w/w) or more, 70% (w/w) or more, 80% (w/w) or more, 90% (w/w) or more, or 95% (w/w) or more. The application of the objective substance recovered is not particularly limited. The objective substance recovered may be used separately from the active ingredient, or may be used in combination with the active ingredient. The objective substance may be, for example, prepared as a fermented seasoning containing the objective substance, and used in combination with the active ingredient.

Alternatively, a fraction containing both the objective substance and a cell wall of the gram-positive bacterium may be used as the active ingredient. For example, a culture product containing both the objective substance and the cell of the gram-positive bacterium may be used directly, or may be, for example, dried as appropriate, and then used as the active ingredient.

The active ingredient may be, for example, used in combination with any other ingredient (i.e., any other ingredient than the active ingredient). Examples of such any other ingredient include the objective substance and a culture product of the gram-positive bacterium. The active ingredient may be used in combination with any other ingredient, resulting in an enhanced flavor improvement effect as compared with a case where the active ingredient is used singly. Specifically, for example, the active ingredient may be used in combination with L-glutamic acid and/or a culture product of the gram-positive bacterium, resulting in an enhanced flavor improvement effect as compared with a case where the active ingredient is used singly. For example, the active ingredient may be used in combination with L-glutamic acid, resulting in enhanced umami of food as compared with a case where the active ingredient is used singly. Unless otherwise noted, "active ingredient being used in combination with L-glutamic acid" also includes the case of the active ingredient used in combination with L-glutamic acid in any form, and, in other words, also includes the case of the active ingredient used in combination with L-glutamic acid by itself or a L-glutamic acid-containing fraction (for example, a culture product containing L-glutamic acid).

The objective substance is as described above. As the objective substance, a commercially available product may be used, or one obtained by being produced as appropriate may be used. The objective substance can be produced by, for example, chemical synthesis, enzymatic reaction, a fermentation method, an extraction method, or any combination thereof. The objective substance can be specifically, for example, produced by a fermentation method using a microorganism having an objective substance-producing ability. The microorganism having an objective substance-producing ability may or may not be the same as the gram-positive bacterium from which the active ingredient is derived. The objective substance may be, for example, produced along with production of the active ingredient, or separately produced from the active ingredient. The objective substance may or may not be purified to a desired degree. That is, as the objective substance, a purified product may be used or a material containing the objective substance may be used. The term "material containing the objective substance" means a material where the amount of the objective substance contained is 0.1% (w/w) or more. Specific examples of the material containing the objective substance include fermented products such as culture products, bacterial cells, and culture supernatants obtained by culturing microorganisms having an objective substance-producing ability, and processed products thereof. Examples of such processed products include those obtained by subjecting materials of the above fermented products to treatment such as heating, concentration, dilution, drying, fractionation, extraction, and/or purification. The amount of the objective substance contained in the material containing the objective substance may be, for example, 1% (w/w) or more, 3% (w/w) or more, 5% (w/w) or more, 10% (w/w) or more, 30% (w/w) or more, 50% (w/w) or more, 70% (w/w) or more, 90% (w/w) or more, or 95% (w/w) or more. Herein, "active ingredient being used in combination with objective substance", is not limited to any case where the active ingredient and the objective substance individually obtained are used in combination, and also includes a case where the active ingredient and the objective substance collectively obtained are used. Examples of the case where the active ingredient and the objective substance collectively obtained are used include a case where a culture product containing both the objective substance and the cell of the gram-positive bacterium is dried directly, or dried as appropriate and then used as the active ingredient.

A culture product of the gram-positive bacterium, as any other ingredient (i.e., any other ingredient than the active ingredient), can be obtained by culture of the gram-positive bacterium. Culture of the gram-positive bacterium is as mentioned above. The gram-positive bacterium from which the culture product as any other ingredient is derived may or may not be the same as the gram-positive bacterium from which the active ingredient is derived. The culture product as any other ingredient may be, for example, produced along with production of the active ingredient, or separately produced from the active ingredient. The culture product as any other ingredient may be, for example, used directly, or subjected to treatment such as fractionation (separation of the bacterial cell, separation of the objective substance, and the like), concentration, drying, and/or heating, and then used as an additional ingredient. Such fractionation may separate (i.e., may remove the culture product) the cell at a proportion of, for example, 90% or more, 95% or more, 97% or more, or 99% or more based on the total number of bacteria. The culture product as any other ingredient may or may not contain the cell of the gram-positive bacterium. When the culture product as any other ingredient contains the cell of the gram-positive bacterium, the cell of the gram-positive bacterium contained in the culture product may be regarded as the active ingredient. The culture product as any other ingredient may or may not contain the objective substance (for example, L-glutamic acid). Herein, "combination use of active ingredient and culture product of gram-positive bacterium", is not limited to any case where the active ingredient and the culture product individually obtained are used in combination, and also includes a case where the active ingredient and the culture product collectively obtained are used. Examples of the case where the active ingredient and the culture product collectively obtained are used include a case where a culture product containing the cell of the gram-positive bacterium is used directly, or dried as appropriate and then used as the active ingredient.

### <2> Composition of Present Invention

The composition of the present invention is a composition comprising an active ingredient.

That is, the composition of the present invention is a composition comprising the following ingredient (A):
(A) a cell wall-containing fraction of a gram-positive bacterium.

The composition of the present invention can be used to improve the flavor of food, i.e., obtain a flavor improvement effect. Thus, the composition of the present invention can be used for improvement of the flavor of food. That is, the composition of the present invention may be, for example, a composition for improvement of the flavor of food. Such improvement of the flavor may be, for example, enhancement of spiciness and/or impartment of a rich taste. Such improvement of the flavor may be particularly enhancement of pungency of a spice.

The composition of the present invention can be used to produce food with improved flavor. Thus, the composition of the present invention may be used for production of food (specifically, production of food with improved flavor). That is, the composition of the present invention may be, for example, a composition for production of food (specifically, production of food with improved flavor).

The composition of the present invention may be, for example, a seasoning. The composition of the present invention may be, specifically, for example, a seasoning for improvement of the flavor of food, or a seasoning for production of food (specifically, production of food with improved flavor).

The composition of the present invention, by itself, may or may not have an improved flavor. For example, when the composition of the present invention comprises a spice, the composition of the present invention, by itself, may have enhanced spiciness (specifically, enhanced spiciness as compared with the case of no active ingredient contained).

The composition of the present invention may be used for improvement of the flavor or production of food in an aspect described with respect to the method of the present invention as described later.

The composition of the present invention may be composed of an active ingredient, or may comprise any other ingredient than an active ingredient. In one aspect, a composition composed of an active ingredient may be excluded from the composition of the present invention. As any other ingredient than the active ingredient, one ingredient may be used, or two or more ingredients may be used in combination.

The active ingredient compriseed in the composition of the present invention may be produced by the above method of producing the active ingredient. Thus, the method of producing the composition of the present invention may include a step of producing the active ingredient by the above method of producing the active ingredient. The step of producing the active ingredient in the method of producing the composition of the present invention may be specifically, a step of culturing a gram-positive bacterium in a medium to provide a culture product.

Such any other ingredient than the active ingredient is not particularly limited as long as the flavor improvement effect is not impaired (i.e., the flavor improvement effect by the active ingredient is obtained). Such any other ingredient than the active ingredient can be selected as appropriate, for example, depending on various conditions such as the type of food. Examples of such any other ingredient than the active ingredient include ingredients mixed with food or pharmaceuticals.

Specific examples of such any other ingredient than the active ingredient include ingredients that are effective for production of food. Examples of ingredients that are effective for production of food include raw materials of food, described later. Such any other ingredient than the active ingredient particularly includes a culture product of a gram-positive bacterium, an objective substance, and a spice. That is, the composition of the present invention may be, for example, a composition (for example, seasoning) comprising the active ingredient and a culture product of a gram-positive bacterium, a composition (for example, seasoning) comprising the active ingredient and an objective substance, a composition (for example, seasoning) comprising the active ingredient and a spice, a composition (for example, seasoning) comprising the active ingredient, a culture product of a gram-positive bacterium, and a spice, a composition (for example, seasoning) comprising the active ingredient, an objective substance, and a spice, or a composition (for example, seasoning) comprising the active ingredient, a culture product of a gram-positive bacterium, an objective substance, and a spice.

The objective substance is as described above. Herein, "composition comprising active ingredient and objective substance" is not limited to any case where the active ingredient and the objective substance individually obtained are comprised in the composition, and also includes a case where the active ingredient and the objective substance collectively obtained are comprised in the composition. Examples of the case where the active ingredient and the objective substance collectively obtained are comprised in the composition include a case where a culture product containing both the objective substance and a cell of the gram-positive bacterium is directly comprised in the composition, or is dried as appropriate and then comprised in the composition.

The culture product of the gram-positive bacterium, as any other ingredient than the active ingredient, is as described above. Herein, "composition comprising active ingredient and culture product of gram-positive bacterium", is not limited to any case where the active ingredient and the culture product individually obtained are comprised in the composition, and also includes a case where the active ingredient and the culture product collectively obtained are comprised in the composition. Examples of the case where the active ingredient and the culture product collectively obtained are comprised in the composition include a case where a culture product containing the cell of the gram-positive bacterium is comprised in the composition, or dried as appropriate and then comprised in the composition.

The term "spice" may mean one that corresponds to leaves, stalks, cortexes, roots, flowers, buds, seeds, fruits, or fruit skins of plants, and which is to be used for the purpose of imparting a special flavor to food. The term "spice" may include one so-called "herb". Examples of the spice include spices of the *Lauraceae* family (laurel, cinnamon, and the like), spices of the *Piperaceae* family (black pepper, white pepper, and the like), spices of the *Lamiaceae* family (thyme, sage, basil, oregano, marjoram, rosemary, mint, and the like), spices of the *Apiaceae* family (fennel (*Foeniculum vulgare*), caraway, cumin, coriander, parsley, Italian parsley, celery, celery seed, dill, and the like), spices of the *Solanaceae* family (chili pepper, cayenne pepper, and the like), spices of the *Myristicaceae* family (nutmeg, mace, and the like), spices of the *Alliaceae* family (garlic powder, onion powder, and the like), spices of the *Myrtaceae* family (all spice, clove, and the like), spices of the *Schisandraceae* family (star anise, and the like), spices of the *Fabaceae* family (fenugreek and the like), spices of the *Polygonaceae* family (polygonum and the like), spices of the *Brassicaceae* family (garden cress, wasabi, Japanese mustard, and the like), spices of the *Zingiberaceae* family (cardamom, turmeric, ginger, and the like), and spices of the *Rutaceae* family (Japanese pepper and the like). Examples of the spice particularly include those having pungency, such as spices of the *Piperaceae* family, spices of the *Solanaceae* family, spices of the *Brassicaceae* family, and spices of the *Rutaceae* family. In the present invention, allin and onion subjected to cooking, as fresh vegetables in a fresh state, may be excluded from the spice, according to common conventions. In the present invention, one spice may be used, or two or more spices may be used in combination.

The composition of the present invention can be produced, for example, by mixing the active ingredient and any other ingredient as appropriate.

The composition of the present invention may be, for example, formulated as appropriate. In formulation, additives may be used as appropriate. Examples of the additives include excipients, binders, disintegrants, lubricants, stabilizers, correctives, diluents, surfactants, and solvents. The additives can be selected as appropriate, for example, depending on various conditions such as the shape of the composition of the present invention.

The form of the composition of the present invention is not particularly limited. For example, the composition of the present invention may be in any form, such as powder, flake, tablet, paste, or liquid.

The amounts of ingredients (i.e., active ingredient and any other ingredient) and the amount ratio therebetween comprised in the composition of the present invention are not particularly limited as long as the flavor improvement effect is obtained. The amounts of ingredients and the amount ratio therebetween comprised in the composition of the present invention can be set as appropriate depending on various conditions such as the mode of use of the composition of the present invention.

The amount of the active ingredient comprised in the composition of the present invention is more than 0% (w/w) and 100% (w/w) or less. The amount of the active ingredient comprised in the composition of the present invention, for example, converted into the dry mass of the cell of the gram-positive bacterium, may be 0.1% (w/w) or more, 0.2% (w/w) or more, 0.5% (w/w) or more, 1% (w/w) or more, 2% (w/w) or more, 5% (w/w) or more, 10% (w/w) or more, or 20% (w/w) or more, or may be 100% (w/w) or less, less than 100% (w/w), 99.9% (w/w) or less, 90% (w/w) or less, 50% (w/w) or less, 20% (w/w) or less, 10% (w/w) or less, 5% (w/w) or less, 2% (w/w) or less, or 1% (w/w) or less, or may be any non-contradictory combination thereof. The amount of the active ingredient comprised in the composition of the present invention, specifically, for example, converted into the dry mass of the cell of the gram-positive bacterium, may be 0.2 to 99.9% (w/w), 0.2 to 90% (w/w), 0.5 to 50% (w/w), or 1 to 20% (w/w). The dry mass of the cell may also be referred to as "dry cell weight (Dry Cell Weight; DCW)".

The amount of the active ingredient can be measured as the value obtained by conversion into the dry mass of the cell of the gram-positive bacterium, for example, according to a weight method, a packed volume method, a turbidimetric method, a cell counting method, or an indirect measurement method described in 57 to 60 pages of "Introduction to Microbiology 7-Microorganism Fermentation Engineering, edited by Hisaharu Taguchi and Shiro Nagai, Kyoritsu Shuppan Co., Ltd., 1985". That is, the dry cell weight of a gram-positive bacterium contained in a subject (for example, composition or food) can be determined by, for example, separating a cell of the gram-positive bacterium from the subject and drying the cell, and measuring the weight of the resulting dry product. The dry cell weight of a gram-positive bacterium contained in a subject (for example, composition or food) can be determined by, specifically, for example, dispersing the subject in water, separating the cell by a solid-liquid separation procedure such as centrifugation or filtration, performing, if necessary, washing such as washing with water and a solid-liquid separation procedure such as centrifugation or filtration once or multiple times, performing drying by a drying procedure such as drying under reduced pressure, and measuring the weight of the resulting dry product. The dry cell weight of the gram-positive bacterium may also be indirectly calculated by conversion from a parameter reflecting the dry cell weight of the gram-positive bacterium. In other words, the phrase "dry cell weight of gram-positive bacterium" may also mean one thus indirectly calculated. Examples of the parameter reflecting the dry cell weight of the gram-positive bacterium include the bacterial count of the gram-positive bacterium. That is, for example, correlation between the dry cell weight and the bacterial count of a selected gram-positive bacterium may be calculated, and the dry cell weight may be calculated from the bacterial count, based on the correlation. When the active ingredient is used in other form than the cell (for example, a fragment form of the cell), the dry cell weight of the gram-positive bacterium may be calculated from the dry mass of a cell wall of the gram-positive bacterium.

When the composition of the present invention contains a spice, the amount of the spice comprised in the composition of the present invention may be, for example, 0.1% (w/w) or more, 0.2% (w/w) or more, 0.5% (w/w) or more, 1% (w/w) or more, 2% (w/w) or more, 5% (w/w) or more, 10% (w/w) or more, or 20% (w/w) or more, may be 90% (w/w) or less, 50% (w/w) or less, 20% (w/w) or less, 10% (w/w) or less, 5% (w/w) or less, 2% (w/w) or less, or 1% (w/w) or less, or may be any non-contradictory combination thereof. The amount of the spice comprised in the composition of the present invention may be, specifically, for example, 0.2 to 90% (w/w), 0.5 to 50% (w/w), or 1 to 20% (w/w).

When the composition of the present invention contains a spice, the amount of the spice comprised in the composition of the present invention, based on 1 part by weight of the active ingredient comprised in the composition of the present invention (converted into the dry mass of the cell of the gram-positive bacterium), may be, for example, 0.2 parts by weight or more, 0.5 parts by weight or more, 1 part by weight or more, 2 parts by weight or more, 5 parts by weight or more, 10 parts by weight or more, 20 parts by weight or more, 50 parts by weight or more, or 100 parts by weight or more, or may be 1000 parts by weight or less, 500 parts by weight or less, 200 parts by weight or less, 100 parts by weight or less, 50 parts by weight or less, 20 parts by weight or less, 10 parts by weight or less, 5 parts by weight or less, or 2 parts by weight or less, or may be any non-contradictory combination thereof. The amount of the spice comprised in the composition of the present invention, specifically, based on 1 part by weight of the active ingredient comprised in the composition of the present invention (converted into the dry mass of the cell of the gram-positive bacterium), may be, for example, 0.2 to 500 parts by weight, 1 to 500 parts by weight, 5 to 500 parts by weight, 50 to 500 parts by weight, 0.2 to 100 parts by weight, 1 to 100 parts by weight, 5 to 100 parts by weight, 50 to 100 parts by weight, 0.2 to 100 parts by weight, 0.5 to 50 parts by weight, or 1 to 20 parts by weight. The amount of the spice comprised in the composition of the present invention, particularly, based on 1 part by weight of the active ingredient comprised in the composition of the present invention (converted into the dry mass of the cell of the gram-positive bacterium), may be 0.2 to 500 parts by weight, or may be 50 to 500 parts by weight.

When the composition of the present invention contains an objective substance (for example, L-glutamic acid), the amount of the objective substance (for example, L-glutamic acid) comprised in the composition of the present invention may be, for example, 0.1% (w/w) or more, 0.2% (w/w) or more, 0.5% (w/w) or more, 1% (w/w) or more, 2% (w/w) or more, 5% (w/w) or more, 10% (w/w) or more, or 20% (w/w) or more, or may be 90% (w/w) or less, 50% (w/w) or less, 20% (w/w) or less, 10% (w/w) or less, 5% (w/w) or less, 2% (w/w) or less, or 1% (w/w) or less, or may be any non-contradictory combination thereof. The amount of the objective substance (for example, L-glutamic acid) comprised in the composition of the present invention may be, specifically, for example, 0.2 to 90% (w/w), 0.5 to 50% (w/w), 1 to 50% (w/w), 1 to 20% (w/w), 5 to 50% (w/w), 10 to 50% (w/w), or 20 to 50% (w/w).

When the composition of the present invention contains an objective substance (for example, L-glutamic acid), the amount of the objective substance (for example, L-glutamic acid) comprised in the composition of the present invention, for example, based on 1 part by weight of the active ingredient comprised in the composition of the present invention (converted into the dry mass of the cell of the gram-positive bacterium), may be 0.1 parts by weight or more, 0.2 parts by weight or more, 0.5 parts by weight or more, 1 part by weight or more, 2 parts by weight or more, or 5 parts by weight or more, or may be 50 parts by weight or less, 20 parts by weight or less, 10 parts by weight or less, 5 parts by weight or less, 2 parts by weight or less, or 1 part by weight or less, or may be any non-contradictory combination thereof. The amount of the objective substance (for example, L-glutamic acid) comprised in the composition of the present invention, for example, based on 1 part by weight of the active ingredient comprised in the composition of the present invention (converted into the dry mass of the cell of the gram-positive bacterium), may be, specifically, for example, 0.1 to 20 parts by weight.

When the composition of the present invention contains a culture product of the gram-positive bacterium, as any other ingredient than the active ingredient, the amount of the culture product of the gram-positive bacterium, comprised in the composition of the present invention, for example, converted into the amount of the original culture product, may be 0.01% (w/w) or more, 0.1% (w/w) or more, 1% (w/w) or more, 5% (w/w) or more, or 10% (w/w) or more, or may be 10000% (w/w) or less, 5000% (w/w) or less, 1000% (w/w) or less, 500% (w/w) or less, 300% (w/w) or less, 200% (w/w) or less, 150% (w/w) or less, 100% (w/w) or less, 70% (w/w) or less, 50% (w/w) or less, 30% (w/w) or less, 10% (w/w) or less, 5% (w/w) or less, or 1% (w/w) or less, or may be any non-contradictory combination thereof. The term "original culture product" refers to a culture product not changed in concentration due to concentrating, dilution, or the like, after culture, and may specifically refer to a culture product immediately after culture. Herein, an amount contained of more than 100% (w/w) means that the culture product is concentrated and then contained in a subject (here, the composition of the present invention). That is, for example, an amount contained of 200% (w/w) means that the culture product is concentrated two-fold and then contained in a subject.

When the composition of the present invention contains a culture product of the gram-positive bacterium, as any other ingredient than the active ingredient, and the culture product of the gram-positive bacterium contains the cell of the gram-positive bacterium, the amount of the culture product of the gram-positive bacterium, comprised in the composition of the present invention, may be set, for example, such that the amount of the active ingredient comprised in the composition of the present invention is within the above-illustrated range.

When the composition of the present invention contains a culture product of the gram-positive bacterium, as any other ingredient than the active ingredient, and the culture product of the gram-positive bacterium contains an objective substance (for example, L-glutamic acid), the amount of the culture product of the gram-positive bacterium, comprised in the composition of the present invention, may be set, for example, such that the amount of the objective substance (for example, L-glutamic acid) comprised in the composition of the present invention is within the above-illustrated range.

The amounts of the ingredients comprised in the composition of the present invention (i.e., the active ingredient and any other ingredient) can be set, for example, to achieve the amounts of the ingredients added in the method of the present invention as described later.

The ingredients comprised in the composition of the present invention (i.e., active ingredient and any other ingredient) may be mixed each other and comprised in the composition of the present invention, or may be comprised each separately or separately in any combination in the composition of the present invention. For example, the composition of the present invention may be provided as a set of such ingredients that are separately packaged. In such a case, such ingredients comprised in the set can be used in combination as appropriate when used.

### <3> Method of Present Invention

The method of the present invention is a method comprising a step in which an active ingredient is used.

That is, the method of the present invention is a method comprising a step in which the following ingredient (A) is used:
(A) a cell wall-containing fraction of a gram-positive bacterium.

The method of the present invention, specifically, use of the active ingredient enables improvement of the flavor of food, i.e., provides a flavor improvement effect. Thus, the method of the present invention may be performed for improvement of the flavor of food. That is, the method of the present invention may be, for example, a method of improving the flavor of food. The method is also referred to as "flavor improvement method of the present invention". Such improvement of the flavor may be, for example, enhancement of spiciness and/or impartment of a rich taste. Such improvement of the flavor may be particularly enhancement of pungency of a spice.

The method of the present invention, specifically, use of the active ingredient also enables production of food with improved flavor. Thus, the method of the present invention may be performed for production of food (specifically, producing food with improved flavor). That is, the method of the present invention may be, for example, a method of producing food (specifically, food with improved flavor). The method is also referred to as "method of producing food of the present invention".

The active ingredient can be used for improvement of the flavor or production of food by adding the active ingredient to a raw material of food in production of food. That is, examples of the use of the active ingredient include adding the active ingredient to a raw material of food. That is, the method of the present invention may be, specifically, for example, a method of improving the flavor of food, comprising adding the active ingredient to a raw material of food. The method of the present invention may also be, specifically, for example, a method of producing food (specifically, producing food with improved flavor), comprising adding the active ingredient to a raw material of food. The term "adding" is also referred to as "mixing".

The active ingredient may be used in the method of the present invention, for example, in a form of the composition of the present invention. That is, "use of the active ingredient" also includes use of the composition of the present invention. For example, "addition of the active ingredient" also includes addition of the composition of the present invention.

Food obtained by the method of the present invention is also referred to as "food of the present invention". The food of the present invention is, specifically, food with improved flavor. In other words, the food of the present invention is food to which the active ingredient is added food.

The improvement of the flavor or production of food may be performed, for example, in the same manner as the normal production of food except that the active ingredient is used. That is, the improvement of the flavor or production of food may be performed, for example, under the same production conditions using the same raw material as normal food except that the active ingredient is used. Both the raw material of food and production conditions may also be used for the improvement of the flavor or production of food, after appropriate modification.

The type of the food is not particularly limited as long as improvement of the flavor is desired. The food also includes any drink. The food also includes seasonings. The food may be, for example, a liquid or a solid. Specific examples of the food include drinks such as milk, soft drinks, alcohol drinks, and soups; processed meat foods such as ham, sausages, Chinese-style dumplings, shaomai, hamburgers, fried chicken, and pork cutlet; processed marine products, such as salmon flakes, spicy cod roe, salted cod roe, grilled fish, dried fish, salted squid, fish sausage, fish cakes, boiled fish, tsukudani, and canned fish; snack foods, such as potato chips, potato snacks, corn snacks, wheat snacks, cinnamon cookies, rice crackers, and cubic rice crackers; noodle soups, such as udon soup, soba soup, somen soup, ramen soup, chanpon soup, and pasta sauce; rice cooked products, such as rice balls, pilaf, fried rice, mixed rice, rice porridge, and chazuke; boiled cooking such as curry, stew, chili con carne, feijoada, and Mapo Tofu; rouxes such as stew roux and curry roux; processed vegetable products, such as kimchi and pickles; other processed foods such as breads, noodles, gratin, croquettes, and mashed potato; sauces such as Chinese sauce, oyster sauce, cheese sauce, tomato sauce, white sauce, demiglace sauce, curry sauce, Genovese sauce, chilli sauce, and Tabasco sauce; seasoning oil such as hot sesame oil; basis seasonings such as soy sauce and miso; flavor seasonings such as bonito flavor, chicken flavor, pork flavor, and beef flavor; pungent seasonings such as seven-flavored spice, doubanjiang, and gochujang; seasonings for menu (dedicated seasonings tailored for cooking menu); and other seasonings such as dressings, miso, mayonnaise, tomato ketchup, and consomme. The term "soft drink" may mean any non-alcoholic drink (drink having an alcohol concentration of less than 1%) except for milk and dairy products. Specific examples of soft drinks include water, fruit juices, vegetable juices, teas (chai, cinnamon tea, and the like), coffee drinks (coffee, milk drinks with coffee, and the like), carbonated beverages (ginger ale, lemon carbonated beverage, and the like), and isotonic drinks. Specific examples of soups include dal soup, Tom yum goong, egg drop soup, wakame seaweed soup, shark fin soup, Chinese soup, consomme soup, curry flavored soup, suimono, miso soup, and potage soup. Examples of the food particularly include spice-containing food (for example, food illustrated above, containing a spice). Examples of the food also include spices by themselves. The food may be provided in a ready-to-eat form, or in a form that requires preparation before or during eating, such as concentrated or dried products. The food is not limited to any common food, and also includes any so-called health food or medical food, such as nutritional supplementary food (supplement), nutrient functional food, or specified health food. That is, for example, each food illustrated above may be provided as common food, or provided as health food or medical food.

The term "raw material of food" means a food material for production of food. The raw material of food is not particularly limited as long as food can be produced. The raw material of food can be selected as appropriate, for example, depending on various conditions such as the type of the food. Examples of the raw material of food include ingredients as illustrated above that can be usually used for production of food. Specific examples of the raw material of food include ingredients such as grains, vegetables, meats, fish, and egg; seasoning ingredients such as sugars, inorganic salts, organic acids, nucleic acids, amino acids, and protein hydrolysates; dairy products such as milk and cheeses; spices; flavors; fats and oils; and alcohols. Examples of organic acids, nucleic acids, and amino acids include those illustrated with respect to the objective substance.

The active ingredient may be added to the raw material of food during any step in the food production process provided that the flavor improvement effect is obtained. That is, "raw material of food" to which the active ingredient is to be added may be that in any step in the food production process. For example, "raw material of food" to which the active ingredient is to be added may include complete food before addition of the active ingredient. The active ingredient can be added to the raw material of food directly or after being appropriately prepared into a desired form such as a solution. Operations to allow the active ingredient to coexist with the raw material of food may be collectively referred to as "addition of the active ingredient". Any other ingredient than the active ingredient may also be added as appropriate to the raw material of food. That is, the method of the present invention may further comprise adding any other ingredient than the active ingredient to the raw material of food. Such any other ingredient than the active ingredient is not particularly limited as long as the flavor improvement effect is not impaired (i.e., the flavor improvement effect by the active ingredient is obtained). Such any other ingredient than the active ingredient can be selected as appropriate, for example, depending on various conditions such as the type of food. Such any other ingredient than the active ingredient includes a culture product of a gram-positive bacterium, an objective substance, and a spice. The description on the active ingredient can also be applied correspondingly to addition of any other ingredient than the active ingredient. All the ingredients (i.e., active ingredient and any other ingredient) may be added to the raw material of food at the same time, or may be added to the raw material of food each separately or separately in any combination. The order in which the ingredients are added to the raw material of food is not particularly limited.

The amounts of the ingredients (i.e., active ingredient and any other ingredient) and the amount ratio therebetween added in the method of the present invention are not particularly limited as long as the flavor improvement effect is obtained. The amounts of the ingredients and the amount ratio therebetween added in the method of the present invention can be set as appropriate depending on various conditions such as the type of the raw material of food and the type of the food.

The active ingredient may be added to the raw material of food, for example, such that the concentration of the active ingredient at the time of eating is within a desired range (for example, the range of the concentration of the active ingredient at the time of eating, described later).

The concentration of the active ingredient at the time of eating, for example, converted into the dry mass of the cell of the gram-positive bacterium, may be 0.005% (w/w) or more, 0.007% (w/w) or more, 0.01% (w/w) or more, 0.02% (w/w) or more, 0.03% (w/w) or more, 0.04% (w/w) or more, 0.05% (w/w) or more, 0.07% (w/w) or more, 0.1% (w/w) or more, 0.2% (w/w) or more, 0.3% (w/w) or more, 0.4% (w/w) or more, or 0.5% (w/w) or more, or may be 5% (w/w) or less, 2% (w/w) or less, 1% (w/w) or less, 0.7% (w/w) or less, 0.5% (w/w) or less, 0.4% (w/w) or less, 0.3% (w/w) or less, 0.2% (w/w) or less, or 0.1% (w/w) or less, or may be any non-contradictory combination thereof. The concentration of the active ingredient at the time of eating, specifically, for example, converted into the dry mass of a cell of the gram-positive bacterium, may be 0.005 to 2% (w/w), 0.005 to 1% (w/w), 0.005 to 0.5% (w/w), 0.01 to 2% (w/w), 0.02 to 1% (w/w), or 0.03 to 0.5% (w/w).

The description of addition of the active ingredient can also be applied correspondingly to a case where the composition of the present invention is added. For example, the composition of the present invention can be added to achieve the above-illustrated amount of the active ingredient added.

The food of the present invention may contain, in addition to the active ingredient, a spice. That is, the food of the present invention may be produced to contain a spice. That is, the method of the present invention may further comprise adding a spice to the raw material of food. The food containing a spice may be produced, for example, by adding a spice itself, or by adding a material containing a spice, such as a seasoning containing a spice. When the composition of the present invention contains a spice, such a spice may be added by adding the composition of the present invention. Addition of a spice can be performed similarly as addition of the active ingredient. A spice may be added to the raw material of food, for example, such that the amount of a spice contained in the food of the present invention is within a desired range (for example, a range of an amount contained, described later). The raw material of food may originally contain a spice.

When the food of the present invention contains a spice, the amount of the spice contained in the food of the present invention, as a concentration at the time of eating, may be, for example, 0.01% (w/w) or more, 0.02% (w/w) or more, 0.03% (w/w) or more, 0.04% (w/w) or more, 0.05% (w/w) or more, 0.07% (w/w) or more, 0.1% (w/w) or more, 0.2% (w/w) or more, 0.3% (w/w) or more, 0.4% (w/w) or more, or 0.5% (w/w) or more, or may be 5% (w/w) or less, 2% (w/w) or less, 1% (w/w) or less, 0.7% (w/w) or less, 0.5% (w/w) or less, 0.4% (w/w) or less, 0.3% (w/w) or less, 0.2% (w/w) or less, or 0.1% (w/w) or less, or may be any non-contradictory combination thereof. The amount of the spice contained in the food of the present invention, specifically, for example, as a concentration at the time of eating, may be 0.01 to 2% (w/w), 0.02 to 1% (w/w), or 0.03 to 0.5% (w/w).

When the food of the present invention contains a spice, the amount of the spice contained in the food of the present invention, for example, based on 1 part by weight of the active ingredient contained in the food of the present invention (conversion into the dry mass of a cell of the gram-positive bacterium), may be 0.2 parts by weight or more, 0.5 parts by weight or more, 1 part by weight or more, 2 parts by weight or more, 5 parts by weight or more, 10 parts by weight or more, 20 parts by weight or more, 50 parts by weight or more, or 100 parts by weight or more, or may be 1000 parts by weight or less, 500 parts by weight or less, 200 parts by weight or less, 100 parts by weight or less, 50 parts by weight or less, 20 parts by weight or less, 10 parts by weight or less, 5 parts by weight or less, or 2 parts by weight or less, or may be any non-contradictory combination thereof. The amount of the spice contained in the food of the present invention, specifically, for example, based on 1 part by weight of the active ingredient contained in the food of the present invention (conversion into the dry mass of a cell of the gram-positive bacterium), may be 0.2 to 500 parts by weight, 1 to 500 parts by weight, 5 to 500 parts by weight, 50 to 500 parts by weight, 0.2 to 100 parts by weight, 1 to 100 parts by weight, 5 to 100 parts by weight, 50 to 100 parts by weight, 0.2 to 100 parts by weight, 0.5 to 50 parts by weight, or 1 to 20 parts by weight. The amount of the spice contained in the food of the present invention, particularly, based on 1 part by weight of the active ingredient contained in the food of the present invention (conversion into the dry mass of a cell of the gram-positive bacterium), may be 0.2 to 500 parts by weight, or may be 50 to 500 parts by weight.

The food of the present invention may contain, in addition to the active ingredient, an objective substance. That is, the food of the present invention may be produced to contain an objective substance. That is, the method of the present invention may further comprise adding an objective substance to the raw material of food. Such an objective substance is as described above. The food containing an objective substance may be produced, for example, by adding an objective substance itself, or by adding a material containing an objective substance, such as a seasoning containing an objective substance. Here, "adding active ingredient and objective substance" is not limited to any case where the active ingredient and the objective substance individually obtained are added, and also includes a case where the active ingredient and the objective substance collectively obtained are added. Examples of the case where the active ingredient and the objective substance collectively obtained are added include a case where a culture product containing both the objective substance and a cell of the gram-positive bacterium is added directly or dried as appropriate and then added. When the composition of the present invention contains an objective substance, the objective substance may be added by adding the composition of the present invention. Addition of the objective substance can be performed similarly as addition of the active ingredient. The objective substance may be added to the raw material of food, for example, such that the amount of the objective substance contained in the food of the present invention is within a desired range (for example, a range of an amount contained, described later). The objective substance may also be originally contained in the raw material of food.

When the food of the present invention contains an objective substance (for example, L-glutamic acid), the amount of the objective substance (for example, L-glutamic acid) contained in the food of the present invention, as a concentration at the time of eating, may be, for example, 0.01% (w/w) or more, 0.02% (w/w) or more, 0.03% (w/w) or more, 0.04% (w/w) or more, 0.05% (w/w) or more, 0.07% (w/w) or more, 0.1% (w/w) or more, 0.2% (w/w) or more, 0.3% (w/w) or more, 0.4% (w/w) or more, or 0.5% (w/w) or more, or may be 5% (w/w) or less, 2% (w/w) or less, 1% (w/w) or less, 0.7% (w/w) or less, 0.5% (w/w) or less, 0.4% (w/w) or less, 0.3% (w/w) or less, 0.2% (w/w) or less, or 0.1% (w/w) or less, or may be any non-contradictory combination thereof. The amount of the objective substance (for example, L-glutamic acid) contained in the food of the present invention, specifically, for example, as a concentration at the time of eating, may be 0.01 to 2% (w/w), 0.02 to 1% (w/w), or 0.03 to 0.5% (w/w).

When the food of the present invention contains an objective substance (for example, L-glutamic acid), the amount of the objective substance (for example, L-glutamic acid) contained in the food of the present invention, for example, based on 1 part by weight of the active ingredient contained in the food of the present invention (conversion into the dry mass of a cell of the gram-positive bacterium), may be 0.1 parts by weight or more, 0.2 parts by weight or more, 0.5 parts by weight or more, 1 part by weight or more, 2 parts by weight or more, or 5 parts by weight or more, or may be 50 parts by weight or less, 20 parts by weight or less, 10 parts by weight or less, 5 parts by weight or less, 2 parts by weight or less, or 1 part by weight or less, or may be any non-contradictory combination thereof. The amount of the objective substance (for example, L-glutamic acid) contained in the food of the present invention, for example, based on 1 part by weight of the active ingredient contained in the food of the present invention (conversion into the dry mass of a cell of the gram-positive bacterium), may be, specifically, for example, 0.1 to 20 parts by weight.

The food of the present invention may contain, in addition to the active ingredient, a culture product of the gram-positive bacterium. That is, the food of the present invention may be produced to contain a culture product of the gram-positive bacterium. That is, the method of the present invention may further comprise adding a culture product of the gram-positive bacterium to the raw material of food. Such a culture product of the gram-positive bacterium, as any other ingredient than the active ingredient, is as described above. The food containing a culture product of the gram-positive bacterium may be produced, for example, by adding a culture product of the gram-positive bacterium, itself, or by adding a material containing a culture product of the gram-positive bacterium. Here, "addition active ingredient and culture product of gram-positive bacterium" is not limited to any case where the active ingredient and the culture product individually obtained are added, and also includes a case where the active ingredient and the culture product collectively obtained are added. Examples of the case where the active ingredient and the culture product collectively obtained are added include a case where a culture product containing a cell of the gram-positive bacterium is added directly or dried as appropriate and then added. When the composition of the present invention contains a culture product of the gram-positive bacterium, the culture product of the gram-positive bacterium may be added by adding the composition of the present invention. Addition of the culture product of the gram-positive bacterium can be performed similarly as addition of the active ingredient. The culture product of the gram-positive bacterium may be added to the raw material of food, for example, such that the amount of the culture product of the gram-positive bacterium, contained in the food of the present invention, is within a desired range (for example, a range of an amount contained, described later). The culture product of the gram-positive bacterium may also be originally contained in the raw material of food.

When the food of the present invention contains a culture product of the gram-positive bacterium, as any other ingredient than the active ingredient, and the culture product of the gram-positive bacterium contains a cell of the gram-positive bacterium, the amount of the culture product of the gram-positive bacterium, contained in the food of the present invention, may be set, for example, such that the amount of the active ingredient contained in the food of the present invention is within the above-illustrated range.

When the food of the present invention contains a culture product of the gram-positive bacterium, as any other ingredient than the active ingredient, and the culture product of the gram-positive bacterium contains an objective substance (for example, L-glutamic acid), the amount of the culture product of the gram-positive bacterium, contained in the food of the present invention, may be set, for example, such that the amount of the objective substance (for example, L-glutamic acid) contained in the food of the present invention is within the above-illustrated range.

When the food of the present invention contains a culture product of the gram-positive bacterium, as any other ingredient than the active ingredient, the amount of the culture product of the gram-positive bacterium, contained in the food of the present invention, for example, converted into the original amount of the culture product, may be 0.01% (w/w) or more, 0.1% (w/w) or more, 1% (w/w) or more, 5% (w/w) or more, or 10% (w/w) or more, or may be 10000% (w/w) or less, 5000% (w/w) or less, 1000% (w/w) or less, 500% (w/w) or less, 300% (w/w) or less, 200% (w/w) or less, 150% (w/w) or less, 100% (w/w) or less, 70% (w/w) or less, 50% (w/w) or less, 30% (w/w) or less, 10% (w/w) or less, 5% (w/w) or less, or 1% (w/w) or less, or may be any non-contradictory combination thereof.

### <4> Use of Active Ingredient

The present invention also discloses use of an active ingredient in the application illustrated above. That is, the present invention discloses, for example, use of an active ingredient for improvement of the flavor or production of food, and use of an active ingredient for production of a composition for improvement of the flavor or production of food.

The present invention also discloses an active ingredient for use in the application illustrated above. That is, the present invention discloses, for example, an active ingredient for use in the improvement of the flavor or production of food, and an active ingredient for use in the production of a composition for improvement of the flavor or production of food.

The present invention also discloses use of an active ingredient for combination use with any other ingredient (for example, spice). An active ingredient may be used in combination with any other ingredient (for example, spice), for the application illustrated above.

### <5> Another Aspect of Present Invention

Another aspect of the present invention relates to use of an L-glutamic acid-producing bacterium having "specified mutation". The L-glutamic acid-producing bacterium having "specified mutation" is as mentioned above. In another aspect of the present invention, the L-glutamic acid-producing bacterium having "specified mutation" is also simply referred to as "L-glutamic acid-producing bacterium".

The L-glutamic acid-producing bacterium can be used for, for example, production of L-glutamic acid. L-glutamic acid is as mentioned above. In another aspect of the present invention, L-glutamic acid is also referred to as "active ingredient".

L-glutamic acid may be produced and/or used as a free form, or as a salt, or as a combination thereof. That is, unless otherwise noted, the term "L-glutamic acid" may mean L-glutamic acid in a free form, or a salt thereof, or a combination thereof. L-glutamic acid may be produced and/or used as a non-hydrate, or as a hydrate, or as a combination thereof. That is, unless otherwise noted, the term "L-glutamic acid" (for example, "L-glutamic acid in a free form" or "salt of L-glutamic acid") may include a non-hydrate and a hydrate. L-glutamic acid may be in any form, such as an ionic form, when being used. The amount of L-glutamic acid (for example, the amount contained (concentration) or the amount used) in the case of L-glutamic acid forming a salt or hydrate will be calculated based on the value obtained by converting the mass of the salt or hydrate into the mass of an equimolar of a free form. The description on the salt of the objective substance can also be applied correspondingly to the salt of L-glutamic acid. Examples of the salt of L-glutamic acid particularly include sodium L-glutamate (for example, monosodium L-glutamate; MSG) and ammonium L-glutamate (for example, monoammonium L-glutamate).

L-glutamic acid can be used to enhance the umami of food, i.e., obtain an effect of enhancing the umami of food. The effect is also referred to as "umami enhancement effect". Enhancement of the umami of food is also simply referred to as "enhancement of the umami". Specifically, L-glutamic acid can be used to enhance the umami of food as compared with a case where L-glutamic acid is not used. Thus, L-glutamic acid may be used for enhancement of the umami of food.

Measurement and comparison of the umami can be performed, for example, by sensory evaluation with an expert panel.

L-glutamic acid can be produced by, for example, culturing an L-glutamic acid-producing bacterium, in a medium. That is, the method of producing L-glutamic acid may include, for example, a step of culturing an L-glutamic acid-producing bacterium, in a medium. The step is also referred to as "culture step". The culture step may also be, specifically, a step of culturing an L-glutamic acid-producing bacterium, in a medium, to provide a culture product. The culture step may be more specifically a step of culturing an L-glutamic acid-producing bacterium, in a medium, to provide a culture product containing L-glutamic acid.

The medium and culture conditions in culture of an L-glutamic acid-producing bacterium are not particularly limited as long as an L-glutamic acid-producing bacterium can be cultivated and L-glutamic acid is produced. For example, the descriptions on the medium and culture conditions of the gram-positive bacterium can also be applied correspondingly to the medium and culture conditions of an L-glutamic acid-producing bacterium. For example, the medium may contain a raw material of food (for example, *Solanaceae* family plant such as a tomato).

An L-glutamic acid-producing bacterium is cultured to provide a culture product (specifically, culture product containing a cell of the L-glutamic acid-producing bacterium, and L-glutamic acid) of the L-glutamic acid-producing bacterium.

L-glutamic acid may be, for example, used as the active ingredient as it is contained in the culture product (specifically medium), or may be recovered from the culture product (specifically medium) and then used as the active ingredient. That is, the method of producing L-glutamic acid may include, for example, a step of recovering L-glutamic acid from the culture product (specifically medium). L-glutamic acid may be recovered as an appropriate L-glutamic acid-containing fraction. Examples of such a fraction include a culture supernatant. L-glutamic acid may also be further separated and purified from the above fraction. For example, the cell of the L-glutamic acid-producing bacterium may be separated from the culture product to provide a culture supernatant and L-glutamic acid may be recovered from the culture supernatant. Herein, "recovery of L-glutamic acid" may include removal of impurities from the L-glutamic acid-containing fraction. Recovery of L-glutamic acid can be performed by, for example, a known procedure. Examples of such a include ion-exchange resin methods (Nagai, H. et al., Separation Science and Technology, 39(16), 3691-3710), precipitation methods, membrane separation methods (JP H9-164323 A, JP H9-173792 A), and crystallization methods (WO2008/078448, WO2008/078646). The L-glutamic acid-containing fraction (for example, culture product and/or culture supernatant) may be subjected as appropriate to treatment and then used as the active ingredient. Examples of the treatment include concentration, drying, and heating. All of concentration, drying, and heating can be performed by, for example, known procedures. That is, examples of the active ingredient include a culture product of the L-glutamic acid-producing bacterium, a culture supernatant recovered from the culture product, any treated product thereof, and L-glutamic acid recovered therefrom. That is, L-glutamic acid may be used as the active ingredient, for example, in the form of a culture product of the L-glutamic acid-producing bacterium, a culture supernatant recovered from the culture product, any treated product thereof, L-glutamic acid recovered therefrom, or any combination thereof. In other words, examples of the active ingredient include L-glutamic acid contained in a culture product of the L-glutamic acid-producing bacterium, L-glutamic acid contained in a culture supernatant recovered from the culture product, L-glutamic acid contained in any treated product thereof, and L-glutamic acid recovered therefrom. That is, L-glutamic acid may be used as the active ingredient, for example, in the form of L-glutamic acid contained in a culture product of the L-glutamic acid-producing bacterium, L-glutamic acid contained in a culture supernatant recovered from the culture product, L-glutamic acid contained in any treated product thereof, L-glutamic acid recovered therefrom, or any combination thereof.

L-glutamic acid may be used in combination with, for example, any other ingredient (i.e., any other ingredient than L-glutamic acid). Examples of such any other ingredient include a cell wall-containing fraction of a gram-positive bacterium. The cell wall-containing fraction of a gram-positive bacterium is as described above. The gram-positive bacterium may or may not be the same as the L-glutamic acid-producing bacterium. The cell wall-containing fraction of the gram-positive bacterium may be, for example, produced along with production of L-glutamic acid, or separately produced from L-glutamic acid. Herein, " L-glutamic acid and a cell wall-containing fraction of gram-positive bacterium being used in combination" is not limited to any case where L-glutamic acid and the cell wall-containing fraction of the gram-positive bacterium, individually obtained, are used in combination, and also includes a case where L-glutamic acid and the cell wall-containing fraction of the gram-positive bacterium, collectively obtained, are used. Examples of the case where L-glutamic acid and the cell wall-containing fraction of the gram-positive bacterium, collectively obtained, are used include a case where a culture product of the L-glutamic acid-producing bacterium is used directly, or dried as appropriate and then used as the active ingredient. L-glutamic acid and the cell wall-containing fraction of the gram-positive bacterium may be used in combination, thereby imparting a flavor improvement effect. The flavor improvement effect is as described above.

A composition of another aspect of the present invention is a composition comprising an active ingredient (herein, L-glutamic acid produced by culturing the L-glutamic acid-producing bacterium in a medium).

A composition of another aspect of the present invention can be used to enhance the umami of food, i.e., obtain an umami enhancement effect. Thus, a composition of another aspect of the present invention may be used for enhancement of the umami of food. That is, a composition of another aspect of the present invention may be, for example, a composition for enhancement of the umami of food.

A composition of another aspect of the present invention can be used to produce food with enhanced umami. Thus, a composition of another aspect of the present invention may be used for production of food (specifically, production of food with enhanced umami). That is, a composition of another aspect of the present invention may be, for example, a composition for production of food (specifically, production of food with enhanced umami).

A composition of another aspect of the present invention may be, for example, a seasoning. A composition of another aspect of the present invention may be, specifically, for example, a seasoning for enhancement of the umami of food, or a seasoning for production of food (specifically, production of food with enhanced umami).

A composition of another aspect of the present invention may be used for enhancement of the umami or production of food in an aspect described with respect to a method of another aspect of the present invention described later.

A composition of another aspect of the present invention may be composed of an active ingredient, or may contain any other ingredient than the active ingredient. In one aspect, a composition composed of the active ingredient may be excluded from a composition of another aspect of the present invention. As any other ingredient than the active ingredient, one ingredient may be used, or two or more ingredients may be used in combination.

The active ingredient comprised in a composition of another aspect of the present invention may be produced by the above method of producing the active ingredient. Thus, the method of producing a composition of another aspect of the present invention may include a step of producing the active ingredient by the above method of producing the active ingredient. The step of producing the active ingredient in the method of producing a composition of another aspect of the present invention may be specifically a step of culturing an L-glutamic acid-producing bacterium, in a medium, to provide a culture product. The step of producing the active ingredient in the method of producing a composition of another aspect of the present invention may be more specifically a step of culturing an L-glutamic acid-producing bacterium, in a medium, to provide a culture product containing L-glutamic acid.

Such any other ingredient than the active ingredient is not particularly limited as long as the umami enhancement effect is not impaired (i.e., the umami enhancement effect by the active ingredient is obtained). Such any ingredient than the active ingredient can be selected as appropriate, for example, depending on various conditions such as the type of food. Examples of such any other ingredient than the active ingredient include ingredients mixed to foods or pharmaceuticals.

Specific examples of such any other ingredient than the active ingredient include ingredients that are effective for production of food. Examples of ingredients that are effective for production of food include the above-mentioned raw materials of food. Such any other ingredient than the active ingredient particularly includes a cell wall-containing fraction of a gram-positive bacterium, and a spice. That is, a composition of another aspect of the present invention may be, for example, a composition (for example, seasoning) comprising the active ingredient and a cell wall-containing fraction of a gram-positive bacterium, a composition (for example, seasoning) comprising the active ingredient and a spice, or a composition (for example, seasoning) comprising the active ingredient, a cell wall-containing fraction of a gram-positive bacterium, and a spice. The cell wall-containing fraction of a gram-positive bacterium is as described above. Herein, "composition comprising active ingredient and a cell wall-containing fraction of gram-positive bacterium" is not limited to any case where the active ingredient and the cell wall-containing fraction of a gram-positive bacterium, individually obtained, are comprised in the composition, and also includes a case where the active ingredient and the cell wall-containing fraction of a gram-positive bacterium, collectively obtained, are comprised in the composition. Examples of the case where the active ingredient and the cell wall-containing fraction of a gram-positive bacterium, collectively obtained, are comprised in the composition include a case where a culture product of the L-glutamic acid-producing bacterium is contained directly, or dried as appropriate and then comprised in the composition. The spice is as described above. In one aspect, a composition of another aspect of the present invention may contain no L-glutamic acid produced by other method than the above method of producing the active ingredient.

A composition of another aspect of the present invention can be produced by, for example, mixing the active ingredient and any other ingredient as appropriate.

A composition of another aspect of the present invention may be, for example, formulated as appropriate. In formulation, additives may be used as appropriate. Examples of the additives include excipients, binders, disintegrants, lubricants, stabilizers, correctives, diluents, surfactants, and solvents. The additives can be selected as appropriate, for example, depending on various conditions such as the shape of a composition of another aspect of the present invention.

The form of a composition of another aspect of the present invention is not particularly limited. A composition of another aspect of the present invention may be in any form, such as powder, flake, tablet, paste, or liquid.

The amounts of the ingredients (i.e., active ingredient and any other ingredient) and the amount ratio therebetween comprised in a composition of another aspect of the present invention are not particularly limited as long as the umami enhancement effect is obtained. The amounts of the ingredients and the amount ratio therebetween comprised in a composition of another aspect of the present invention can be set as appropriate depending on various conditions such as the mode of use of a composition of another aspect of the present invention. For example, the descriptions on the amounts of the ingredients and the amount ratio therebetween comprised in the composition of the present invention can also be applied correspondingly to the amounts of the ingredients and the amount ratio therebetween comprised in a composition of another aspect of the present invention. That is, the amount of L-glutamic acid comprised in a composition of another aspect of the present invention may be, for example, within the range of the amount of L-glutamic acid comprised in the composition of the present invention. When a composition of another aspect of the present invention contains a cell wall-containing fraction of a gram-positive bacterium, the amount of the cell wall-containing fraction of a gram-positive bacterium, comprised in a composition of another aspect of the present invention, may be, for example, the range of the amount of the cell wall-containing fraction of a gram-positive bacterium comprised in the above composition of the present invention. When a composition of another aspect of the present invention contains a spice, the amount of the spice comprised in a composition of another aspect of the present invention may be, for example, within the range of the amount of the spice comprised in the above composition of the present invention. When a composition of another aspect of the present invention contains a cell wall-containing fraction of a gram-positive bacterium, the ratio between the amount of the L-glutamic acid contained and the amount of the cell wall-containing fraction of a gram-positive bacterium comprised in a composition of another aspect of the present invention may be within, for example, the range of the ratio between the amount of the L-glutamic acid contained and the amount of the cell wall-containing fraction of a gram-positive bacterium comprised in the above composition of the present invention. When a composition of another aspect of the present invention contains a cell wall-containing fraction of a gram-positive bacterium, and a spice, the ratio between the amount of the cell wall-containing fraction of a gram-positive bacterium comprised and the amount of the spice comprised in a composition of another aspect of the present invention may be, for example, the ratio between the amount of the cell wall-containing fraction of a gram-positive bacterium comprised and the amount of the spice comprised in the composition of the present invention.

The amount of L-glutamic acid comprised in a composition of another aspect of the present invention is more than 0% (w/w) and 100% (w/w) or less. The amount of L-glutamic acid comprised in a composition of another aspect of the present invention may be, for example, 0.1% (w/w) or more, 0.2% (w/w) or more, 0.5% (w/w) or more, 1% (w/w) or more, 2% (w/w) or more, 5% (w/w) or more, 10% (w/w) or more, or 20% (w/w) or more, or may be 100% (w/w) or less, less than 100% (w/w), 99.9% (w/w) or less, 90% (w/w) or less, 50% (w/w) or less, 40% (w/w) or less, 20% (w/w) or less, 10% (w/w) or less, 5% (w/w) or less, 2% (w/w) or less, or 1% (w/w) or less, or may be any non-contradictory combination thereof. The amount of L-glutamic acid comprised in a composition of another aspect of the present invention may be, specifically, for example, 0.2 to 90% (w/w), 0.5 to 50% (w/w), 1 to 50% (w/w), 1 to 20% (w/w), 5 to 50% (w/w), 10 to 50% (w/w), 20 to 50% (w/w), 1 to 40% (w/w), 5 to 40% (w/w), 10 to 40% (w/w), or 20 to 40% (w/w).

When a composition of another aspect of the present invention contains a cell wall-containing fraction of a gram-positive bacterium, the amount of the cell wall-containing fraction of a gram-positive bacterium comprised in a composition of another aspect of the present invention, for example, converted into the dry mass of a cell of the gram-positive bacterium, and may be 0.1% (w/w) or more, 0.2% (w/w) or more, 0.5% (w/w) or more, 1% (w/w) or more, 2% (w/w) or more, 5% (w/w) or more, 10% (w/w) or more, or 20% (w/w) or mor, or may be 90% (w/w) or less, 50% (w/w) or less, 20% (w/w) or less, 10% (w/w) or less, 5% (w/w) or less, 2% (w/w) or less, or 1% (w/w) or less, or may be any non-contradictory combination thereof. The amount of the cell wall-containing fraction of a gram-positive bacterium comprised in a composition of another aspect of the present invention, specifically, for example, converted into the dry mass of a cell of the gram-positive bacterium, may be 0.2 to 99.9% (w/w), 0.2 to 90% (w/w), 0.5 to 50% (w/w), or 1 to 20% (w/w).

The ingredients comprised in a composition of another aspect of the present invention (i.e., active ingredient and any other ingredient) may be mixed each other and comprised in such a composition of another aspect of the present invention, or may be comprised each separately or separately in any combination in such a composition of another aspect of the present invention. For example, such a composition of another aspect of the present invention may be provided as a set of such ingredients that are separately packaged. In such a case, such ingredients comprised in the set can be used in combination as appropriate when used.

A method of another aspect of the present invention is a method comprising a step in which an active ingredient (here corresponding to L-glutamic acid produced by culturing an L-glutamic acid-producing bacterium, in a medium) is used.

A method of another aspect of the present invention, specifically, use of the active ingredient enables enhancement of the umami of food, i.e., provides an umami enhancement effect. Thus, a method of another aspect of the present invention may be performed for enhancement of the umami of food. That is, a method of another aspect of the present invention may be, for example, a method of enhancing the umami of food. The method is also referred to as "umami enhancement method of another aspect of the present invention".

A method of another aspect of the present invention, specifically, use of the active ingredient also enables production of food with enhanced umami. Thus, a method of another aspect of the present invention may be performed for production of food (specifically, production of food with enhanced umami). That is, a method of another aspect of the present invention may be, for example, a method of producing food (specifically, producing food with enhanced umami). The method is also referred to as "method of producing food of another aspect of the present invention".

The active ingredient can be used for enhancement of the umami or production of food by adding the active ingredient to a raw material of food in production of food. That is, examples of the use of the active ingredient include adding the active ingredient to a raw material of food. That is, a method of another aspect of the present invention may be, specifically, for example, a method of enhancing the umami of food, comprising adding the active ingredient to a raw material of food. A method of another aspect of the present invention may also be, specifically, for example, a method of producing food (specifically, producing food with enhanced umami), comprising adding the active ingredient to a raw material of food. The term "adding" is also referred to as "mixing".

The active ingredient may be used in a method of another aspect of the present invention, for example, in a form of a composition of another aspect of the present invention. That is, "use of active ingredient" also includes use of a composition of another aspect of the present invention. For example, "addition of active ingredient" also includes addition of a composition of another aspect of the present invention.

Food obtained by a method of another aspect of the present invention is also referred to as "food of another aspect of the present invention". Food of another aspect of the present invention is, specifically, food with enhanced umami. In other words, food of another aspect of the present invention is food to which the active ingredient is added.

The enhancement of the umami or production of food may be performed, for example, in the same manner as the normal production of food except that the active ingredient is used. That is, the enhancement of the umami or production of food may be performed for example, under the same production conditions using the same raw material as normal food except that the active ingredient is used. Both the raw material of food and production conditions may also be appropriately modified and then used for enhancement of the umami or production of food. The raw material of food and production conditions of food are as described above.

The active ingredient may be added to the raw material of food during any step in the food production process provided that the umami enhancement effect is obtained. That is, "raw material of food" to which the active ingredient is to be added may be that in any step in the food production process. For example, "raw material of food" to which the active ingredient is to be added may include complete food before addition of the active ingredient. The active ingredient can be added to the raw material of food directly or after being appropriately prepared into a desired form such as a solution. Operations to allow the active ingredient to coexist with the raw material of food may be collectively referred to as "addition of the active ingredient". Any other ingredient than the active ingredient may also be added as appropriate to the raw material of food. That is, a method of another aspect of the present invention may further comprise adding any other ingredient than the active ingredient to the raw material of food. Such any other ingredient than the active ingredient is not particularly limited as long as the umami enhancement effect is not impaired (i.e., the umami enhancement effect by the active ingredient is obtained). Such any other ingredient than the active ingredient can be selected as appropriate, for example, depending on various conditions such as the type of food. Examples of such any other ingredient than the active ingredient includes a cell wall-containing fraction of the gram-positive bacterium, and a spice. In one aspect, a method of another aspect of the present invention does not necessarily include adding L-glutamic acid produced by other method than the above method of producing the active ingredient, to the raw material of food. The description on the active ingredient can also be applied correspondingly to addition of any other ingredient than the active ingredient. All the ingredients (i.e., active ingredient and any other ingredient) may be added to the raw material of food at the same time, or may be added to the raw material of food each separately or separately in any combination. The order in which the ingredients are added to the raw material of food is not particularly limited.

The amounts of the ingredients (i.e., active ingredient and any other ingredient) and the amount ratio therebetween added in a method of another aspect of the present invention are not particularly limited as long as the umami enhancement effect is obtained. The amounts of the ingredients and the amount ratio therebetween added in a method of another aspect of the present invention can be set as appropriate depending on various conditions such as the type of the raw material of food and the type of the food. For example, the descriptions on the amounts of the ingredients and the amount ratio therebetween added in the method of the present invention can also be applied correspondingly to the amounts of the ingredients and the amount ratio therebetween added in a method of another aspect of the present invention. That is, the amount of L-glutamic acid added in a method of another aspect of the present invention may be within, for example, the range of the amount of L-glutamic acid in the above method of the present invention. When a method of another aspect of the present invention include adding a cell wall-containing fraction of a gram-positive bacterium, the amount of the cell wall-containing fraction of a gram-positive bacterium, added in a method of another aspect of the present invention, may be within, for example, the range of the amount of the cell wall-containing fraction of a gram-positive bacterium, in the above method of the present invention (for example, the amount added which allows the amount of the cell wall-containing fraction of a gram-positive bacterium, contained in the food of the present invention, to be achieved). When a method of another aspect of the present invention includes a step of adding a spice, the amount of the spice added in a method of another aspect of the present invention may be within, for example, the range of the amount of the spice added in the above method of the present invention (for example, the amount added which allows the amount of the spice contained in the above food of the present invention to be achieved). When a method of another aspect of the present invention includes adding a cell wall-containing fraction of a gram-positive bacterium, the ratio between the amount of L-glutamic acid added and the amount of the cell wall-containing fraction of a gram-positive bacterium, added, in a method of another aspect of the present invention may be within, for example, the range of the ratio between the amount of L-glutamic acid added and the amount of the cell wall-containing fraction of a gram-positive bacterium, added, in the above method of the present invention (for example, the ratio between the amounts added, which allows the amount of L-glutamic acid contained and the amount of the cell wall-containing fraction of a gram-positive bacterium, contained in the food of the present invention, to be achieved). When a method of another aspect of the present invention includes adding a cell wall-containing fraction of a gram-positive bacterium, and a spice, the ratio between the amount of the cell wall-containing fraction of a gram-positive bacterium, added, and the amount of the spice added in a method of another aspect of the present invention may be within, for example, the range of the ratio between the amount of the cell wall-containing fraction of a gram-positive bacterium, added, and the amount of the spice added in the above method of the present invention (for example, the ratio of the amounts added, which allows the amount of the cell wall-containing fraction of a gram-positive bacterium, contained, and the amount of the spice contained in the food of the present invention to be achieved).

L-glutamic acid may be added to the raw material of food, for example, such that the amount of L-glutamic acid contained in food of another aspect of the present invention is within a desired range (for example, a range of an amount contained, described later).

The amount of L-glutamic acid contained in food of another aspect of the present invention, for example, as a concentration at the time of eating, may be 0.01% (w/w) or more, 0.02% (w/w) or more, 0.03% (w/w) or more, 0.04% (w/w) or more, 0.05% (w/w) or more, 0.07% (w/w) or more, 0.1% (w/w) or more, 0.2% (w/w) or more, 0.3% (w/w) or more, 0.4% (w/w) or more, or 0.5% (w/w) or more, or may be 5% (w/w) or less, 2% (w/w) or less, 1% (w/w) or less, 0.7% (w/w) or less, 0.5% (w/w) or less, 0.4% (w/w) or less, 0.3% (w/w) or less, 0.2% (w/w) or less, or 0.1% (w/w) or less, or may be any non-contradictory combination thereof. The amount of L-glutamic acid in food of another aspect of the present invention, specifically, for example, as a concentration at the time of eating, may be 0.01 to 2% (w/w), 0.02 to 1% (w/w), or 0.03 to 0.5% (w/w).

### EXAMPLES

Hereafter, the present invention will be more specifically explained with reference to examples. However, the present invention is not limited by these examples.

### Example 1: Acquirement of Highly L-glutamic Acid-Productive Strain from Corynebacterium casei JCM 12072

### (1-1) Production of Variation Strain Library of C. casei JCM 12072 Strain

A *C. casei* JCM 12072 strain was inoculated in a Sakaguchi flask where 30 mL of a medium described in Table 2(A) was stuck, and shaken and cultured at 30°C for one day, and then a cell was recovered. The cell was suspended in a solution containing 0.1 M potassium phosphate buffer (pH 7.0), 6.0% dimethyl sulfoxide, and 0.1 mg/mL N-methyl-N-nitrosoguanidine (NTG), and left to still stand at room temperature for 50 minutes. The cell was collected and washed with 0.1 M potassium phosphate buffer (pH 7.0) three times. Culture was made at 30°C for 2 hours in a Sakaguchi flask where 30 mL of a medium for recovery culture described in Table 2(B) was stuck, and the cell was recovered. The cell was suspended in 20% glycerol, and stored at -80°C. This was adopted as a mutant strain library.

**Table 2**

| (A) | |
|---|---|
| Component | **Final Concentration** |
| Soy peptone | 40 g/L |
| Yeast extract | 8 g/L |
| MgSO₄·7H₂O | 1 g/L |
| **Autoclave sterilization at pH 7.5 (KOH) and 120°C for 20 minutes** | |

| (B) | |
|---|---|
| Component | **Final Concentration** |
| Soy peptone | 40 g/L |
| Yeast extract | 8 g/L |
| MgSO₄·7H₂O | 1 g/L |
| Na₂C₄H₄O₄·6H₂O | 2 g/L |
| **Autoclave sterilization at pH 7.0 (KOH) and 120°C for 20 minutes** | |

### (1-2) Screening of Highly L-Glutamic Acid-Productive Strain from Variation Strain Library

Next, the mutant strain library prepared was shaken and cultured at 30°C for 29 hours in a Glu-minimum medium described in Table 3 to which 100 mg/L ampicillin was added, and then seeded on an agar medium described in Table 2(A). Colonies grown were inoculated respectively to the Glu-minimum medium described in Table 3 and the agar medium described in Table 2(A), and 26 clone strains lowered in ability to assimilate L-glutamic acid (L-Glu) were selected as candidate strains.

**Table 3**

| Component | **Final Concentration** |
|---|---|
| KH₂PO₄ | 3 g/L |
| NaCl | 0.5 g/L |
| Na₂HPO₄ | 6.78 g/L |
| NH₄Cl | 1 g/L |
| **Sodium hydrogen L(+)-glutamate monohydrate** | 4.49 g/L |
| MgSO₄ | 0.24 g/L |
| CaCl₂ | 0.01 g/L |
| Biotin | 0.1 µg/L |

| | |
|---|---|
| **pH not adjusted, filter sterilization** | |

Twenty six strains as the candidate strains were shaken and cultured at 30°C for 48 hours in a 500 µL evaluation medium (Table 4) stuck in a 96 deep well plate, and the amount of L-Glu accumulated in the medium was measured with Biotec Analyzer AS210 (SAKURA SI CO., LTD.). The measurement results are shown in Figure 1. An RUN5-2-96 strain (NITE ABP-03688) having the highest L-Glu concentration among the strains evaluated was selected. The RUN5-2-96 strain has all of 135 mutations of Group A (i.e., all of mutations A-1 to A-135).

**Table 4**

| < **A zone >** | |
|---|---|
| Component | **Final Concentration** |
| Glucose | 55 g/L |
| **Autoclave sterilization at pH not adjusted and 120°C for 20 minutes** | |

| **< B zone >** | |
|---|---|
| Component | **Final Concentration** |
| (NH₄)₂SO₄ | 5 g/L |
| KH₂PO₄ | 1 g/L |
| MgSO₄·7H₂O | 0.4 g/L |
| FeSO₄·7H₂O | 0.01 g/L |
| MnSO₄·5H₂O | 0.01 g/L |
| VB1 | 0.0002 g/L |
| Biotin | 0.0006 g/L |
| Bacto Peptone | 10 g/L |
| Yeast Extract | 5 g/L |
| **Autoclave sterilization at pH 7.5 (KOH) and 120°C for 20 minutes Mixed at ratio of 20% of A zone and 80% of B zone** | |

### Example 2: Preparation of Dry Bacterial Cells of C. casei JCM 12072 Strain and RUN5-2-96 Strain

Culture of a *C*. *casei* JCM 12072 strain and an RUN5-2-96 strain was performed by jar culture with a medium shown in Table 5. A fermentation tank having a volume of 1 L was used for the jar culture. The culture temperature was regulated at constant 30°C, the culture pH was regulated by ammonia gas so as to be 6.8, and the concentration of dissolved oxygen was regulated by control with stirring so as to be 23% or more as a relative value under the assumption that the concentration of saturated dissolved oxygen was 100%. The resulting culture solution was subjected to sterilization treatment at 80°C for 20 minutes, and a medium ingredient was removed by centrifugation, thereby providing a cell pellet. Furthermore, an operation involving placing water in the same amount as that of the medium to wash the cell and removing the supernatant by centrifugation was repeated twice to perform washing of the cell. The cell pellet after washing was frozen at -80°C, and then moisture was removed by a lyophilizer, thereby acquiring each dry cell of the *C. casei* JCM 12072 strain and the RUN5-2-96 strain.

**Table 5**

| Component | **Final Concentration** |
|---|---|
| Potato peptone | 10 g/L |
| Yeast Extract | 10 g/L |
| **Glucose** | 80 g/L |
| Biotin | 3 mg/L |
| KH₂PO₄ | 1 g/L |
| **Ammonium sulfate** | 1 g/L |
| **Defoamer (O-50D)** | 0.4 mL/L |

### Example 3: Preparation of Fermented Condiment with C. casei RUN5-2-96 Strain (3-1)

L-Glu fermentation with a *C*. *casei* RUN5-2-96 strain was performed by the following procedure.

First, seed culture of an RUN5-2-96 strain was performed by j ar culture with a seed medium shown in Table 6. The culture temperature was regulated at constant 30°C, and the culture pH was regulated by ammonia gas so as to be 6.8. Subsequently, main culture of the RUN5-2-96 strain (n=2; respectively designated as J1 and J2) was performed by jar culture with a medium shown in Table 7. The culture pH was regulated with ammonia gas to 6.8, as in the seed culture. The culture temperature and the concentration of dissolved oxygen were respectively regulated to 30°C and 23% or more until OD620 reached 40. Once OD620 reached 40, the temperature was raised to 35°C, and culture was continued until glucose was consumed. Thus, an L-Glu-containing culture solution was obtained. The amount of L-Glu accumulated in the medium and the amount of glucose in the medium were measured with Biotec Analyzer AS210 (SAKURA SI CO., LTD.). The dry cell weight (Dry Cell Weight; DCW) was measured by preparing a dry cell from the culture solution by the same procedure as in Example 2 (provided that no sterilization treatment was performed). Table 8 shows the results of culture.

**Table 6**

| Component | **Final Concentration** | |
|---|---|---|
| **Glucose** | 45 | g/L |
| KH₂PO₄ | 3.52 | g/L |
| MgSO₄ · 7H₂O | 0.45 | g/L |
| FeSO₄ · 7H₂O | 10 | mg/L |
| **Bean Concentrated** | 1540 | mg/L |
| **2Na succinate** | 2 | g/L |
| L (+) **Ascorbic Acid** | 8.55 | mg/L |
| VB1.HCl | 23 | mg/L |
| VB12 | 4 | µg/L |
| Biotin | 3.2 | mg/L |
| Yeast Extract | 5 | g/L |
| **Defoamer (PP-AJ-2K)** | 0.1 | mL/L |

**Table 7**

| Component | **Final Concentration** | |
|---|---|---|
| **Glucose** | 100 | g/L |
| KH₂PO₄ | 3.46 | g/L |
| MgSO₄ · 7H₂O | 0.5 | g/L |
| FeSO₄ · 7H₂O | 10 | mg/L |
| **Bean Concentrated** | 500 | mg/L |
| **2Na succinate** | 2 | g/L |
| **L (+) Ascorbic Acid** | 8.55 | mg/L |
| VB1.HCl | 23 | mg/L |
| VB12 | 4 | µg/L |
| Biotin | 0.5 | mg/L |
| **Defoamer (PP-AJ-2K)** | 0.2 | mL/L |
| Betaine | 0.6 | g/L |

**Table 8**

| | J1 | J2 |
|---|---|---|
| L-Glu Yield (%) | 39.0 | 40.6 |
| Pdt (BO vol.) (g/L/h) | 1.40 | 1.39 |
| BO-Glu conc. (g/L) | 42.3 | 43.8 |
| Total Sugar Consumption (g/B) | 30.1 | 30.1 |
| Main CT (h) | 31.8 | 33.1 |
| DCW (g/L) | 7.7 | 7.1 |

(3-2)
The culture solution obtained in (3-1) was transferred to a 2-L Duran bottle, and sterilized by stirring for 10 minutes after the temperature was allowed to reach 70°C in a water bath. Next, the culture solution sterilized was dispensed to a spin-down tube (Himac 500PA 330437A), centrifugation was performed with a centrifuge (Hitachi CR20 GIII, PRP9-2 rotor) under conditions of 7,000 rpm (9,400 × G), 25°C, and 10 minutes, and the supernatant was recovered by decantation and electric pipetting. Pure water at half the weight of that of the culture solution was added to the remaining cell in the spin-down tube in the cell separation operation, and suspension with vortexes and then centrifugation with a centrifuge (Hitachi CR20 GIII, PRP9-2 rotor) under conditions of 7,000 rpm (9,400 × G), 25°C, and 10 minutes were performed. After centrifugation, the supernatant was recovered by decantation and electric pipetting. This operation was performed twice, thereby separating any ingredient other than the cell, as the supernatant, and providing a cell washed.

(3-3)
The supernatant recovered in (3-2) was transferred to a 2-L Duran bottle and warmed in a water bath, granular activated carbon (NORIT(R) GAC1240, Cabot Norit) in an amount equal to that of L-Glu in the liquid was added after the temperature reached 60°C, and these were stirred for 1 hour. Thereafter, activated carbon was taken by filtration with round quantitative filter paper No. 5C (Advantec Toyo Kaisha, Ltd.), a Buchner funnel, and a filtration bottle, and the filtrate was acquired. A fine powder of activated carbon, leaked to the filtrate, was removed by dispensing the filtrate to a spin-down tube (Himac 500PA 330437A), performing centrifugation with a centrifuge (Hitachi CR20 GIII, PRP9-2 rotor) under conditions of 7,000 rpm (9,400 × G), 25°C, and 10 minutes, and recovering the supernatant by decantation.

(3-4)
An aqueous 27% sodium hydroxide solution was added to the filtrate obtained in (3-3) (addition of sodium hydroxide in a molar number 1.3 times that of L-Glu in the liquid), and concentration under reduced pressure was performed with a rotary evaporator under conditions of a degree of depressurization of 90 hPa and a water bath temperature of 70°C. An operation involving addition of pure water in an amount two-thirds of the amount of the liquid before concentration, every time the amount of the liquid reached an amount about one-third, and concentration under reduced pressure was repeated twice, and then concentration under reduced pressure was performed until the amount of the liquid reached an amount about one-third. The remaining fine powder in the concentrated liquid was removed with a round quantitative filter paper No. 5C (Advantec Toyo Kaisha, Ltd.), a Buchner funnel, and a filtration bottle.

(3-5)
The cell washed of the *C. casei* RUN5-2-96 strain obtained in (3-2) was added to the concentrated liquid obtained in (3-4). The adding ratio was adapted to the separating ratio in separation of the culture solution before cell separation into a liquid subjected to bacterial eradication and the cell. Maltodextrin (CIRANDA Inc., Conventional Non-GMO Tapioca Maltodextrin DE10) was added thereto so that the solid content and the content of L-Glu in a product powder after spray drying were respectively 97% and 35%. The resultant was spray dried with a spray drier (B-290 manufactured by BUCHI Japan) under conditions of an inlet temperature of 180°C, a flow rate of 5 mL/min, a flow rate of compressed gas of 473 L/h, and an amount of hot air of 38 m³/h, thereby acquiring a fermented seasoning containing the cell of the *C. casei* RUN5-2-96 strain (hereinafter, also referred to as "C. *casei* cell-containing seasoning"). The *C. casei* cell-containing seasoning contained 7% by weight of the cell of the *C. casei* RUN5-2-96 strain.

(3-6)
Maltodextrin (CIRANDA Inc., Conventional Non-GMO Tapioca Maltodextrin DE10) was added to the concentrated liquid obtained in (3-4) so that the solid content and the content of L-Glu in a product powder after spray drying were respectively 97% and 35%. The resultant was spray dried with a spray drier (B-290 manufactured by BUCHI Japan) under conditions of an inlet temperature of 180°C, a flow rate of 5 mL/min, a flow rate of compressed gas of 473 L/h, and an amount of hot air of 38 m³/h, thereby acquiring a fermented seasoning containing no cell of the *C. casei* RUN5-2-96 strain (hereinafter, also referred to as "C. *casei* broth seasoning").

### Example 4: Effect of Addition of C. casei Bacterial Cell to Cheese Sauce

The dry cell of the *C. casei* JCM 12072 strain prepared in Example 2 was added in an amount of 0.1% by weight to a commercially available alfredo cheese sauce (BERUTOLLI alfredo cheese sauce; manufactured by Unilever Japan K.K.), thereby providing a C. *casei* cell-added alfredo cheese sauce. The C. *casei* cell-added alfredo cheese sauce was subjected to sensory evaluation according to the free word method by three expert panels with the C. *casei* cell-free commercially available alfredo cheese sauce as a control article.

It was confirmed from the results of sensory evaluation that the C. *casei* cell-added alfredo cheese sauce was enhanced in thickness and robustness as compared with the *C. casei* cell-free alfredo cheese sauce. It was indicated from this result that addition of the *C. casei* cell enhanced the thickness and robustness of cheese, and enhanced the preference of cheese-containing food. It was revealed from the foregoing that addition of the *C. casei* cell provided a flavor-improving effect, for example, impartment of a rich taste in food.

### Example 5: Effect of Addition of C. casei Bacterial Cell to Tomato Sauce

The dry cell of the *C. casei* JCM 12072 strain prepared in Example 2 was added in an amount of 0.1% by weight to a commercially available tomato sauce (manufactured by RAGU), thereby providing a *C. casei* cell-added tomato sauce. The *C. casei* cell-added tomato sauce was subjected to sensory evaluation according to the free word method by three expert panels with the *C. casei* cell-free commercially available tomato sauce as a control article.

It was confirmed from the results of sensory evaluation that the C. *casei* cell-added tomato sauce was enhanced in thickness, ripe feeling, and robustness as compared with the *C. casei* cell-free tomato sauce. It was indicated from this result that addition of the *C. casei* cell enhanced the thickness, ripe feeling, and robustness of tomatoes, and enhanced the preference of tomato-containing food. It was revealed from the foregoing that addition of the C. *casei* cell provided a flavor-improving effect, for example, impartment of a rich taste in food.

### Example 6: Effect of Addition of C. casei Bacterial Cell to Curry Sauce

A curry sauce was obtained by adding 9.4 parts by weight of boiling water to 1 part by weight of a commercially available powder curry roux (manufactured by House Foods Corp.) and stirring them. The dry cell of the C. *casei* JCM 12072 strain prepared in Example 2 was added in an amount of 0.005, 0.01, 0.025, 0.05, 0.1, 0.25, 0.5, or 1% by weight to the curry sauce, and these were stirred, thereby providing a *C. casei* cell-added curry sauce. The *C. casei* cell-added curry sauce was subjected to sensory evaluation by three expert panels with the *C. casei* cell-free curry sauce as a control article. The sensory evaluation was performed with respect to each of the thickness impartment effect and the pungency enhancement effect, according to the scoring system in the range of scores 0 to 10 (a higher score indicated a higher effect and score 1 or higher was determined to be effective). The pungency enhancement effect was scored under the assumption that the intensity of pungency of the control article was score 0 and the intensity of pungency, corresponding to 2-fold that of the control article, was score 5. The thickness enhancement effect was scored under the assumption that the intensity of thickness of the control article was score 0 and the intensity of thickness, corresponding to 2-fold that of the control article, was score 5.

Table 9 shows the results. In the Table, "Score" represents the average value of scores by three expert panels. In the Table, "Concentration (%) Added" represents the concentration of the dry cell of the *C. casei* JCM 12072 strain added. While the thickness impartment effect and the pungency enhancement effect were very weak in the case of addition of 0.005% by weight of the C. *casei* cell, the thickness impartment effect and the pungency enhancement effect were observed in the case of addition of the *C*. *casei* cell at a concentration of 0.01% or more. If the concentration of the *C*. *casei* cell added was 1% by weight, a tendency was demonstrated in which an unusual taste and a bitter taste were generated to result in the change in quality of taste. It was indicated from the foregoing results that in the case of addition of 0.01 to 0.5% by weight of the *C*. *casei* cell to the curry sauce, the effect of enhancing thickness and pungency was exerted without any large change in quality of taste. It was revealed from the foregoing that addition of the *C*. *casei* cell (for example, addition at 0.01% to 0.5%) provided flavor-improving effects, for example, impartment of a rich taste and enhancement of spiciness in food (for example, spice-containing food).

**Table 9**

| **Concentration (%) added** | **Score** | | **Comments** |
|---|---|---|---|
| | **Impartment of thickness** | **Enhancement of pungency** | |
| 0.005 | 0.6 | 0.6 | **Weak effect of addition** |
| 0.01 | 1.4 | 1.8 | **Effect gradually easily seen** |
| 0.025 | 2.6 | 3.2 | **Effect further easily seen** |
| 0.05 | 4.2 | 4.4 | **Effect further easily seen** |
| 0.1 | 5.0 | 5.0 | **Effect most easily seen** |
| 0.25 | 5.0 | 5.1 | **Little unusual taste** |
| 0.5 | 4.9 | 5.0 | **Mellower. Slight bitter taste** |
| 1 | 4.8 | 5.1 | **Bitter taste and unusual taste. Flouriness** |

### Example 7: Effect of Addition of C. casei Bacterial Cell to Black Pepper-Containing Mashed Potato

Mashed potato was obtained by adding 6 parts by weight of boiling water to 1 part by weight of commercially available dry mashed potato (manufactured by Calbee, Inc.), and stirring them. A commercially available black pepper powder (manufactured by House Gaban Corporation) was added in an amount of 0.15% by weight to the mashed potato, and these were stirred, thereby providing black pepper-containing mashed potato. The dry cell of the *C*. *casei* JCM 12072 strain prepared in Example 2 was added in an amount of 0.005, 0.01, 0.025, 0.05, 0.1, 0.25, 0.5, or 1% by weight to the black pepper-containing mashed potato, and these were stirred, thereby providing *C*. *casei* cell-added black pepper-containing mashed potato. The *C. casei* cell-added black pepper-containing mashed potato was subjected to sensory evaluation by three expert panels with the *C. casei* cell-free black pepper-containing mashed potato as a control article. The sensory evaluation was performed with respect to the pungency enhancement effect, according to the scoring system in the range of scores 0 to 10 (a higher score indicated a higher effect and score 1 or higher was determined to be effective). The pungency enhancement effect was scored under the assumption that the intensity of pungency of the control article was score 0 and the intensity of pungency of 0.3% by weight black pepper-containing mashed potato was score 5.

Table 10 shows the results. In the Table, "Score" represents the average value of scores by three expert panels. In the Table, "Concentration (%) Added" represents the concentration of the dry cell of the *C. casei* JCM 12072 strain added. While the pungency enhancement effect was very weak in the case of addition of 0.005% by weight of the *C. casei* cell, the thickness impartment effect and the pungency enhancement effect were observed in the case of addition of the *C. casei* cell at a concentration of 0.01% or more. If the concentration of the *C. casei* cell added was 1% by weight, a tendency was demonstrated in which an unusual taste and flouriness were generated to result in the change in quality of taste. It was indicated from the foregoing results that in the case of addition of 0.01 to 0.5% by weight of the *C*. *casei* cell to the black pepper-containing mashed potato, the effect of enhancing thickness and pungency was exerted without impartment of any unusual taste and the like. It was revealed from the foregoing that addition of the *C*. *casei* cell (for example, addition at 0.01% to 0.5%) provided flavor-improving effects, for example, impartment of a rich taste and enhancement of spiciness in food (for example, spice-containing food).

**Table 10**

| **Concentration (%) added** | **Score** | **Comments** |
|---|---|---|
| | **Enhancement of pungency** | |
| 0.005 | 0.6 | **Weak effect of addition** |
| 0.01 | 1.4 | **Effect gradually easily seen** |
| 0.025 | 3.2 | **Effect highly increased at 0.01 → 0.025%** |
| 0.05 | 4.2 | **Effect further increased** |
| 0.1 | 5.0 | **Effect further increased** |
| 0.25 | 6.8 | **Strongest effect at 0.25 to 0.5%** |
| 0.5 | 7.1 | |
| 1 | 6.9 | **Flouriness. Slight unusual taste, and effect weakened by masking** |

### Example 8: Effect of Addition of C. casei Bacterial Cell-Containing Condiment to Cheese Sauce

The *C. casei* cell-containing seasoning prepared in Example 3 (3-5) was added in an amount of 0.1 to 0.3% by weight (corresponding to 0.007 to 0.021% by weight in terms of the amount of the cell) to a commercially available alfredo cheese sauce (BERUTOLLI alfredo cheese sauce; manufactured by Unilever Japan K.K.), thereby providing a *C. casei* cell-containing seasoning-added alfredo cheese sauce. The *C. casei* cell-containing seasoning-added alfredo cheese sauce was subjected to sensory evaluation according to the free word method by three expert panels with the *C*. *casei* cell-containing seasoning-free commercially available alfredo cheese sauce as a control article.

It was confirmed from the results of sensory evaluation that the *C*. *casei* cell-containing seasoning-added alfredo cheese sauce was enhanced in thickness, umami, and robustness as compared with the *C*. *casei* cell-containing seasoning-free alfredo cheese sauce. It was indicated from this result that addition of the *C*. *casei* cell-containing seasoning enhanced the thickness, umami, and robustness of cheese, and enhanced the preference of cheese-containing food. It was revealed from the foregoing that addition of the *C. casei* cell (for example, addition of the *C. casei* cell-containing fermented seasoning) provided a flavor-improving effect, for example, impartment of a rich taste in food.

### Example 9: Effect of Addition of C. casei Bacterial Cell-Containing Condiment to Tomato Sauce

The *C. casei* cell-containing seasoning prepared in Example 3 (3-5) was added in an amount of 0.1 to 0.3% by weight (corresponding to 0.007 to 0.021% by weight in terms of the amount of the cell) to a commercially available tomato sauce (manufactured by RAGU), thereby providing a *C. casei* cell-containing seasoning-added tomato sauce. The *C. casei* cell-containing seasoning-added tomato sauce was subjected to sensory evaluation according to the free word method by three expert panels with the *C. casei* cell-containing seasoning-free commercially available tomato sauce as a control article.

It was confirmed from the results of sensory evaluation that the *C*. *casei* cell-containing seasoning-added tomato sauce was enhanced in thickness, umami, complex taste, and ripe feeling as compared with the *C*. *casei* cell-containing seasoning-free tomato sauce. It was indicated from this result that addition of the *C*. *casei* cell-containing seasoning enhanced the thickness, umami, complex taste, and ripe feeling of tomatoes, and enhanced the preference of tomato-containing food. It was revealed from the foregoing that addition of the *C*. *casei* cell (for example, addition of the *C*. *casei* cell-containing fermented seasoning) provided a flavor-improving effect, for example, impartment of a rich taste in food.

### Example 10: Effect of Addition of C. casei Bacterial Cell-Containing Condiment to Curry Sauce

A curry sauce was obtained by adding 9.4 parts by weight of boiling water to 1 part by weight of a commercially available powder curry roux (manufactured by House Foods Corp.) and stirring the resulting mixture. The *C. casei* cell-containing seasoning prepared in Example 3 (3-5) was added in an amount of 0.1 to 0.3% by weight (corresponding to 0.007 to 0.021% by weight in terms of the amount of the cell) to this curry sauce, thereby providing a *C. casei* cell-containing seasoning-added curry sauce. The *C. casei* cell-containing seasoning-added curry sauce was subjected to sensory evaluation according to the free word method by three expert panels with the *C. casei* cell-containing seasoning-free curry sauce as a control article.

It was confirmed from the results of sensory evaluation that the *C*. *casei* cell-containing seasoning-added curry sauce was enhanced in thickness, umami, and pungency as compared with the *C. casei* cell-containing seasoning-free curry sauce. It was indicated from this result that addition of the *C. casei* cell-containing seasoning enhanced the thickness, umami, and pungency of curry, and enhanced the preference of curry. It was revealed from the foregoing that addition of the *C*. *casei* cell (for example, addition of the *C*. *casei* cell-containing fermented seasoning) provided flavor-improving effects, for example, impartment of a rich taste and enhancement of spiciness in food (for example, spice-containing food).

### Example 11: Examination of Optimal C. casei Bacterial Cell/Broth Ratio to Curry Sauce

A curry sauce was obtained by adding 9.4 parts by weight of boiling water to 1 part by weight of a commercially available powder curry roux (manufactured by House Foods Corp.) and stirring the resulting mixture. The *C. casei* broth seasoning prepared in Example 3 (3-6) was added in an amount of 0.2% by weight to this curry sauce, and these were stirred, thereby providing a *C. casei* broth seasoning-added curry sauce. The dry cell of the *C. casei* JCM 12072 strain prepared in Example 2 was added in an amount of 0.005, 0.01, 0.025, 0.05, 0.1, 0.25, 0.5, or 1% by weight to the *C. casei* broth seasoning-added curry sauce, and these were stirred, thereby providing a curry sauce to which the *C. casei* broth seasoning and the *C. casei* cell were added. The weight ratio of the amount of the *C. casei* cell added, to the amount of the *C. casei* broth seasoning added, is also referred to as "*C*. *casei* cell/broth ratio". The curry sauce thus obtained was subjected to sensory evaluation by three expert panels with *C. casei* broth seasoning-free and *C. casei* cell-free curry sauces as control articles. The sensory evaluation was performed with respect to each of the thickness impartment effect and the pungency enhancement effect, according to the scoring system in the range of scores 0 to 10 (a higher score indicated a higher effect and score 1 or higher was determined to be effective). The pungency enhancement effect was scored under the assumption that the intensity of pungency of the control article was score 0 and the intensity of pungency, corresponding to 2-fold that of the control article, was score 5.

Table 11 shows the results. In the Table, "Score" represents the average value of scores by three expert panels. While no pungency enhancement effect was observed in the case of addition of only the *C*. *casei* broth seasoning, the pungency enhancement effect was observed in the case of addition of the *C*. *casei* broth seasoning and the *C*. *casei* cell. The case of addition of the *C*. *casei* broth seasoning and the *C. casei* cell resulted in increase in score of pungency enhancement at each concentration of the *C. casei* cell added, namely, enhancement of the pungency enhancement effect, as compared with the case of addition of only the *C. casei* cell (Example 6; Table 9). Specifically, in the case of use of the *C. casei* broth seasoning in combination with the *C. casei* cell, the pungency enhancement effect was observed at a concentration of the *C. casei* cell added, of 0.005% by weight or more. If the concentration of the *C. casei* cell added was 1% by weight, a tendency was demonstrated in which an unusual taste and a bitter taste were generated to result in the change in quality of taste. It was indicated from the foregoing results that in the case of use of the *C. casei* broth seasoning in combination with the *C. casei* cell, the effect of enhancing pungency was exerted without impartment of any unusual taste by adding 0.005 to 0.5% by weight of the *C. casei* cell to the curry sauce. Specifically, when the *C. casei* cell/broth ratio was 0.025 to 2.5, the pungency enhancement effect was observed. If the *C. casei* cell/broth ratio reached 5, a tendency was demonstrated in which an unusual taste was generated to result in the change in quality of taste. It was indicated from the foregoing results that in the case of use of the *C. casei* broth seasoning in combination with the *C. casei* cell, the effect of enhancing pungency was exerted without impartment of any unusual taste by adding the *C*. *casei* broth seasoning and the *C*. *casei* cell at a *C*. *casei* cell/broth ratio of 0.05 to 2.5, to the curry sauce. It was revealed from the foregoing that addition of the *C*. *casei* cell provided a flavor-improving effect, for example, enhancement of spiciness in food (for example, spice-containing food), and use of the *C*. *casei* broth seasoning in combination with the *C*. *casei* cell (for example, combination at a *C*. *casei* cell/broth ratio of 0.025 to 2.5) further enhanced a flavor-improving effect, for example, enhancement of spiciness.

**Table 11**

| **Concentration (%) of broth added** | **Concentration (%) of bacterial cell added** | **Bacterial Cell /Broth Ratio** | **Score** | **Comments** |
|---|---|---|---|---|
| | | | **Enhancement of pungency** | |
| 0.2 | 0 | 0 | 0.0 | **Umami enhanced. Mild** |
| | 0.005 | 0.025 | 1.2 | **Slight pungency generated** |
| | 0.01 | 0.05 | 2.5 | **Enhancement of pungency** |
| | 0.025 | 0.125 | 4.2 | **Enhancement of pungency** |
| | 0.05 | 0.25 | 5.7 | **Enhancement of pungency** |
| | 0.1 | 0.5 | 6.5 | **Enhancement of pungency** |
| | 0.25 | 1.25 | 7.1 | **Enhancement of pungency** |
| | 0.5 | 2.5 | 7.6 | **Pungency enhanced. Slight flouriness and unusual taste** |
| | 1 | 5 | 7.6 | **Pungency enhanced. Unusual taste generated** |

### Example 12: Examination of Optimal C. casei Bacterial Cell/Broth Ratio to Black Pepper-Containing Mashed Potato

Mashed potato was obtained by adding 6 parts by weight of boiling water to 1 part by weight of commercially available dry mashed potato (manufactured by Calbee, Inc.), and stirring them. A commercially available black pepper powder (manufactured by House Gaban Corporation) was added in an amount of 0.15% by weight to the mashed potato, and these were stirred, thereby providing black pepper-containing mashed potato. The *C*. *casei* broth seasoning prepared in Example 3 (3-6) was added in an amount of 0.2% by weight to the black pepper-containing mashed potato, and these were stirred, thereby providing *C*. *casei* broth seasoning-added black pepper-containing mashed potato. The dry cell of the *C*. *casei* JCM 12072 strain prepared in Example 2 was added in an amount of 0.005, 0.01, 0.025, 0.05, 0.1, 0.25, 0.5, or 1% by weight to the *C*. *casei* broth seasoning-added black pepper-containing mashed potato, and these were stirred, thereby providing black pepper-containing mashed potato to which the *C*. *casei* broth seasoning and the *C*. *casei* cell were added. The black pepper-containing mashed potato thus obtained was subjected to sensory evaluation by three expert panels with the *C. casei* broth seasoning-free black pepper-containing mashed potato and *C. casei* cell-free black pepper-containing mashed potato as control articles. The sensory evaluation was performed with respect to each of the thickness impartment effect and the pungency enhancement effect, according to the scoring system in the range of scores 0 to 10 (a higher score indicated a higher effect and score 1 or higher was determined to be effective). The pungency enhancement effect was scored under the assumption that the intensity of pungency of the control article was score 0 and the intensity of pungency of 0.3% by weight black pepper-containing mashed potato was score 5.

Table 12 shows the results. In the Table, "Score" represents the average value of scores by three expert panels. While no pungency enhancement effect was observed in the case of addition of only the *C. casei* broth seasoning, the pungency enhancement effect was observed in the case of addition of the *C. casei* broth seasoning and the *C. casei* cell. The case of addition of the *C. casei* broth seasoning and the *C. casei* cell resulted in increase in score of pungency enhancement at each concentration of the *C. casei* cell added, namely, enhancement of the pungency enhancement effect, as compared with the case of addition of only the *C*. *casei* cell (Example 7; Table 10). Specifically, in the case of use of the *C*. *casei* broth seasoning in combination with the *C*. *casei* cell, the pungency enhancement effect was observed at a concentration of the *C*. *casei* cell added, of 0.01% by weight or more. If the concentration of the *C*. *casei* cell added was 1% by weight, a tendency was demonstrated in which an unusual taste and a bitter taste were generated to result in the change in quality of taste. It was indicated from the foregoing results that in the case of use of the *C*. *casei* broth seasoning in combination with the *C*. *casei* cell, the effect of enhancing pungency was exerted without impartment of any unusual taste by adding of 0.01 to 0.5% by weight of the *C. casei* cell, to the black pepper-containing mashed potato. Specifically, when the *C. casei* cell/broth ratio was 0.025 to 2.5, the pungency enhancement effect was observed. If the *C*. *casei* cell/broth ratio reached 5, a tendency was demonstrated in which an unusual taste was generated to result in the change in quality of taste. It was indicated from the foregoing results that in the case of use of the *C. casei* broth seasoning in combination with the *C. casei* cell, the effect of enhancing pungency was exerted without impartment of any unusual taste by addition to the black pepper-containing mashed potato at a *C. casei* cell/broth ratio of 0.05 to 2.5. It was revealed from the foregoing that addition of the *C. casei* cell provided a flavor-improving effect, for example, enhancement of spiciness in food (for example, spice-containing food), and use of the *C. casei* broth seasoning in combination with the *C. casei* cell (for example, combination at a *C. casei* cell/broth ratio of 0.025 to 2.5) further enhanced a flavor-improving effect, for example, enhancement of spiciness.

**Table 12**

| **Concentration (%) of broth added** | **Concentration (%) of bacterial cell added** | **Bacterial Cell /Broth Ratio** | **Score** | **Comments** |
|---|---|---|---|---|
| | | | **Enhancement of pungency** | |
| 0.2 | 0 | 0 | 0.0 | **Umami slightly increased, but no change in pungency** |
| | 0.005 | 0.025 | 0.9 | **Pungency slightly enhanced** |
| | 0.01 | 0.05 | 2.8 | **Enhancement of pungency** |
| | 0.025 | 0.125 | 4.0 | **Enhancement of pungency** |
| | 0.05 | 0.25 | 5.3 | **Pungency enhanced. Umami/pungency well-balanced** |
| | 0.1 | 0.5 | 6.2 | **Enhancement of pungency** |
| | 0.25 | 1.25 | 6.8 | **Enhancement of pungency** |
| | 0.5 | 2.5 | 7.2 | **Pungency enhanced. Slight flouriness and unusual taste.** |
| | 1 | 5 | 7.5 | **Pungency enhanced. Flouriness and unusual taste** |

### Example 13: Preparation of Dry Bacterial Cells of Various Bacteria

(13-1)
Culture of *Bacillus subtilis* JCM 1465 was performed by jar culture with a medium shown in Table 13. The culture temperature was regulated at constant 30°C, the culture pH was regulated by ammonia gas so as to be 6.8, and the concentration of dissolved oxygen was regulated by control with stirring so as to be 23% or more as a relative value under the assumption that the concentration of saturated dissolved oxygen was 100%. The resulting culture solution was subjected to sterilization treatment under two-stage conditions of at 80°C for 60 minutes and 121°C for 60 minutes, and a medium ingredient was removed by centrifugation, thereby providing a cell pellet. Furthermore, an operation involving placing water in the same amount as that of the medium to wash the cell and removing the supernatant by centrifugation was repeated twice to perform washing of the cell. The cell pellet after washing was frozen at -80°C, and then moisture was removed by a lyophilizer, thereby acquiring a dry cell of *Bacillus subtilis* JCM 1465.

**Table 13**

| Component | **Final Concentration** |
|---|---|
| Pea peptone | 10 g/L |
| Yeast Extract | 10 g/L |
| **Glucose** | 60 g/L |
| Biotin | 3 mg/L |
| KH₂PO₄ | 1 g/L |
| **Ammonium Sulfate** | 1 g/L |
| **Defoamer (OD-50D)** | 0.2 mL/L |

(13-2)
Culture of *Gluconacetobacter kombuchae* NBRC 14820 was performed by penetration at 30°C for 24 hours in a Sakaguchi flask where 50 mL of a medium shown in Table 14 was stuck. The resulting culture solution was subjected to sterilization treatment at 80°C for 30 minutes, and a medium ingredient was removed by centrifugation, thereby providing a cell pellet. Furthermore, an operation involving placing water in the same amount as that of the medium to wash the cell and removing the supernatant by centrifugation was repeated twice to perform washing of the cell. The cell pellet after washing was frozen at -80°C, and then moisture was removed by a lyophilizer, thereby acquiring a dry cell of *Gluconacetobacter kombuchae* NBRC 14820.

**Table 14**

| Component | **Final Concentration** |
|---|---|
| Potato peptone | 10 g/L |
| Yeast Extract | 10 g/L |
| **Glucose** | 10 g/L |

### (13-3) Preparation of Dry Bacterial Cells of Brevibacterium casei, Corynebacterium flavescens, and Brachybacterium alimentarium

Culture of *Brevibacterium casei* DSM 20657, *Corynebacterium flavescens* NBRC 14136, *Brachybacterium alimentarium* NBRC 16118, *Lactobacillus hilgardii* NBRC 15886, and *Lactobacillus Brevis* JCM 1102 was performed by rotating and turning at 30°C for 24 hours in an obliquely-pleated 300-mL conical flask where 100 mL of a medium shown in Table 15 was stuck. Culture of *Lactobacillus* mali NBRC 102159 was performed by rotating and turning at 30°C for 48 hours in an obliquely-pleated 300-mL conical flask where 100 mL of a medium shown in Table 15, with 20 g/L of coconut oil further added, was stuck. The resulting culture solution was subjected to sterilization treatment at 120°C for 20 minutes, and a medium ingredient was removed by centrifugation, thereby providing a cell pellet. Furthermore, an operation involving placing water in the same amount as that of the medium to wash the cell and removing the supernatant by centrifugation was repeated twice to perform washing of the cell. The cell pellet after washing was frozen at -80°C, and then moisture was removed by a lyophilizer, thereby acquiring each dry cell of these bacteria.

**Table 15**

| Component | **Final Concentration** |
|---|---|
| Potato peptone | 10 g/L |
| Yeast Extract | 20 g/L |
| **Glucose** | 10 g/L |
| NaCl | 5 g/L |

(13-4)
Excipients were removed from sterilized cell EC-12 of *Enterococcus faecalis* and sterilized cell BR-108 of *Bifidobacterium longum* (both manufactured by Combi Corporation), and dry cells of these bacteria were acquired.

### Example 14: Effect of Addition of Each cell of Various Bacteria to Curry Sauce

A curry sauce was obtained by adding 9.4 parts by weight of boiling water to 1 part by weight of a commercially available powder curry roux (manufactured by House Foods Corp.) and stirring the resulting mixture. The dry cell of each bacterium acquired in Example 13 was added to this curry sauce so that the concentration was 0.1%, and suspension was performed, thereby providing a cell-added curry sauce. The cell-added curry sauce was subjected to sensory evaluation by four expert panels with the cell-free curry sauce as a control article. The sensory evaluation was performed with respect to the pungency enhancement effect, according to the scoring system in the range of scores 0 to 10 (a higher score indicated a higher effect and score 1 or higher was determined to be effective). The pungency enhancement effect was scored under the assumption that the intensity of pungency of the control article was score 0 and the intensity of pungency, corresponding to 2-fold that of the control article, was score 5.

Table 16 shows the results. In the Table, "Score" represents the average value of scores by four expert panels. Almost no pungency enhancement effect was observed in the case of addition of the cell of the gram-negative bacterium. On the other hand, the pungency enhancement effect was observed in the case of addition of the cell of each gram-positive bacterium. In particular, a remarkable pungency enhancement effect was observed in the case of the cell of each bacterium in the *Actinobacteria* division. It was revealed from the foregoing that addition of the cell of each of the gram-positive bacteria (particularly bacteria in the *Actinobacteria* division) provided a flavor-improving effect, for example, enhancement of spiciness in food (for example, spice-containing food).

**Table 16**

| **Gram Staining** | **Division** | **Type of Bacterium** | **Score** |
|---|---|---|---|
| | | | **Enhancement of pungency** |
| Negative | Proteobacteria | Gluconacetobacter kombuchae | 0.2 |
| Positive | Firmicutes | Bacillus subtilis | 1.7 |
| | Firmicutes | Enterococcus faecal is | 1.25 |
| | Firmicutes | Lactobacillus mali | 1.46 |
| | Firmicutes | Lactobacillus hilgardii | 1.67 |
| | Firmicutes | Lactobacillus Brevis | 1.46 |
| | Actinobacteria | Corynebacterium casei | 5.0 |
| | Actinobacteria | Corynebacterium flavescens | 5.1 |
| | Actinobacteria | Bifidobacterium longum | 5.0 |
| | Actinobacteria | Brevibacterium casei | 5.0 |
| | Actinobacteria | Brachybacterium alimentarium | 4.7 |

### Example 14: Evaluation of Influence of Enzyme Treatment of Bacterial Cell

A dry cell of a *C. casei* JCM 12072 strain (one prepared in the same manner as in Example 2) was subjected to enzyme treatment with lysozyme or protease, according to the following procedure. In other words, 50 mg of the dry cell was suspended in 4.5 mL of distilled water, 0.5 mL of an enzyme liquid was added, and the pH was adjusted to 6.5 with 1 M hydrochloric acid or 1 M sodium hydroxide. Such a mixed liquid was incubated at 45°C for 5 hours to perform enzymatic reaction. After enzymatic reaction, centrifugation was performed under conditions of 20°C and 2500 rpm, and a precipitate was obtained. To the precipitate obtained was added 5 mL of distilled water, and a centrifugation operation was performed under the same conditions as described above. Such washing with distilled water and centrifugation were additionally repeated twice, thereby providing an enzyme-treated cell. Herein, the enzyme liquid used as a lysozyme solution was an aqueous 1% lysozyme solution ("lysozyme BIO" manufactured by BIOCON (JAPAN) LTD., dissolved in distilled water at a proportion of 1%), and that used as a protease solution was an aqueous 0.1% papain solution ("Papain W-40" manufactured by Amano Enzyme Inc., dissolved in distilled water at a proportion of 0.1%). The dry cell before enzyme treatment is also referred to as "untreated cell".

Mashed potato was obtained by adding 6 parts by weight of boiling water to 1 part by weight of commercially available dry mashed potato (manufactured by Calbee, Inc.), and stirring them. A commercially available chili pepper powder was added in an amount of 0.2% by weight to this mashed potato, and these were stirred, thereby providing 0.2% by weight chili pepper-containing mashed potato. The pungency intensity was evaluated by adding to the chili pepper-containing mashed potato, the untreated cell in an amount of 0.1% by weight or the enzyme-treated cell in an amount of 0.1% by weight, as converted into the amount of the original cell. The pungency intensity was evaluated according to sensory evaluation by two expert panels under the assumption that the pungency intensity of 0.2% by weight chili pepper-containing mashed potato was score 0 and the pungency intensity of 0.4% by weight chili pepper-containing mashed potato was score 100.

Table 17 shows the results. The pungency intensity of the untreated cell-added sample exhibited score 100. The pungency intensity of the protease-treated cell-added sample exhibited score 90, and almost no depression of the pungency enhancement effect by protease treatment was observed. On the other hand, the pungency intensity of the lysozyme-treated cell-added sample exhibited score 20, and the pungency enhancement effect was remarkably depressed by lysozyme treatment.

A curry sauce was obtained by adding 400 mL of boiling water to 28 g of a commercially available curry roux ("Java Curry PRIME, low-calorie" manufactured by House Foods Corp.), and stirring them. The pungency intensity was evaluated by adding to the curry sauce, the untreated cell in an amount of 0.1% by weight or the enzyme-treated cell in an amount of 0.1% by weight, as converted into the amount of the original cell. The pungency intensity was evaluated according to sensory evaluation by three expert panels under the assumption that the pungency intensity of a cell-free curry sauce was score 0 and the pungency intensity in addition of 0.1% by weight of the untreated cell was score 100.

Table 18 shows the results. The pungency intensity of the protease-treated cell-added sample exhibited score 95 and almost no depression of pungency enhancement effect by protease treatment was observed. On the other hand, the pungency intensity of the lysozyme-treated cell-added sample exhibited score 20, and the pungency enhancement effect was remarkably depressed by lysozyme treatment.

It was supposed from these results that peptide glycan as one ingredient constituting a cell wall of a gram-positive bacterium contributed to a flavor-improving effect of a cell of a gram-positive bacterium, for example, enhancement of spiciness.

**Table 17 Pungency enhancement effect in 0.2% chili pepper-containing mashed potato**

| **Bacterial cell added** | | **Pungency intensity** | **Comments** |
|---|---|---|---|
| | **NC (Not added)** | 0 | |
| **Untreated bacterial cell** | | 100 | **Initial to after pungency enhanced about 2-fold** |
| | **Protease-treated bacterial cell** | 90 | **Initial to middle pungency enhanced. Slightly weak as compared with untreated one.** |
| | **Lysozyme-treated bacterial cell** | 20 | **Pungency enhancement effect lowered to the same level as NC.** |

| | | | |
|---|---|---|---|
| **(Evaluation by persons in charge, n=2, Council system)** | | | |

**Table 18 Pungency enhancement effect in curry sauce**

| **Bacterial cell added** | | **Pungency intensity** | **Comments** |
|---|---|---|---|
| | **NC (Not added)** | 0 | |
| **Untreated bacterial cell** | | 100 | **Initial to after pungency enhanced about 2-fold** |
| | **Protease-treated bacterial cell** | 95 | **Initial to middle pungency enhanced. Slightly weak as compared with untreated one.** |
| | **Lysozyme-treated bacterial cell** | 20 | **Pungency enhancement effect lowered to the same level as NC.** |

| | | | |
|---|---|---|---|
| **(Evaluation by persons in charge, n=3, Council system)** | | | |

### Example 15: Preparation and Sensory Evaluation of Fermented Product of Tomato Juice with C. casei

First, seed culture of a *C. casei* JCM 12072 strain and an RUN5-2-96 strain (NITE ABP-03688) was performed by flask culture with a medium shown in Table 19. The culture temperature was regulated at constant 30°C, and culture for 30 hours was performed under a rotating and stirring condition of 160 rpm. After the termination of culture, a cell was separated from the culture solution by centrifugation. A washing operation involving suspending the cell in saline in an amount equal to that of the medium and again centrifugating the resultant was repeated three times. A medium ingredient was removed by this washing operation, and living cells of the JCM 12072 strain and the RUN5-2-96 strain were obtained.

**Table 19**

| Component | **Final Concentration** |
|---|---|
| Potato peptone | 10 g/L |
| KH₂PO₄ | 5.2 g/L |
| K₂HPO4 | 10.7 g/L |
| MgSO₄ • 7H₂O | 0.4 g/L |
| FeSO₄ • 7H₂O | 10 mg/L |
| **Bean concentrated** | 1200 mg/L |
| Biotin | 3.2 mg/L |
| Glucose | 2.5 g/L |
| Yeast Extract | 5 g/L |

Next, jar culture with a commercially available salt-free tomato juice (manufactured by KAGOME CO., LTD.) as a medium was performed with living cells of a JCM 12072 strain and an RUN5-2-96 strain (AJ111891). The living cell of each of the strains was inoculated so that OD620 reached 4. The pH in culture was regulated by intermittent addition of sodium hydroxide so as to be within the range of 5.5 to 7.5, the culture temperature was regulated to 30°C, and thus culture for 16 hours was performed. The culture solution was sampled after 0 hours, 8 hours, and 16 hours from the start of culture, and a *C*. *casei* tomato juice fermented product was obtained.

A *C*. *casei* tomato juice fermented product-added tomato sauce was prepared by adding 0.5% of such each *C*. *casei* tomato juice fermented product to a commercially available tomato sauce (manufactured by RAGU). The *C*. *casei* tomato juice fermented product-added tomato sauce was subjected to sensory evaluation with a *C. casei* tomato juice fermented product-free tomato sauce as a control article.

Table 20 shows the results. In the Table, "Evaluation" indicates that A represents the highest rating and C represent the lowest rating in the order of A > B > C. It was confirmed that the *C. casei* tomato juice fermented product-added tomato sauce was enhanced in umami, thickness of tomato, flavor, meat feeling, and/or spiciness as compared with the *C. casei* tomato juice fermented product-free tomato sauce (control article) (Nos. 2 to 3 and Nos. 5 to 6). It was indicated from this result that addition of the *C. casei* tomato juice fermented product enhanced the preference of tomato-containing food. The results are also matched with the results of Example 9. It was revealed from the foregoing that addition of the tomato juice fermented product prepared with *C. casei* provided a flavor-improving effect in food.

**Table 20**

| No. | **Sample** | | | **Rating** | **Comments** |
|---|---|---|---|---|---|
| | **Strain** | **Culture time** (hr) | **Glu concentration** (g/L) | | |
| 1 | RUN 5-2-96 **Strain** | 0 | 1.5 | C | **Flavor substantially comparable with control article.** |
| 2 | | 8.0 | 5.7 | A | **Umami imparted to middle taste and aftertaste. Strong initial taste and flavoring as compared with No.3.** |
| 3 | | 16.0 | 4.5 | B | **Flavor close to No.2, but flavor harmonized to provide muffled feeling as compared with No.2.** |
| 4 | JCM 12072 **Strain** | 0 | 1.5 | C | **Flavor substantially comparable with control article.** |
| 5 | | 8.0 | 0 | B | **Thickness imparted to middle taste and aftertaste. Meat feeling imparted** |
| 6 | | 16.0 | 0 | B | **Thickness imparted to middle taste and aftertaste. Spiciness such as basil flavor also enhanced.** |

### INDUSTRIAL AVAILABILITY

The present invention can improve the flavor of food.

## Claims

1. A composition for improving a flavor of food, comprising the following ingredient (A):
(A) a cell wall-containing fraction of a gram-positive bacterium.

2. The composition according to claim 1, wherein the improvement of the flavor is enhancement of spiciness and/or impartment of a rich taste.

3. The composition according to claim 1 or 2, wherein the improvement of the flavor is enhancement of pungency of a spice.

4. The composition according to any one of claims 1 to 3, further comprising a spice.

5. A composition comprising the following ingredient (A) and a spice:
(A) a cell wall-containing fraction of a gram-positive bacterium.

6. The composition according to any one of claims 1 to 5, wherein the ingredient (A) is a cell of the gram-positive bacterium, or a fragment thereof.

7. The composition according to any one of claims 1 to 6, wherein the gram-positive bacterium is a bacterium belonging to the *Actinobacteria* division or the *Firmicutes* division.

8. The composition according to any one of claims 1 to 7, wherein the gram-positive bacterium is a bacterium belonging to the *Actinobacteria* division.

9. The composition according to any one of claims 1 to 8, wherein the gram-positive bacterium is selected from the group consisting of a coryneform bacterium, a bacterium belonging to the *Bifidobacteriaceae* family, a bacterium belonging to the *Dermabacteraceae* family, a bacterium belonging to the *Bacillaceae* family, a bacterium belonging to the *Enterococcaceae* family, or a bacterium belonging to the *Lactobacillaceae* family.

10. The composition according to any one of claims 1 to 9, wherein the gram-positive bacterium is selected from the group consisting of a *Corynebacterium* genus bacterium, a *Brevibacterium* genus bacterium, a *Bifidobacterium* genus bacterium, a *Brachybacterium* genus bacterium, a *Bacillus* genus bacterium, an *Enterococcus* genus bacterium, or a *Lactobacillus* genus bacterium.

11. The composition according to any one of claims 1 to 10, wherein the gram-positive bacterium is selected from the group consisting of *Corynebacterium casei, Corynebacterium flavescens, Brevibacterium casei, Bifidobacterium longum, Brachybacterium alimentarium, Bacillus subtilis*, *Enterococcus faecalis*, *Lactobacillus mali, Lactobacillus hilgardii,* or *Lactobacillus Brevis.*

12. The composition according to any one of claims 1 to 11, wherein the amount of the ingredient (A) contained, converted into the dry mass of a cell of the gram-positive bacterium, is 0.1% (w/w) or more.

13. The composition according to any one of claims 1 to 12, further comprising the following ingredient (B):
(B) one or more ingredients selected from the group consisting of L-amino acid, nucleic acid, and organic acid.

14. The composition according to claim 13, further comprising at least the L-amino acid, wherein the L-amino acid is L-glutamic acid.

15. The composition according to claim 14, wherein the amount of L-glutamic acid contained is 0.1 to 20 parts by weight based on 1 part by weight of the ingredient (A) converted into the dry mass of a cell of the gram-positive bacterium.

16. The composition according to any one of claims 4 to 15, wherein the amount of the spice contained is 0.2 to 500 parts by weight based on 1 part by weight of the ingredient (A) converted into the dry mass of a cell of the gram-positive bacterium.

17. The composition according to any one of claims 4 to 16, wherein the spice is one or more spices selected from the group consisting of spices of the *Lauraceae* family, spices of the *Piperaceae* family, spices of the *Lamiaceae* family, spices of the *Apiaceae* family, spices of the *Solanaceae* family, spices of the *Myristicaceae* family, spices of the *Alliaceae* family, spices of the *Myrtaceae* family, spices of the *Schisandraceae* family, spices of the *Fabaceae* family, spices of the *Polygonaceae* family, spices of the *Brassicaceae* family, spices of the *Zingiberaceae* family, and spices of the *Rutaceae* family.

18. The composition according to any one of claims 4 to 17, wherein the spice is a spice having pungency.

19. The composition according to any one of claims 1 to 18, wherein the composition is a seasoning.

20. The composition according to any one of claims 1 to 19, wherein the ingredient (A) is produced by culturing the gram-positive bacterium in a medium containing a raw material of food.

21. The composition according to claim 20, wherein the raw material contained in the medium is a tomato.

22. The composition according to any one of claims 1 to 21, wherein the ingredient (A) is heat-treated.

23. The composition according to any one of claims 1 to 22, wherein the gram-positive bacterium is a bacterium which has an L-glutamic acid-producing ability and which has one or more mutations selected from mutations shown in Table 1.
**Table 1**
| No. | **Position on genome** | **Base before mutation** | **Base after mutation** |
|---|---|---|---|
| A-1 | 78,486 | C | T |
| A-2 | 83,592 | G | A |
| A-3 | 87,955 | C | T |
| A-4 | 90,041 | C | T |
| A-5 | 186,221 | C | T |
| A-6 | 193,010 | C | T |
| A-7 | 196,531 | C | T |
| A-8 | 225,429 | C | T |
| A-9 | 297,920 | G | A |
| A-10 | 320,354 | C | T |
| A-11 | 335,878 | C | T |
| A-12 | 341,763 | C | T |
| A-13 | 346,969 | C | T |
| A-14 | 349,856 | C | T |
| A-15 | 356,232 | C | T |
| A-16 | 357,008 | C | T |
| A-17 | 366,674 | G | A |
| A-18 | 369,871 | G | A |
| A-19 | 377,420 | G | A |
| A-20 | 378,652 | G | A |
| A-21 | 432,252 | C | A |
| A-22 | 439,021 | G | A |
| A-23 | 440,764 | G | A |
| A-24 | 454,682 | G | A |
| A-25 | 458,729 | G | A |
| A-26 | 470,562 | G | A |
| A-27 | 471,288 | G | A |
| A-28 | 472,023 | G | A |
| A-29 | 504,885 | G | A |
| A-30 | 505,785 | G | A |
| A-31 | 514,371 | G | A |
| A-32 | 518,684 | G | A |
| A-33 | 521,126 | G | A |
| A-34 | 524,551 | G | A |
| A-35 | 660,841 | C | T |
| A-36 | 732,121 | C | T |
| A-37 | 787,055 | C | T |
| A-38 | 806,047 | C | T |
| A-39 | 872,482 | G | A |
| A-40 | 878,069 | C | T |
| A-41 | 903,037 | C | T |
| A-42 | 922,802 | C | T |
| A-43 | 948,145 | C | T |
| A-44 | 955,819 | C | T |
| A-45 | 968,915 | C | T |
| A-45 | 973,013 | C | T |
| A-47 | 974,797 | C | T |
| A-4.$ | 994,815 | C | T |
| A-49 | 1,000,498 | C | T |
| A-50 | 1,019,704 | C | T |
| A-51 | 1,049,052 | C | T |
| A-52 | 1,069,322 | C | T |
| A-53 | 1,070,554 | C | T |
| A-54 | 1,131,016 | C | T |
| A-55 | 1,138,639 | C | T |
| A-56 | 1,162,588 | C | T |
| A-57 | 1,193,273 | C | T |
| A-58 | 1,203,146 | C | T |
| A-59 | 1,222,633 | C | T |
| A-60 | 1,226,969 | G | A |
| A-61 | 1,264,895 | G | A |
| A-62 | 1,268,790 | G | A |
| A-63 | 1,279,676 | G | A |
| A-64 | 1,363,909 | T | C |
| A-65 | 1,387,476 | G | A |
| A-66 | 1,401,171 | G | A |
| A-67 | 1,416,228 | C | T |
| A-68 | 1,420,034 | C | T |
| A-69 | 1,447,494 | C | T |
| A-70 | 1,448,318 | C | T |
| A-71 | 1,448,776 | C | T |
| A-72 | 1,451,922 | C | T |
| A-73 | 1,466,961 | C | T |
| A-74 | 1,503,736 | C | T |
| A-75 | 1,504,207 | C | T |
| A-76 | 1,505,998 | C | T |
| A-77 | 1,507,027 | C | T |
| A-78 | 1,544,310 | C | T |
| A-79 | 1,554,973 | C | T |
| A-80 | 1,558,509 | C | T |
| A-81 | 1,552,459 | C | T |
| A-82 | 1,572,716 | C | T |
| A-83 | 1,594,314 | C | T |
| A-84 | 1,602,545 | C | T |
| A-85 | 1,659,808 | C | T |
| A-86 | 1,682,132 | C | T |
| A-87 | 1,689,863 | C | T |
| A-88 | 1,744,963 | C | T |
| A-89 | 1,784,642 | C | T |
| A-90 | 1,814,866 | C | T |
| A-91 | 1,829,145 | C | T |
| A-92 | 1,852,511 | G | A |
| A-93 | 1,861,170 | G | A |
| A-94 | 1,902,133 | G | A |
| A-95 | 1,916,048 | C | T |
| A-96 | 1,917,434 | C | T |
| A-97 | 1,938,271 | C | T |
| A-98 | 1,949,357 | G | T |
| A-99 | 1,954,368 | C | T |
| A-100 | 1,967,997 | C | T |
| A-101 | 1,975,599 | C | T |
| A-102 | 2,141,466 | C | T |
| A-103 | 2,308,064 | C | T |
| A-104 | 2,310,428 | C | T |
| A-105 | 2,354,420 | C | T |
| A-106 | 2,449,270 | T | C |
| A-107 | 2,449,278 | C | A |
| A-108 | 2,449,291 | G | C |
| A-109 | 2,449,318 | G | A |
| A-110 | 2,496,945 | C | T |
| A-111 | 2,505,022 | C | T |
| A-122 | 2,505,285 | C | T |
| A-113 | 2,525,513 | G | A |
| A-124 | 2,565,856 | C | T |
| A-115 | 2,601,306 | G | A |
| A-116 | 2,615,688 | G | A |
| A-117 | 2,650,740 | G | A |
| A-118 | 2,653,259 | G | A |
| A-119 | 2,663,827 | G | A |
| A-120 | 2,667,322 | G | A |
| A-121 | 2,674,077 | G | A |
| A-122 | 2,679,915 | G | A |
| A-123 | 2,686,979 | G | A |
| A-124 | 2,693,950 | C | T |
| A-125 | 2,696,737 | C | T |
| A-126 | 2,706,442 | C | T |
| A-127 | 2,709,469 | C | T |
| A-128 | 2,711,214 | C | T |
| A-129 | 2,714,651 | C | T |
| A-130 | 2,721,339 | G | A |
| A-131 | 2,731,030 | G | A |
| A-132 | 2,746,202 | G | A |
| A-133 | 2,805,389 | C | T |
| A-134 | 2,816,733 | G | A |
| A-135 | 2,827,114 | G | A |
| B-1 | 29,724 | G | A |
| B-2 | 92,869 | G | A |
| B-3 | 116,733 | G | A |
| B-4 | 131,184 | G | A |
| B-5 | 156,247 | G | A |
| B-6 | 177,083 | G | A |
| B-7 | 184,379 | G | A |
| B-8 | 212,586 | G | A |
| B-9 | 282,162 | G | A |
| 8-10 | 309,483 | G | A |
| B-11 | 376,164 | C | T |
| B-12 | 440,885 | C | T |
| B-13 | 479,120 | G | A |
| B-14 | 722,430 | G | A |
| 8-15 | 745,504 | G | A |
| B-16 | 809,993 | G | A |
| B-17 | 859,643 | G | A |
| B-18 | 923,209 | G | A |
| B-19 | 924,973 | G | A |
| B-20 | 998,893 | C | T |
| B-21 | 1,062,144 | C | T |
| B-22 | 1,095,062 | C | T |
| B-23 | 1,102,484 | C | T |
| B-24 | 1,103,812 | C | T |
| B-25 | 1,105,749 | C | T |
| B-26 | 1,107,561 | C | T |
| B-27 | 1,205,722 | C | T |
| B-28 | 1,233,449 | C | T |
| B-29 | 1,242,484 | C | T |
| B-30 | 1,248,388 | C | T |
| B-31 | 1,249,270 | C | T |
| B-32 | 1,291,377 | C | T |
| B-33 | 1,308,597 | C | T |
| B-34 | 1,329,535 | C | T |
| B-35 | 1,367,486 | C | T |
| B-36 | 1,382,065 | C | T |
| B-37 | 1,403,043 | C | T |
| B-38 | 1,433,914 | C | T |
| B-39 | 1,442,447 | C | T |
| B-40 | 1,501,903 | G | A |
| B-41 | 1,504,744 | C | T |
| B-42 | 1,651,403 | G | A |
| B-43 | 1,695,473 | G | A |
| B-44 | 1,779,939 | G | A |
| B-45 | 1,797,452 | G | A |
| B-46 | 1,801,284 | G | A |
| B-47 | 1,816,679 | G | A |
| B-48 | 1,832,252 | G | A |
| B-49 | 1,843,841 | G | A |
| B-50 | 1,868,285 | G | A |
| B-51 | 1,879,922 | G | A |
| B-52 | 1,892,007 | G | A |
| B-53 | 1,916,016 | G | A |
| B-54 | 1,937,604 | G | A |
| B-55 | 1,947,044 | G | A |
| B-56 | 1,948,411 | G | A |
| B-57 | 1,948,649 | G | A |
| B-58 | 1,967,697 | G | A |
| B-59 | 1,974,137 | G | A |
| B-60 | 2,028,284 | C | T |
| B-61 | 2,050,998 | G | A |
| B-62 | 2,052,708 | G | A |
| B-63 | 2,054,372 | G | A |
| B-64 | 2,065,568 | G | A |
| B-65 | 2,067,167 | G | A |
| B-66 | 2,082,577 | G | A |
| B-67 | 2,121,006 | A | G |
| B-68 | 2,149,369 | C | T |
| B-69 | 2,159,680 | G | A |
| B-70 | 2,380,965 | G | A |
| B-71 | 2,477,728 | G | A |
| B-72 | 2,542,800 | G | A |
| B-73 | 2,570,107 | G | A |
| B-74 | 2,647,383 | G | A |
| B-75 | 2,726,248 | C | T |
| B-76 | 2,825,055 | C | T |
| B-77 | 2,837,078 | C | T |
| B-78 | 2,865,322 | C | T |
| B-79 | 2,872,907 | C | T |
| B-80 | 2,880,351 | C | T |
| B-81 | 2,889,394 | C | T |
| B-82 | 2,906,471 | C | T |
| B-83 | 2,927,044 | C | T |
| B-84 | 2,929,963 | C | T |
| B-85 | 2,940,673 | C | T |
| B-86 | 2,946,285 | C | T |
| B-87 | 2,962,909 | C | T |
| B-88 | 2,975,742 | C | T |
| B-89 | 2,987,052 | C | T |
| B-90 | 3,079,560 | C | T |
| B-91 | 3,083,927 | C | T |
| B-92 | 3,090,163 | C | T |

24. The composition according to any one of claims 1 to 23, further comprising a culture product of the gram-positive bacterium.

25. A method of improving a flavor of food, the method comprising:
a step of adding the following ingredient (A) to a raw material of food:
(A) a cell wall-containing fraction of a gram-positive bacterium.

26. A method of producing food with improved flavor, the method comprising
a step of adding the following ingredient (A) to a raw material of food:
(A) a cell wall-containing fraction of a gram-positive bacterium.

27. The method according to claim 25 or 26, wherein the improvement of the flavor is enhancement of spiciness and/or impartment of a rich taste.

28. The method according to any one of claims 25 to 27, wherein the improvement of the flavor is enhancement of pungency of a spice.

29. The method according to any one of claims 25 to 28, wherein the ingredient (A) is a cell of the gram-positive bacterium, or a fragment thereof.

30. The method according to any one of claims 25 to 29, wherein the gram-positive bacterium is a bacterium belonging to the *Actinobacteria* division or the *Firmicutes* division.

31. The method according to any one of claims 25 to 30, wherein the gram-positive bacterium is a bacterium belonging to the *Actinobacteria* division.

32. The method according to any one of claims 25 to 31, wherein the gram-positive bacterium is selected from the group consisting of a coryneform bacterium, a bacterium belonging to the *Bifidobacteriaceae* family, a bacterium belonging to the *Dermabacteraceae* family, a bacterium belonging to the *Bacillaceae* family, a bacterium belonging to the *Enterococcaceae* family, or a bacterium belonging to the *Lactobacillaceae* family.

33. The method according to any one of claims 25 to 32, wherein the gram-positive bacterium is selected from the group consisting of a *Corynebacterium* genus bacterium, a *Brevibacterium* genus bacterium, a *Bifidobacterium* genus bacterium, a *Brachybacterium* genus bacterium, a *Bacillus* genus bacterium, an *Enterococcus* genus bacterium, or a *Lactobacillus* genus bacterium.

34. The method according to any one of claims 25 to 33, wherein the gram-positive bacterium is selected from the group consisting of *Corynebacterium casei, Corynebacterium flavescens, Brevibacterium casei, Bifidobacterium longum, Brachybacterium alimentarium, Bacillus subtilis, Enterococcus faecalis*, *Lactobacillus mali, Lactobacillus hilgardii,* or *Lactobacillus Brevis.*

35. The method according to any one of claims 25 to 34, wherein the ingredient (A) is added so that the concentration thereof at the time of eating, converted into the dry mass of a cell of the gram-positive bacterium, is 0.005 to 2% (w/w).

36. The method according to any one of claims 25 to 35, further comprising a step of adding the following ingredient (B) to a raw material of food:
(B) one or more ingredients selected from the group consisting of L-amino acid, nucleic acid, and organic acid.

37. The method according to claim 36, wherein at least the L-amino acid is added, and the L-amino acid is L-glutamic acid.

38. The method according to claim 37, wherein L-glutamic acid is added so that the concentration thereof at the time of eating is 0.01 to 2% (w/w).

39. The method according to claim 37 or 38, wherein the method is performed so that the amount of L-glutamic acid contained in the food is 0.1 to 20 parts by weight based on 1 part by weight of the ingredient (A) converted into the dry mass of a cell of the gram-positive bacterium.

40. The method according to any one of claims 25 to 39, wherein the food is spice-containing food.

41. The method according to claim 40, wherein the amount of the spice contained in the food, as a concentration at the time of eating, is 0.01 to 2% (w/w).

42. The method according to claim 40 or 41, wherein the method is performed so that the amount of the spice contained in the food is 0.2 to 500 parts by weight based on 1 part by weight of the ingredient (A) converted into the dry mass of a cell of the gram-positive bacterium.

43. The method according to any one of claims 40 to 42, wherein the spice is one or more spices selected from the group consisting of spices of the *Lauraceae* family, spices of the *Piperaceae* family, spices of the *Lamiaceae* family, spices of the *Apiaceae* family, spices of the *Solanaceae* family, spices of the *Myristicaceae* family, spices of the *Alliaceae* family, spices of the *Myrtaceae* family, spices of the *Schisandraceae* family, spices of the *Fabaceae* family, spices of the *Polygonaceae* family, spices of the *Brassicaceae* family, spices of the *Zingiberaceae* family, and spices of the *Rutaceae* family.

44. The method according to any one of claims 40 to 43, wherein the spice is a spice having pungency.

45. The method according to any one claims 25 to 44, wherein the ingredient (A) is produced by culturing the gram-positive bacterium in a medium containing a raw material of food.

46. The method according to claim 45, wherein the raw material contained in the medium is a tomato.

47. The method according to any one of claims 25 to 46, wherein the ingredient (A) is heat-treated.

48. The method according to any one of claims 25 to 47, wherein the gram-positive bacterium is a bacterium which has an L-glutamic acid-producing ability and which has one or more mutations selected from mutations shown in Table 1.
**Table 1**
| No. | **Position on genome** | **Base before mutation** | **Base after mutation** |
|---|---|---|---|
| A-1 | 78,486 | C | T |
| A-2 | 83,592 | G | A |
| A-3 | 87,955 | C | T |
| A-4 | 90,041 | C | T |
| A-5 | 186,221 | C | T |
| A-6 | 193,010 | C | T |
| A-7 | 196,531 | C | T |
| A-8 | 225,429 | C | T |
| A-9 | 297,920 | G | A |
| A-10 | 320,354 | C | T |
| A-11 | 335,878 | C | T |
| A-12 | 341,763 | C | T |
| A-13 | 346,969 | C | T |
| A-14 | 349,856 | C | T |
| A-15 | 356,232 | C | T |
| A-16 | 357,008 | C | T |
| A-17 | 366,674 | G | A |
| A-18 | 369,871 | G | A |
| A-19 | 377,420 | G | A |
| A-20 | 378,652 | G | A |
| A-21 | 432,252 | C | A |
| A-22 | 439,021 | G | A |
| A-23 | 440,764 | G | A |
| A-24 | 454,682 | G | A |
| A-25 | 458,729 | G | A |
| A-26 | 470,562 | G | A |
| A-27 | 471,288 | G | A |
| A-28 | 472,023 | G | A |
| A-29 | 504,885 | G | A |
| A-30 | 505,785 | G | A |
| | | | |
| A-31 | 514,371 | G | A |
| A-32 | 518,684 | G | A |
| A-33 | 521,126 | G | A |
| A-34 | 524,551 | G | A |
| A-35 | 660,841 | C | T |
| A-36 | 732,121 | C | T |
| A-37 | 787,055 | C | T |
| A-38 | 806,047 | C | T |
| A-39 | 872,482 | G | A |
| A-40 | 878,069 | C | T |
| A-41 | 903,037 | C | T |
| A-42 | 922,802 | C | T |
| A-43 | 948,145 | C | T |
| A-44 | 955,819 | C | T |
| A-45 | 968,915 | C | T |
| A-45 | 973,013 | C | T |
| A-47 | 974,797 | C | T |
| A-48 | 994,815 | C | T |
| A-49 | 1,000,498 | C | T |
| A-50 | 1,019,704 | C | T |
| A-51 | 1,049,052 | C | T |
| A-52 | 1,069,322 | C | T |
| A-53 | 1,070,554 | C | T |
| A-54 | 1,131,016 | C | T |
| A-55 | 1,138,639 | C | T |
| A-56 | 1,162,588 | C | T |
| A-57 | 1,193,273 | C | T |
| A-58 | 1,203,146 | C | T |
| A-59 | 1,222,633 | C | T |
| A-60 | 1,226,969 | G | A |
| A-61 | 1,264,895 | G | A |
| A-62 | 1,268,790 | G | A |
| A-63 | 1,279,676 | G | A |
| A-64 | 1,363,909 | T | C |
| A-65 | 1,387,476 | G | A |
| A-66 | 1,401,171 | G | A |
| A-67 | 1,416,228 | C | T |
| A-68 | 1,420,034 | C | T |
| A-69 | 1,447,494 | C | T |
| A-70 | 1,448,318 | C | T |
| A-71 | 1,448,776 | C | T |
| A-72 | 1,451,922 | C | T |
| A-73 | 1,466,961 | C | T |
| A-74 | 1,503,736 | C | T |
| A-75 | 1,504,207 | C | T |
| A-76 | 1,505,998 | C | T |
| A-77 | 1,507,027 | C | T |
| A-78 | 1,544,310 | C | T |
| A-79 | 1,554,973 | C | T |
| A-80 | 1,558,509 | C | T |
| A-81 | 1,552,459 | C | T |
| A-82 | 1,572,716 | C | T |
| A-83 | 1,594,314 | C | T |
| A-84 | 1,602,545 | C | T |
| A-85 | 1,659,808 | C | T |
| A-86 | 1,682,132 | C | T |
| A-87 | 1,689,863 | C | T |
| A-88 | 1,744,963 | C | T |
| A-89 | 1,784,642 | C | T |
| A-90 | 1,814,866 | C | T |
| A-91 | 1,829,145 | C | T |
| A-92 | 1,852,511 | G | A |
| A-93 | 1,861,170 | G | A |
| A-94 | 1,902,133 | G | A |
| A-95 | 1,916,048 | C | T |
| A-96 | 1,917,434 | C | T |
| A-97 | 1,938,271 | C | T |
| A-98 | 1,949,357 | G | T |
| A-99 | 1,954,368 | C | T |
| A-100 | 1,967,997 | C | T |
| A-101 | 1,975,599 | C | T |
| A-102 | 2,141,466 | C | T |
| A-103 | 2,308,064 | C | T |
| A-104 | 2,310,428 | C | T |
| A-105 | 2,354,420 | C | T |
| A-106 | 2,449,270 | T | C |
| A-107 | 2,449,278 | C | A |
| A-108 | 2,449,291 | G | C |
| A-109 | 2,449,318 | G | A |
| A-110 | 2,496,945 | C | T |
| A-111 | 2,505,022 | C | T |
| A-112 | 2,505,285 | C | T |
| A-113 | 2,525,513 | G | A |
| A-114 | 2,565,856 | C | T |
| A-115 | 2,601,306 | G | A |
| A-116 | 2,615,688 | G | A |
| A-117 | 2,650,740 | G | A |
| A-118 | 2,653,259 | G | A |
| A-119 | 2,663,827 | G | A |
| A-120 | 2,667,322 | G | A |
| A-121 | 2,674,077 | G | A |
| A-122 | 2,679,915 | G | A |
| A-123 | 2,686,979 | G | A |
| A-124 | 2,693,950 | C | T |
| A-125 | 2,696,737 | C | T |
| A-126 | 2,706,442 | C | T |
| A-127 | 2,709,469 | C | T |
| A-128 | 2,711,214 | C | T |
| A-129 | 2,714,651 | C | T |
| A-130 | 2,721,339 | G | A |
| A-131 | 2,731,030 | G | A |
| A-132 | 2,746,202 | G | A |
| A-133 | 2,805,389 | C | T |
| A-134 | 2,816,733 | G | A |
| A-135 | 2,827,114 | G | A |
| B-1 | 29,724 | G | A |
| B-2 | 92,869 | G | A |
| B-3 | 116,733 | G | A |
| B-4 | 131,184 | G | A |
| B-5 | 156,247 | G | A |
| B-6 | 177,083 | G | A |
| B-7 | 184,379 | G | A |
| B-8 | 212,586 | G | A |
| B-9 | 282,162 | G | A |
| 8-10 | 309,483 | G | A |
| B-11 | 376,164 | C | T |
| B-12 | 440,885 | C | T |
| B-13 | 479,120 | G | A |
| B-14 | 722,430 | G | A |
| B-15 | 745,504 | G | A |
| B-16 | 809,993 | G | A |
| B-17 | 859,643 | G | A |
| B-18 | 923,209 | G | A |
| B-19 | 924,973 | G | A |
| B-20 | 998,893 | C | T |
| B-21 | 1,062,144 | C | T |
| B-22 | 1,095,062 | C | T |
| B-23 | 1,102,484 | C | T |
| B-24 | 1,103,812 | C | T |
| B-25 | 1,105,749 | C | T |
| B-26 | 1,107,561 | C | T |
| B-27 | 1,205,722 | C | T |
| B-28 | 1,233,449 | C | T |
| B-29 | 1,242,484 | C | T |
| B-30 | 1,248,388 | C | T |
| B-31 | 1,249,270 | C | T |
| B-32 | 1,291,377 | C | T |
| B-33 | 1,308,597 | C | T |
| B-34 | 1,329,535 | C | T |
| B-35 | 1,367,486 | C | T |
| 8-36 | 1,382,065 | C | T |
| B-37 | 1,403,043 | C | T |
| B-38 | 1,433,914 | C | T |
| B-39 | 1,442,447 | C | T |
| B-40 | 1,501,903 | G | A |
| B-41 | 1,504,744 | C | T |
| B-42 | 1,651,403 | G | A |
| B-43 | 1,695,473 | G | A |
| B-44 | 1,779,939 | G | A |
| B-45 | 1,797,452 | G | A |
| B-46 | 1,801,284 | G | A |
| B-47 | 1,816,679 | G | A |
| B-48 | 1,832,252 | G | A |
| B-49 | 1,843,841 | G | A |
| B-50 | 1,868,285 | G | A |
| B-51 | 1,879,922 | G | A |
| B-52 | 1,892,007 | G | A |
| B-53 | 1,916,016 | G | A |
| B-54 | 1,937,604 | G | A |
| B-55 | 1,947,044 | G | A |
| B-56 | 1,948,411 | G | A |
| B-57 | 1,948,649 | G | A |
| B-58 | 1,967,697 | G | A |
| B-59 | 1,974,137 | G | A |
| B-60 | 2,028,284 | C | T |
| B-61 | 2,050,998 | G | A |
| B-62 | 2,052,708 | G | A |
| B-63 | 2,054,372 | G | A |
| B-64 | 2,065,568 | G | A |
| B-65 | 2,067,167 | G | A |
| B-66 | 2,082,577 | G | A |
| B-67 | 2,121,006 | A | G |
| B-68 | 2,149,369 | C | T |
| B-69 | 2,159,680 | G | A |
| B-70 | 2,380,965 | G | A |
| B-71 | 2,477,728 | G | A |
| B-72 | 2,542,800 | G | A |
| B-73 | 2,570,107 | G | A |
| B-74 | 2,647,383 | G | A |
| B-75 | 2,726,248 | C | T |
| B-76 | 2,825,055 | C | T |
| B-77 | 2,837,078 | C | T |
| B-78 | 2,865,322 | C | T |
| B-79 | 2,872,907 | C | T |
| B-80 | 2,880,351 | C | T |
| B-81 | 2,889,394 | C | T |
| B-82 | 2,906,471 | C | T |
| B-83 | 2,927,044 | C | T |
| B-84 | 2,929,963 | C | T |
| B-85 | 2,940,673 | C | T |
| B-86 | 2,946,285 | C | T |
| B-87 | 2,962,909 | C | T |
| B-88 | 2,975,742 | C | T |
| B-89 | 2,987,052 | C | T |
| B-90 | 3,079,560 | C | T |
| B-91 | 3,083,927 | C | T |
| B-92 | 3,090,163 | C | T |

49. The method according to any one of claims 25 to 48, further comprising a step of adding a culture product of the gram-positive bacterium, to a raw material of food.

50. A seasoning comprising the following ingredient (A):
(A) a cell wall-containing fraction of a gram-positive bacterium.

51. The seasoning according to claim 50, wherein the ingredient (A) is a cell of the gram-positive bacterium, or a fragment thereof.

52. The seasoning according to claim 50 or 51, the seasoning further comprising the following ingredient (B):
(B) one or more ingredients selected from the group consisting of L-amino acid, nucleic acid, and organic acid.

53. The seasoning according to claim 52, further containing at least the L-amino acid, wherein the L-amino acid is L-glutamic acid.

54. The seasoning according to any one of claims 50 to 52, further comprising a spice.

55. The seasoning according to any one of claims 50 to 54, wherein the ingredient (A) is produced by culturing the gram-positive bacterium in a medium containing a raw material of food.

56. The seasoning according to claim 55, wherein the raw material contained in the medium is a tomato.

57. The seasoning according to any one of claims 50 to 56, wherein the ingredient (A) is heat-treated.

58. The seasoning according to any one of claims 50 to 57, wherein the gram-positive bacterium is a bacterium which has an L-glutamic acid-producing ability and which has one or more mutations selected from mutations shown in Table 1.
**Table 1**
| No. | **Position on genome** | **Base before mutation** | **Base after mutation** |
|---|---|---|---|
| | | | |
| A-1 | 78,486 | C | T |
| A-2 | 83,592 | G | A |
| A-3 | 87,955 | C | T |
| A-4 | 90,041 | C | T |
| A-5 | 186,221 | C | T |
| A-6 | 193,010 | C | T |
| A-7 | 196,531 | C | T |
| A-8 | 225,429 | C | T |
| A-9 | 297,920 | G | A |
| A-10 | 320,354 | C | T |
| A-11 | 335,878 | C | T |
| A-12 | 341,763 | C | T |
| A-13 | 346,969 | C | T |
| A-14 | 349,856 | C | T |
| A-15 | 356,232 | C | T |
| A-16 | 357,008 | C | T |
| A-17 | 366,674 | G | A |
| A-18 | 369,871 | G | A |
| A-19 | 377,420 | G | A |
| A-20 | 378,652 | G | A |
| A-21 | 432,252 | C | A |
| A-22 | 439,021 | G | A |
| A-23 | 440,764 | G | A |
| A-24 | 454,682 | G | A |
| A-25 | 458,729 | G | A |
| A-26 | 470,562 | G | A |
| A-27 | 471,288 | G | A |
| A-28 | 472,023 | G | A |
| A-29 | 504,885 | G | A |
| A-30 | 505,785 | G | A |
| | | | |
| A-31 | 514,371 | G | A |
| A-32 | 518,684 | G | A |
| A-33 | 521,126 | G | A |
| A-34 | 524,551 | G | A |
| A-35 | 660,841 | C | T |
| A-36 | 732,121 | C | T |
| A-37 | 787,055 | C | T |
| A-38 | 806,047 | C | T |
| A-39 | 872,482 | G | A |
| A-40 | 878,069 | C | T |
| A-41 | 903,037 | C | T |
| A-42 | 922,802 | C | T |
| A-43 | 948,145 | C | T |
| A-44 | 955,819 | C | T |
| A-45 | 968,915 | C | T |
| A-45 | 973,013 | C | T |
| A-47 | 974,797 | C | T |
| A-48 | 994,815 | C | T |
| A-49 | 1,000,498 | C | T |
| A-50 | 1,019,704 | C | T |
| A-51 | 1,049,052 | C | T |
| A-52 | 1,069,322 | C | T |
| A-53 | 1,070,554 | C | T |
| A-54 | 1,131,016 | C | T |
| A-55 | 1,138,639 | C | T |
| A-56 | 1,162,588 | C | T |
| A-57 | 1,193,273 | C | T |
| A-58 | 1,203,146 | C | T |
| A-59 | 1,222,633 | C | T |
| A-60 | 1,226,969 | G | A |
| A-61 | 1,264,895 | G | A |
| A-62 | 1,268,790 | G | A |
| A-63 | 1,279,676 | G | A |
| A-64 | 1,363,909 | T | C |
| A-65 | 1,387,476 | G | A |
| A-66 | 1,401,171 | G | A |
| A-67 | 1,416,228 | C | T |
| A-68 | 1,420,034 | C | T |
| A-69 | 1,447,494 | C | T |
| A-70 | 1,448,318 | C | T |
| A-71 | 1,448,776 | C | T |
| A-72 | 1,451,922 | C | T |
| A-73 | 1,466,961 | C | T |
| A-74 | 1,503,736 | C | T |
| A-75 | 1,504,207 | C | T |
| A-76 | 1,505,998 | C | T |
| A-77 | 1,507,027 | C | T |
| A-78 | 1,544,310 | C | T |
| A-79 | 1,554,973 | C | T |
| A-80 | 1,558,509 | C | T |
| A-81 | 1,562,459 | C | T |
| A-82 | 1,572,716 | C | T |
| A-83 | 1,594,314 | C | T |
| A-84 | 1,602,545 | C | T |
| A-85 | 1,659,808 | C | T |
| A-86 | 1,682,132 | C | T |
| A-87 | 1,689,863 | C | T |
| A-88 | 1,744,963 | C | T |
| A-89 | 1,784,642 | C | T |
| A-90 | 1,814,866 | C | T |
| A-91 | 1,829,145 | C | T |
| A-92 | 1,852,511 | G | A |
| A-93 | 1,861,170 | G | A |
| A-94 | 1,902,133 | G | A |
| A-95 | 1,916,048 | C | T |
| A-96 | 1,917,434 | C | T |
| A-97 | 1,938,271 | C | T |
| A-98 | 1,949,357 | G | T |
| A-99 | 1,954,368 | C | T |
| A-100 | 1,967,997 | C | T |
| A-101 | 1,975,599 | C | T |
| A-102 | 2,141,466 | C | T |
| A-103 | 2,308,064 | C | T |
| A-104 | 2,310,428 | C | T |
| A-105 | 2,354,420 | C | T |
| A-106 | 2,449,270 | T | C |
| A-107 | 2,449,278 | C | A |
| A-108 | 2,449,291 | G | C |
| A-109 | 2,449,318 | G | A |
| A-110 | 2,496,945 | C | T |
| A-111 | 2,505,022 | C | T |
| A-112 | 2,505,285 | C | T |
| A-113 | 2,525,513 | G | A |
| A-114 | 2,565,856 | C | T |
| A-115 | 2,601,306 | G | A |
| A-116 | 2,615,688 | G | A |
| A-117 | 2,650,740 | G | A |
| A-118 | 2,653,259 | G | A |
| A-119 | 2,663,827 | G | A |
| A-120 | 2,667,322 | G | A |
| | | | |
| A-121 | 2,674,077 | G | A |
| A-122 | 2,679,915 | G | A |
| A-123 | 2,686,979 | G | A |
| A-124 | 2,693,950 | C | T |
| A-125 | 2,696,737 | C | T |
| A-126 | 2,706,442 | C | T |
| A-127 | 2,709,469 | C | T |
| A-128 | 2,711,214 | C | T |
| A-129 | 2,714,651 | C | T |
| A-130 | 2,721,339 | G | A |
| A-131 | 2,731,030 | G | A |
| A-132 | 2,746,202 | G | A |
| A-133 | 2,805,389 | C | T |
| A-134 | 2,816,733 | G | A |
| A-135 | 2,827,114 | G | A |
| B-1 | 29,724 | G | A |
| B-2 | 92,869 | G | A |
| B-3 | 116,733 | G | A |
| B-4 | 131,184 | G | A |
| B-5 | 156,247 | G | A |
| B-6 | 177,083 | G | A |
| B-7 | 184,379 | G | A |
| B-8 | 212,586 | G | A |
| B-9 | 282,162 | G | A |
| B-10 | 309,483 | G | A |
| B-11 | 376,164 | C | T |
| B-12 | 440,885 | C | T |
| B-13 | 479,120 | G | A |
| B-14 | 722,430 | G | A |
| B-15 | 745,504 | G | A |
| 8-16 | 809,993 | G | A |
| B-17 | 859,643 | G | A |
| B-18 | 923,209 | G | A |
| B-19 | 924,973 | G | A |
| 8-20 | 998,893 | C | T |
| B-21 | 1,062,144 | C | T |
| B-22 | 1,095,062 | C | T |
| 8-23 | 1,102,484 | C | T |
| B-24 | 1,103,812 | C | T |
| B-25 | 1,105,749 | C | T |
| B-26 | 1,107,561 | C | T |
| B-27 | 1,205,722 | C | T |
| B-28 | 1,233,449 | C | T |
| B-29 | 1,242,484 | C | T |
| B-30 | 1,248,388 | C | T |
| B-31 | 1,249,270 | C | T |
| B-32 | 1,291,377 | C | T |
| B-33 | 1,308,597 | C | T |
| B-34 | 1,329,535 | C | T |
| B-35 | 1,367,486 | C | T |
| B-36 | 1,382,065 | C | T |
| B-37 | 1,403,043 | C | T |
| B-38 | 1,433,914 | C | T |
| B-39 | 1,442,447 | C | T |
| B-40 | 1,501,903 | G | A |
| B-41 | 1,504,744 | C | T |
| B-42 | 1,651,403 | G | A |
| B-43 | 1,695,473 | G | A |
| B-44 | 1,779,939 | G | A |
| B-45 | 1,797,452 | G | A |
| B-46 | 1,801,284 | G | A |
| B-47 | 1,816,679 | G | A |
| B-48 | 1,832,252 | G | A |
| B-49 | 1,843,841 | G | A |
| B-50 | 1,868,285 | G | A |
| B-51 | 1,879,922 | G | A |
| B-52 | 1,892,007 | G | A |
| B-53 | 1,916,016 | G | A |
| B-54 | 1,937,604 | G | A |
| B-55 | 1,947,044 | G | A |
| B-56 | 1,948,411 | G | A |
| 8-57 | 1,948,649 | G | A |
| B-58 | 1,967,697 | G | A |
| B-59 | 1,974,137 | G | A |
| B-60 | 2,028,284 | C | T |
| B-61 | 2,050,998 | G | A |
| B-62 | 2,052,708 | G | A |
| B-63 | 2,054,372 | G | A |
| B-64 | 2,065,568 | G | A |
| B-65 | 2,067,167 | G | A |
| B-66 | 2,082,577 | G | A |
| B-67 | 2,121,006 | A | G |
| B-68 | 2,149,369 | C | T |
| B-69 | 2,159,680 | G | A |
| B-70 | 2,380,965 | G | A |
| B-71 | 2,477,728 | G | A |
| B-72 | 2,542,800 | G | A |
| B-73 | 2,570,107 | G | A |
| B-74 | 2,647,383 | G | A |
| B-75 | 2,726,248 | C | T |
| 8-76 | 2,825,055 | C | T |
| B-77 | 2,837,078 | C | T |
| B-78 | 2,865,322 | C | T |
| B-79 | 2,872,907 | C | T |
| B-80 | 2,880,351 | C | T |
| B-81 | 2,889,394 | C | T |
| B-82 | 2,906,471 | C | T |
| B-83 | 2,927,044 | C | T |
| B-84 | 2,929,963 | C | T |
| B-85 | 2,940,673 | C | T |
| B-86 | 2,946,285 | C | T |
| B-87 | 2,962,909 | C | T |
| B-88 | 2,975,742 | C | T |
| B-89 | 2,987,052 | C | T |
| B-90 | 3,079,560 | C | T |
| B-91 | 3,083,927 | C | T |
| B-92 | 3,090,163 | C | T |

59. The seasoning according to any one of claims 50 to 58, further comprising a culture product of the gram-positive bacterium.

60. A bacterium having an L-glutamic acid-producing ability, the bacterium having:
one or more mutations selected from mutations A-1 to A-135 shown in Table 1.
**Table 1**
| No. | **Position on genome** | **Base before mutation** | **Base after mutation** |
|---|---|---|---|
| A-1 | 78,486 | C | T |
| A-2 | 83,592 | G | A |
| A-3 | 87,955 | C | T |
| A-4 | 90,041 | C | T |
| A-5 | 186,221 | C | T |
| A-6 | 193,010 | C | T |
| A-7 | 196,531 | C | T |
| A-8 | 225,429 | C | T |
| A-9 | 297,920 | G | A |
| A-10 | 320,354 | C | T |
| A-11 | 335,878 | C | T |
| A-12 | 341,763 | C | T |
| A-13 | 346,969 | C | T |
| A-14 | 349,856 | C | T |
| A-15 | 356,232 | C | T |
| A-16 | 357,008 | C | T |
| A-17 | 366,674 | G | A |
| A-18 | 369,871 | G | A |
| A-19 | 377,420 | G | A |
| A-20 | 378,652 | G | A |
| A-21 | 432,252 | C | A |
| A-22 | 439,021 | G | A |
| A-23 | 440,764 | G | A |
| A-24 | 454,682 | G | A |
| A-25 | 458,729 | G | A |
| A-26 | 470,562 | G | A |
| A-27 | 471,288 | G | A |
| A-28 | 472,023 | G | A |
| A-29 | 504,885 | G | A |
| A-30 | 505,785 | G | A |
| A-31 | 514,371 | G | A |
| A-32 | 518,684 | G | A |
| A-33 | 521,126 | G | A |
| A-34 | 524,551 | G | A |
| A-35 | 660,841 | C | T |
| A-36 | 732,121 | C | T |
| A-37 | 787,055 | C | T |
| A-38 | 806,047 | C | T |
| A-39 | 872,482 | G | A |
| A-40 | 878,069 | C | T |
| A-41 | 903,037 | C | T |
| A-42 | 922,802 | C | T |
| A-43 | 948,145 | C | T |
| A-44 | 955,819 | C | T |
| A-45 | 968,915 | C | T |
| A-45 | 973,013 | C | T |
| A-47 | 974,797 | C | T |
| A-48 | 994,815 | C | T |
| A-49 | 1,000,498 | C | T |
| A-50 | 1,019,704 | C | T |
| A-51 | 1,049,052 | C | T |
| A-52 | 1,069,322 | C | T |
| A-53 | 1,070,554 | C | T |
| A-54 | 1,131,016 | C | T |
| A-55 | 1,138,639 | C | T |
| A-56 | 1,162,588 | C | T |
| A-57 | 1,193,273 | C | T |
| A-58 | 1,203,146 | C | T |
| A-59 | 1,222,633 | C | T |
| A-60 | 1,226,969 | G | A |
| A-61 | 1,264,895 | G | A |
| A-62 | 1,268,790 | G | A |
| A-63 | 1,279,676 | G | A |
| A-64 | 1,363,909 | T | C |
| A-65 | 1,387,476 | G | A |
| A-66 | 1,401,171 | G | A |
| A-67 | 1,416,228 | C | T |
| A-68 | 1,420,034 | C | T |
| A-69 | 1,447,494 | C | T |
| A-70 | 1,448,318 | C | T |
| A-71 | 1,448,776 | C | T |
| A-72 | 1,451,922 | C | T |
| A-73 | 1,466,961 | C | T |
| A-74 | 1,503,736 | C | T |
| A-75 | 1,504,207 | C | T |
| A-76 | 1,505,998 | C | T |
| A-77 | 1,507,027 | C | T |
| A-78 | 1,544,310 | C | T |
| A-79 | 1,554,973 | C | T |
| A-80 | 1,558,509 | C | T |
| A-81 | 1,562,459 | C | T |
| A-82 | 1,572,716 | C | T |
| A-83 | 1,594,314 | C | T |
| A-84 | 1,602,545 | C | T |
| A-85 | 1,659,808 | C | T |
| A-86 | 1,682,132 | C | T |
| A-87 | 1,689,863 | C | T |
| A-88 | 1,744,963 | C | T |
| A-89 | 1,784,642 | C | T |
| A-90 | 1,814,866 | C | T |
| A-91 | 1,829,145 | C | T |
| A-92 | 1,852,511 | G | A |
| A-93 | 1,861,170 | G | A |
| A-94 | 1,902,133 | G | A |
| A-95 | 1,916,048 | C | T |
| A-96 | 1,917,434 | C | T |
| A-97 | 1,938,271 | C | T |
| A-98 | 1,949,357 | G | T |
| A-99 | 1,954,368 | C | T |
| A-100 | 1,967,997 | C | T |
| A-101 | 1,975,599 | C | T |
| A-102 | 2,141,466 | C | T |
| A-103 | 2,308,064 | C | T |
| A-104 | 2,310,428 | C | T |
| A-105 | 2,354,420 | C | T |
| A-106 | 2,449,270 | T | C |
| A-107 | 2,449,278 | C | A |
| A-108 | 2,449,291 | G | C |
| A-109 | 2,449,318 | G | A |
| A-110 | 2,496,945 | C | T |
| A-111 | 2,505,022 | C | T |
| A-112 | 2,505,285 | C | T |
| A-113 | 2,525,513 | G | A |
| A-114 | 2,565,856 | C | T |
| A-115 | 2,601,306 | G | A |
| A-116 | 2,615,688 | G | A |
| A-117 | 2,650,740 | G | A |
| A-118 | 2,653,259 | G | A |
| A-119 | 2,663,827 | G | A |
| A-120 | 2,667,322 | G | A |
| A-121 | 2,674,077 | G | A |
| A-122 | 2,679,915 | G | A |
| A-123 | 2,686,979 | G | A |
| A-124 | 2,693,950 | C | T |
| A-125 | 2,696,737 | C | T |
| A-126 | 2,706,442 | C | T |
| A-127 | 2,709,469 | C | T |
| A-128 | 2,711,214 | C | T |
| A-129 | 2,714,651 | C | T |
| A-130 | 2,721,339 | G | A |
| A-131 | 2,731,030 | G | A |
| A-132 | 2,746,202 | G | A |
| A-133 | 2,805,389 | C | T |
| A-134 | 2,816,733 | G | A |
| A-135 | 2,827,114 | G | A |
| B-1 | 29,724 | G | A |
| B-2 | 92,869 | G | A |
| B-3 | 116,733 | G | A |
| B-4 | 131,184 | G | A |
| B-5 | 156,247 | G | A |
| B-6 | 177,083 | G | A |
| B-7 | 184,379 | G | A |
| B-8 | 212,586 | G | A |
| B-9 | 282,162 | G | A |
| B-10 | 309,483 | G | A |
| B-11 | 376,164 | C | T |
| B-12 | 440,885 | C | T |
| B-13 | 479,120 | G | A |
| B-14 | 722,430 | G | A |
| B-15 | 745,504 | G | A |
| 8-16 | 809,993 | G | A |
| B-17 | 859,643 | G | A |
| B-18 | 923,209 | G | A |
| B-19 | 924,973 | G | A |
| 8-20 | 998,893 | C | T |
| B-21 | 1,062,144 | C | T |
| B-22 | 1,095,062 | C | T |
| 8-23 | 1,102,484 | C | T |
| B-24 | 1,103,812 | C | T |
| B-25 | 1,105,749 | C | T |
| B-26 | 1,107,561 | C | T |
| B-27 | 1,205,722 | C | T |
| B-28 | 1,233,449 | C | T |
| B-29 | 1,242,484 | C | T |
| B-30 | 1,248,388 | C | T |
| B-31 | 1,249,270 | C | T |
| B-32 | 1,291,377 | C | T |
| B-33 | 1,308,597 | C | T |
| B-34 | 1,329,535 | C | T |
| B-35 | 1,367,486 | C | T |
| B-36 | 1,382,065 | C | T |
| B-37 | 1,403,043 | C | T |
| B-38 | 1,433,914 | C | T |
| B-39 | 1,442,447 | C | T |
| B-40 | 1,501,903 | G | A |
| B-41 | 1,504,744 | C | T |
| B-42 | 1,651,403 | G | A |
| B-43 | 1,695,473 | G | A |
| B-44 | 1,779,939 | G | A |
| B-45 | 1,797,452 | G | A |
| B-46 | 1,801,284 | G | A |
| B-47 | 1,816,679 | G | A |
| B-48 | 1,832,252 | G | A |
| B-49 | 1,843,841 | G | A |
| B-50 | 1,868,285 | G | A |
| B-51 | 1,879,922 | G | A |
| B-52 | 1,892,007 | G | A |
| B-53 | 1,916,016 | G | A |
| B-54 | 1,937,604 | G | A |
| B-55 | 1,947,044 | G | A |
| B-56 | 1,948,411 | G | A |
| 8-57 | 1,948,649 | G | A |
| B-58 | 1,967,697 | G | A |
| B-59 | 1,974,137 | G | A |
| B-60 | 2,028,284 | C | T |
| B-61 | 2,050,998 | G | A |
| B-62 | 2,052,708 | G | A |
| B-63 | 2,054,372 | G | A |
| B-64 | 2,065,568 | G | A |
| B-65 | 2,067,167 | G | A |
| B-66 | 2,082,577 | G | A |
| B-67 | 2,121,006 | A | G |
| B-68 | 2,149,369 | C | T |
| B-69 | 2,159,680 | G | A |
| B-70 | 2,380,965 | G | A |
| B-71 | 2,477,728 | G | A |
| B-72 | 2,542,800 | G | A |
| B-73 | 2,570,107 | G | A |
| B-74 | 2,647,383 | G | A |
| B-75 | 2,726,248 | C | T |
| 8-76 | 2,825,055 | C | T |
| B-77 | 2,837,078 | C | T |
| B-78 | 2,865,322 | C | T |
| B-79 | 2,872,907 | C | T |
| B-80 | 2,880,351 | C | T |
| B-81 | 2,889,394 | C | T |
| B-82 | 2,906,471 | C | T |
| B-83 | 2,927,044 | C | T |
| B-84 | 2,929,963 | C | T |
| B-85 | 2,940,673 | C | T |
| B-86 | 2,946,285 | C | T |
| B-87 | 2,962,909 | C | T |
| B-88 | 2,975,742 | C | T |
| B-89 | 2,987,052 | C | T |
| B-90 | 3,079,560 | C | T |
| B-91 | 3,083,927 | C | T |
| B-92 | 3,090,163 | C | T |

61. The bacterium according to claim 60, having 50 or more mutations selected from mutations A-1 to A-135 shown in Table 1.

62. The bacterium according to claim 60 or 61, having 100 or more mutations selected from mutations A-1 to A-135 shown in Table 1.

63. The bacterium according to any one of claims 60 to 62, having mutations A-1 to A-135 shown in Table 1.

64. The bacterium according to any one of claims 60 to 63, further having one or more mutations selected from mutations B-1 to B-92 shown in Table 1.

65. The bacterium according to claim 64, having 30 or more mutations selected from mutations B-1 to B-92 shown in Table 1.

66. The bacterium according to claim 64 or 65, having 60 or more mutations selected from mutations B-1 to B-92 shown in Table 1.

67. The bacterium according to any one of claims 60 to 66, wherein the bacterium is a coryneform bacterium.

68. The bacterium according to any one of claims 60 to 67, wherein the bacterium is a *Corynebacterium* genus bacterium.

69. The bacterium according to any one of claims 60 to 68, wherein the bacterium is *Corynebacterium casei.*

70. The bacterium according to any one of claims 60 to 69, wherein the bacterium is a *Corynebacterium casei* JCM 12072 strain-derived modified strain.

71. A method of producing a composition for improving a flavor of food, the method comprising:
a step of culturing a gram-positive bacterium in a medium to provide a culture product,
wherein the composition comprises the following ingredient (A):
(A) a cell wall-containing fraction of a gram-positive bacterium.

72. The method according to claim 71, wherein the improvement of the flavor is enhancement of spiciness and/or impartment of a rich taste.

73. The method according to claim 71 or 72, wherein the improvement of the flavor is enhancement of pungency of a spice.

74. The method according to any one of claims 71 to 73, wherein the composition further comprises a spice.

75. The method according to any one of claims 71 to 74, wherein the ingredient (A) is a cell of the gram-positive bacterium, or a fragment thereof.

76. The method according to any one of claims 71 to 75, wherein the composition further comprises the following ingredient (B):
(B) one or more ingredients selected from the group consisting of L-amino acid, nucleic acid, and organic acid.

77. The method according to claim 76, wherein the composition further comprises at least the L-amino acid, and the L-amino acid is L-glutamic acid.

78. The method according to any one of claims 71 to 77, further comprising a step of heat-treating the culture product.

79. The method according to any one of claims 71 to 78, wherein the gram-positive bacterium is a bacterium which has an L-glutamic acid-producing ability and which has one or more mutations selected from mutations shown in Table 1.
**Table 1**
| No. | **Position on genome** | **Base before mutation** | **Base after mutation** |
|---|---|---|---|
| A-1 | 78,486 | C | T |
| A-2 | 83,592 | G | A |
| A-3 | 87,955 | C | T |
| A-4 | 90,041 | C | T |
| A-5 | 186,221 | C | T |
| A-6 | 193,010 | C | T |
| A-7 | 196,531 | C | T |
| A-8 | 225,429 | C | T |
| A-9 | 297,920 | G | A |
| A-10 | 320,354 | C | T |
| A-11 | 335,878 | C | T |
| A-12 | 341,763 | C | T |
| A-13 | 346,969 | C | T |
| A-14 | 349,856 | C | T |
| A-15 | 356,232 | C | T |
| A-16 | 357,008 | C | T |
| A-17 | 366,674 | G | A |
| A-18 | 369,871 | G | A |
| A-19 | 377,420 | G | A |
| A-20 | 378,652 | G | A |
| A-21 | 432,252 | C | A |
| A-22 | 439,021 | G | A |
| A-23 | 440,764 | G | A |
| A-24 | 454,682 | G | A |
| A-25 | 458,729 | G | A |
| A-26 | 470,562 | G | A |
| A-27 | 471,288 | G | A |
| A-28 | 472,023 | G | A |
| A-29 | 504,885 | G | A |
| A-30 | 505,785 | G | A |
| A-31 | 514,371 | G | A |
| A-32 | 518,684 | G | A |
| A-33 | 521,126 | G | A |
| A-34 | 524,551 | G | A |
| A-35 | 660,841 | C | T |
| A-36 | 732,121 | C | T |
| A-37 | 787,055 | C | T |
| A-38 | 806,047 | C | T |
| A-39 | 872,482 | G | A |
| A-40 | 878,069 | C | T |
| A-41 | 903,037 | C | T |
| A-42 | 922,802 | C | T |
| A-43 | 948,145 | C | T |
| A-44 | 955,819 | C | T |
| A-45 | 968,915 | C | T |
| A-45 | 973,013 | C | T |
| A-47 | 974,797 | C | T |
| A-48 | 994,815 | C | T |
| A-49 | 1,000,498 | C | T |
| A-50 | 1,019,704 | C | T |
| A-51 | 1,049,052 | C | T |
| A-52 | 1,069,322 | C | T |
| A-53 | 1,070,554 | C | T |
| A-54 | 1,131,016 | C | T |
| A-55 | 1,138,639 | C | T |
| A-56 | 1,162,588 | C | T |
| A-57 | 1,193,273 | C | T |
| A-58 | 1,203,146 | C | T |
| A-59 | 1,222,633 | C | T |
| A-60 | 1,226,969 | G | A |
| A-61 | 1,264,895 | G | A |
| A-62 | 1,268,790 | G | A |
| A-63 | 1,279,676 | G | A |
| A-64 | 1,363,909 | T | C |
| A-65 | 1,387,476 | G | A |
| A-66 | 1,401,171 | G | A |
| A-67 | 1,416,228 | C | T |
| A-68 | 1,420,034 | C | T |
| A-69 | 1,447,494 | C | T |
| A-70 | 1,448,318 | C | T |
| A-71 | 1,448,776 | C | T |
| A-72 | 1,451,922 | C | T |
| A-73 | 1,466,961 | C | T |
| A-74 | 1,503,736 | C | T |
| A-75 | 1,504,207 | C | T |
| A-76 | 1,505,998 | C | T |
| A-77 | 1,507,027 | C | T |
| A-78 | 1,544,310 | C | T |
| A-79 | 1,554,973 | C | T |
| A-80 | 1,558,509 | C | T |
| A-81 | 1,562,459 | C | T |
| A-82 | 1,572,716 | C | T |
| A-83 | 1,594,314 | C | T |
| A-84 | 1,602,545 | C | T |
| A-85 | 1,659,808 | C | T |
| A-86 | 1,682,132 | C | T |
| A-87 | 1,689,863 | C | T |
| A-88 | 1,744,963 | C | T |
| A-89 | 1,784,642 | C | T |
| A-90 | 1,814,866 | C | T |
| A-91 | 1,829,145 | C | T |
| A-92 | 1,852,511 | G | A |
| A-93 | 1,861,170 | G | A |
| A-94 | 1,902,133 | G | A |
| A-95 | 1,916,048 | C | T |
| A-96 | 1,917,434 | C | T |
| A-97 | 1,938,271 | C | T |
| A-98 | 1,949,357 | G | T |
| A-99 | 1,954,368 | C | T |
| A-100 | 1,967,997 | C | T |
| A-101 | 1,975,599 | C | T |
| A-102 | 2,141,466 | C | T |
| A-103 | 2,308,064 | C | T |
| A-104 | 2,310,428 | C | T |
| A-105 | 2,354,420 | C | T |
| A-106 | 2,449,270 | T | C |
| A-107 | 2,449,278 | C | A |
| A-108 | 2,449,291 | G | C |
| A-109 | 2,449,318 | G | A |
| A-110 | 2,496,945 | C | T |
| A-111 | 2,505,022 | C | T |
| A-112 | 2,505,285 | C | T |
| A-113 | 2,525,513 | G | A |
| A-114 | 2,565,856 | C | T |
| A-115 | 2,601,306 | G | A |
| A-116 | 2,615,688 | G | A |
| A-117 | 2,650,740 | G | A |
| A-118 | 2,653,259 | G | A |
| A-119 | 2,663,827 | G | A |
| A-120 | 2,667,322 | G | A |
| A-121 | 2,674,077 | G | A |
| A-122 | 2,679,915 | G | A |
| A-123 | 2,686,979 | G | A |
| A-124 | 2,693,950 | C | T |
| A-125 | 2,696,737 | C | T |
| A-126 | 2,706,442 | C | T |
| A-127 | 2,709,469 | C | T |
| A-128 | 2,711,214 | C | T |
| A-129 | 2,714,651 | C | T |
| A-130 | 2,721,339 | G | A |
| A-131 | 2,731,030 | G | A |
| A-132 | 2,746,202 | G | A |
| A-133 | 2,805,389 | C | T |
| A-134 | 2,816,733 | G | A |
| A-135 | 2,827,114 | G | A |
| B-1 | 29,724 | G | A |
| B-2 | 92,869 | G | A |
| B-3 | 116,733 | G | A |
| B-4 | 131,184 | G | A |
| B-5 | 156,247 | G | A |
| B-6 | 177,083 | G | A |
| B-7 | 184,379 | G | A |
| B-8 | 212,586 | G | A |
| B-9 | 282,162 | G | A |
| B-10 | 309,483 | G | A |
| B-11 | 376,164 | C | T |
| B-12 | 440,885 | C | T |
| B-13 | 479,120 | G | A |
| B-14 | 722,430 | G | A |
| B-15 | 745,504 | G | A |
| 8-16 | 809,993 | G | A |
| B-17 | 859,643 | G | A |
| B-18 | 923,209 | G | A |
| B-19 | 924,973 | G | A |
| 8-20 | 998,893 | C | T |
| B-21 | 1,062,144 | C | T |
| B-22 | 1,095,062 | C | T |
| 8-23 | 1,102,484 | C | T |
| B-24 | 1,103,812 | C | T |
| B-25 | 1,105,749 | C | T |
| B-26 | 1,107,561 | C | T |
| B-27 | 1,205,722 | C | T |
| B-28 | 1,233,449 | C | T |
| B-29 | 1,242,484 | C | T |
| B-30 | 1,248,388 | C | T |
| B-31 | 1,249,270 | C | T |
| B-32 | 1,291,377 | C | T |
| B-33 | 1,308,597 | C | T |
| B-34 | 1,329,535 | C | T |
| B-35 | 1,367,486 | C | T |
| B-36 | 1,382,065 | C | T |
| B-37 | 1,403,043 | C | T |
| B-38 | 1,433,914 | C | T |
| B-39 | 1,442,447 | C | T |
| B-40 | 1,501,903 | G | A |
| B-41 | 1,504,744 | C | T |
| B-42 | 1,651,403 | G | A |
| B-43 | 1,695,473 | G | A |
| B-44 | 1,779,939 | G | A |
| B-45 | 1,797,452 | G | A |
| 8-46 | 1,801,284 | G | A |
| B-47 | 1,816,679 | G | A |
| B-48 | 1,832,252 | G | A |
| B-49 | 1,843,841 | G | A |
| B-50 | 1,868,285 | G | A |
| B-51 | 1,879,922 | G | A |
| B-52 | 1,892,007 | G | A |
| B-53 | 1,916,016 | G | A |
| B-54 | 1,937,604 | G | A |
| B-55 | 1,947,044 | G | A |
| B-56 | 1,948,411 | G | A |
| 8-57 | 1,948,649 | G | A |
| B-58 | 1,967,697 | G | A |
| B-59 | 1,974,137 | G | A |
| B-60 | 2,028,284 | C | T |
| B-61 | 2,050,998 | G | A |
| B-62 | 2,052,708 | G | A |
| B-63 | 2,054,372 | G | A |
| B-64 | 2,065,568 | G | A |
| B-65 | 2,067,167 | G | A |
| B-66 | 2,082,577 | G | A |
| B-67 | 2,121,006 | A | G |
| B-68 | 2,149,369 | C | T |
| B-69 | 2,159,680 | G | A |
| B-70 | 2,380,965 | G | A |
| B-71 | 2,477,728 | G | A |
| B-72 | 2,542,800 | G | A |
| B-73 | 2,570,107 | G | A |
| B-74 | 2,647,383 | G | A |
| B-75 | 2,726,248 | C | T |
| 8-76 | 2,825,055 | C | T |
| B-77 | 2,837,078 | C | T |
| B-78 | 2,865,322 | C | T |
| B-79 | 2,872,907 | C | T |
| B-80 | 2,880,351 | C | T |
| B-81 | 2,889,394 | C | T |
| B-82 | 2,906,471 | C | T |
| B-83 | 2,927,044 | C | T |
| B-84 | 2,929,963 | C | T |
| B-85 | 2,940,673 | C | T |
| B-86 | 2,946,285 | C | T |
| B-87 | 2,962,909 | C | T |
| B-88 | 2,975,742 | C | T |
| B-89 | 2,987,052 | C | T |
| B-90 | 3,079,560 | C | T |
| B-91 | 3,083,927 | C | T |
| B-92 | 3,090,163 | C | T |

80. The method according to any one of claims 71 to 79, wherein the composition further comprises a culture product of the gram-positive bacterium.

81. A method of producing a composition for enhancement of umami of food, the method comprising:
a step of culturing the bacterium according to any one of claims 60 to 70, in a medium, to provide a culture product containing L-glutamic acid, wherein
the composition contains the L-glutamic acid.

82. The method according to claim 81, wherein the composition further comprises the following ingredient (A):
(A) a cell wall-containing fraction of a gram-positive bacterium.

83. A method of producing L-glutamic acid, the method comprising:
a step of culturing the bacterium according to any one of claims 60 to 70, in a medium to provide a culture product containing L-glutamic acid; and
a step of recovering the L-glutamic acid.

84. A composition for enhancement of umami of food, the composition comprising L-glutamic acid, wherein
the L-glutamic acid is produced by culturing the bacterium according to any one of claims 60 to 70, in a medium.

85. The composition according to claim 84, further comprising the following ingredient (A):
(A) a cell wall-containing fraction of a gram-positive bacterium.

86. A method of enhancing umami of food, the method comprising:
a step of adding L-glutamic acid to a raw material of food, wherein
the L-glutamic acid is produced by culturing the bacterium according to any one of claims 60 to 70, in a medium.

87. A method of producing food with enhanced umami, the method comprising:
a step of adding L-glutamic acid to a raw material of food, wherein
the L-glutamic acid is produced by culturing the bacterium according to any one of claims 60 to 70, in a medium.

88. The method according to claim 86 or 87, further comprising
a step of adding the following ingredient (A) to a raw material of food:
(A) a cell wall-containing fraction of a gram-positive bacterium.
